# EUROPEAN PATENT APPLICATION

(11) **EP 3 967 691 A1**
(43) Date of publication of application: **16.03.2022**
(21) Application number: 20794845.6
(22) Date of filing: 30.03.2020
(51) Int. Cl.: C07D 471/04, A61P 31/06, A61K 31/437

(54) **PYRIMIDO FIVE-MEMBERED HETEROCYCLIC COMPOUND AND USE THEREOF AS MUTANT IDH2 INHIBITOR**

(30) Priority: 22.04.2019 CN 201910324679
(71) Applicant: Epitas Biosciences (Shanghai) Co., Ltd., Shanghai 201203 (CN)
(72) Inventor: WANG, Meng, Shanghai 201203 (CN); ZHU, Weixing, Shanghai 201203 (CN)
(74) Representative: Nederlandsch Octrooibureau
(86) International application number: PCT/CN2020/082016
(87) International publication number: WO 2020/215998

(57) **Abstract**

The present invention relates to a pyrimido five-membered heterocyclic compound and use thereof as a mutant IDH2 inhibitor. Specifically, disclosed in the present invention are a pyrimido five-membered heterocyclic compound capable of serving as a mutant IDH2 inhibitor, or a stereoisomer or a tautomer, or a pharmaceutically acceptable salt, a hydrate or a solvate thereof. The present invention also relates to a pharmaceutical composition comprising the compound, and use thereof in the prepration of a medicament for preventing and/or treating a disease mediated by mutant IDH2.

## Description

### TECHNICAL FIELD

The present invention relates to the field of medicinal chemistry, and in particular to a pyrimido five-membered heterocyclic compound and use thereof as a mutant IDH2 inhibitor.

### BACKGROUND

Isocitrate dehydrogenase (IDH) is a key rate-limiting enzyme that catalyzes the production of alpha-ketoglutarate (a-KG) from isocitrate in the tricarboxylic acid cycle. There are three subtypes in higher mammals, namely IDH1, IDH2, and IDH3. Among them, IDH1 is mainly located in the cytoplasmic matrix and peroxisomes, while IDH2 and IDH3 are mainly located in mitochondria. IDH1 and IDH2 are in the form of homodimers and use nicotinamide adenine dinucleotide phosphate (NADP+) as a coenzyme to perform enzymatic catalytic function. IDH3 is a heterotetramer composed of two α subunits, one β subunit and one γ subunit, and uses nicotinamide adenine dinucleotide (NAD+) as a coenzyme to catalyze the production of α-KG from isocitrate, and at the same time, NADH is produced to regulate the redox reaction in the cell.

Studies have found that IDH1/2 have mutations in many different types of tumors, including brain tumors, leukemia, chondrosarcoma, cholangiocarcinoma, etc. Compared with IDH1, IDH2 has a mutation rate of 8.7%-19% in acute myelocytic leukemia (AML). The mutation sites are mainly concentrated in R140Q and R172K. Mutated IDH2 (IDH2m) can cause loss of its normal function and convert α-KG into the carcinogenic metabolite 2-hydroxyglutarate (2-HG), allowing 2-HG to accumulate in the mutated tumor cells. Studies have shown that α-KG is similar in structure to 2-HG. 2-HG competitively binds α-KG-dependent dioxygenase activities (such as DNA demethylase and histone demethylase), resulting in hypermethylation of nucleosomes and/or DNA in some key regions of the genome. This epigenetic change is thought to interfere with normal cell differentiation, leading to excessive proliferation of immature cells, which can lead to cancer.

In tumor cells with IDH2 mutations, mutant IDH2 inhibitors can specifically bind to the mutant enzyme protein, effectively inhibiting the activity of the mutant protease, and reducing the carcinogenic metabolite 2-HG in the body, thereby inducing the demethylation of histones and/or DNA to achieve the effect of promoting tumor cell differentiation and inhibiting tumor development. Companies that take IDH2 mutants as targets to participate in new drug development are mainly represented by Agios Pharmaceuticals in the United States. The drug Enasidenib, which targets the IDH2-R140Q mutation, was approved by the FDA on August 1, 2017 and was successfully marketed. However, the research and development of IDH2 inhibitors has insufficient diversification of chemical types, so there is an urgent need to develop new, high-efficiency and low-toxic IDH2m inhibitors.

### SUMMARY

A purpose of the present invention is to provide a class of mutant IDH2 inhibitors with high selectivity, high efficiency and low toxicity.

In a first aspect, the present invention provides a pyrimido five-membered heterocyclic compound represented by formula I, 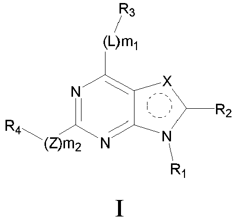 or a stereoisomer or a tautomer, or a pharmaceutically acceptable salt, a hydrate or a solvate thereof,
wherein,
R₁ is absent or selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl;
R₂ is selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₁-C₈ alkoxy, substituted or unsubstituted C₁-C₈ carboxy, substituted or unsubstituted C₂-C₂₀ ester group, substituted or unsubstituted C₆-C₁₀ aryl, or substituted or unsubstituted 5-10 membered heteroaryl with 1-3 heteroatoms selected from N, S and O;
X is selected from N, O, S or CR₅; wherein R₅ is hydrogen, halogen, -CN, substituted or unsubstituted C₁-C₈ alkyl, substituted or unsubstituted C₂-C₈ alkenyl, substituted or unsubstituted C₂-C₈ alkynyl, or substituted or unsubstituted C₃-C₁₀ cycloalkyl;
m₁ is 0, 1, 2, 3, or 4; each L is independently absent or selected from O, S, -CO-, -NH- or -CH₂-;
m₂ is 0, 1 or 2; each Z is independently absent or selected from O, S, -CO-, -NH- or -CH₂-;
R₃ is selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₆-C₁₀ aryl, substituted or unsubstituted 5-10 membered heteroaryl with 1-3 heteroatoms selected from N, S and O, substituted or unsubstituted 4-8 membered heterocyclic group having 1-3 heteroatoms selected from N, S and O;
R₄ is selected from hydrogen, halogen, CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₆-C₁₀ aryl, substituted or unsubstituted 5-10 membered heteroaryl with 1-3 heteroatoms selected from N, S and O;
unless otherwise specified, the term "substituted" refers to being substituted by one or more (for example, 2, 3, 4, etc.) substituents selected from the following group: halogen, C₁-C₆ alkyl, halogenated C₁-C₆ alkyl, C₁-C₆ alkoxy, halogenated C₁-C₆ alkoxy, C₃-C₈ cycloalkyl, halogenated C₃-C₈ cycloalkyl, oxo, -CN, hydroxyl, amino, carboxy, benzyl, C₆-C₁₀ aryl, halogenated C₆-C₁₀ aryl, 5-10 membered heteroaryl with 1-3 heteroatoms selected from N, S and O, halogenated 5-10 membered heteroaryl with 1-3 heteroatoms selected from N, S and O.

In another preferred embodiment, for R₃, the term "substituted" refers to being substituted by one or more (for example, 2, 3, 4, etc.) substituents selected from the following group: halogen, CN, hydroxyl, substituted or unsubstituted C₁-C₆ alkyl, C₁-C₆ haloalkyl or substituted or unsubstituted C₁-C₆ alkoxy.

In another preferred embodiment, for R₄, the term "substituted" refers to being substituted by one or more (for example, 2, 3, 4, etc.) substituents selected from the following group: halogen, CN, hydroxyl, substituted or unsubstituted C₁-C₆ alkyl or substituted or unsubstituted C₁-C₆ haloalkyl.

In another preferred embodiment, the ester group comprises -(substituted or unsubstituted C₁-C₆ alkylene)-C(O)-O-(substituted or unsubstituted C₁-C₆ alkyl).

In another preferred embodiment, X is O or S, and R₁ is absent.

In another preferred embodiment, the compound has a structure represented by formula Ia: 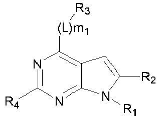 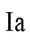 wherein, R₁, R₂, R₃, R₄, L, and m₁ are as defined above.

In another preferred embodiment, m₁ is 2, and -(L)m₁- is -NH-CH₂ or -CH₂-NH-.

In another preferred embodiment, m₂ is 2, and -(Z)m₂- is -NH-CH₂ or -CH₂-NH-.

In another preferred embodiment, L is NH, m₁ is 1, and Z is absent, and m₂ is 0.

In another preferred embodiment, R₃ is a C₃-C₆ cycloalkyl group.

In another preferred embodiment, R₂ is methyl or trifluoromethyl.

In another preferred embodiment, R₄ is a fluorine-substituted phenyl group.

In another preferred embodiment, X is CR₅.

In another preferred embodiment, R₅ is H, C₁-C₄ alkyl, or C₃-C₄ cycloalkyl.

In another preferred embodiment, the compound is compound #1, #2, #3, #4, #5, #6, #7, #8, #9, #10, #11, #12, #13, #14, #15, #16, #17, #18, #19, #20, #21, #22, #23, #24, #25, #26, #27, #28, #29, #30, #31, #32, #33, #34, #35, #36, #37, #38, #39, #40, #41, #42, #43, #44, #45, #46, #47, #48, #49, #50, or #51 in Table 1, or a pharmaceutically acceptable salt thereof.

In another preferred embodiment, the compound is compound #28, #48, #49, or #51 in Table 1, or a pharmaceutically acceptable salt thereof.

In another preferred embodiment, the compound is selected from:

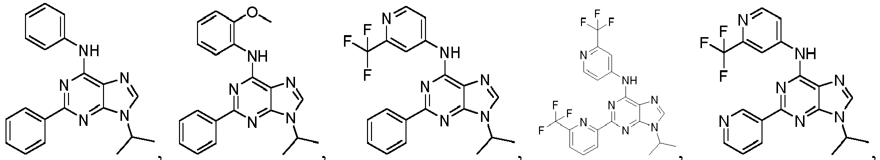

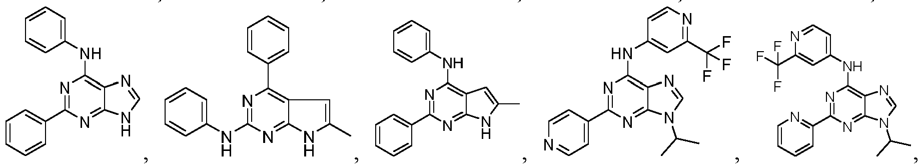

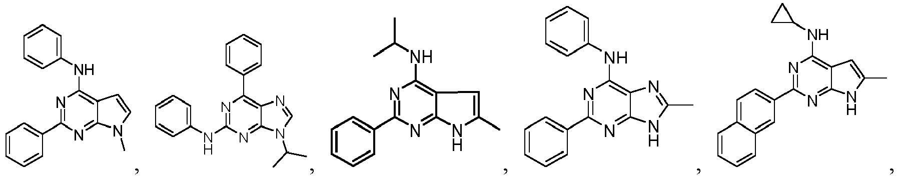

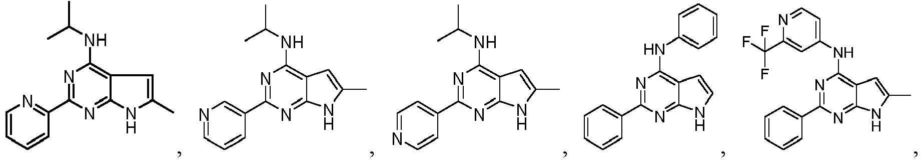

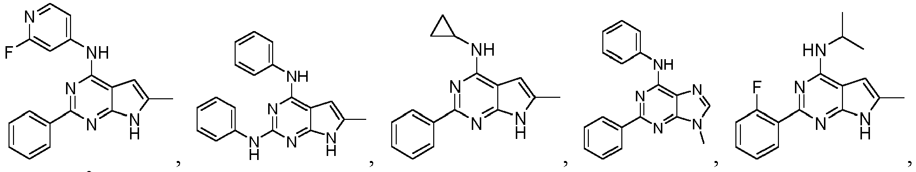

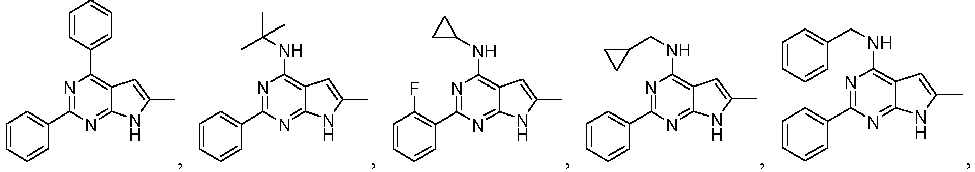

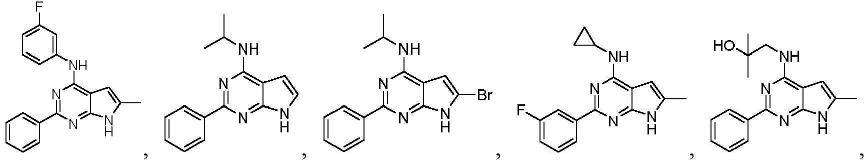

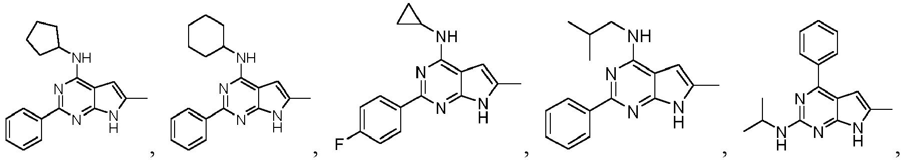

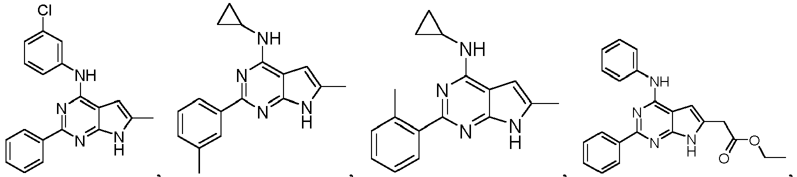

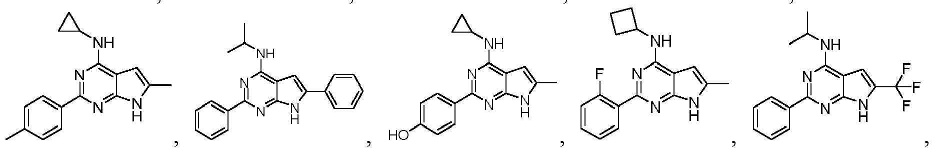

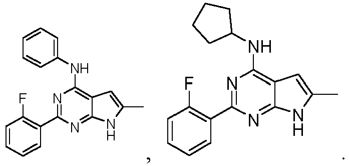

In a second aspect, the present invention provides a pharmaceutical composition, comprising: (1) the compound of the first aspect of the present invention, or a stereoisomer or a tautomer, or a pharmaceutically acceptable salt, a hydrate or a solvate thereof; 2) a pharmaceutically acceptable carrier.

In a third aspect, provided is a use of the compound of the first aspect of the present invention, or a stereoisomer or a tautomer, or a pharmaceutically acceptable salt, a hydrate or a solvate thereof, or the pharmaceutical composition of the second aspect of the present invention for the manufacture of a medecament for preventing and/or treating a disease mediated by mutant IDH2.

In another preferred embodiment, the disease mediated by mutant IDH2 is cancer; preferably, the cancer is selected from bladder cancer, breast cancer, kidney cancer, liver cancer, lung cancer (including small cell lung cancer), esophageal cancer, gallbladder cancer, ovarian cancer, pancreatic cancer, gastric cancer, cervical cancer, thyroid cancer, prostate cancer and skin cancer (including squamous cell carcinoma); hematopoietic tumors of the lymphatic system, including, for example, leukemia, acute lymphoid cell leukemia, acute lymphoblastic leukemia, B-cell lymphoma, T-cell lymphoma, Hodgkin's lymphoma, non-Hodgkin's lymphoma, hair cell lymphoma and Burkitt's lymphoma; tumors derived from mesenchymal cells, including, for example, fibrosarcoma and rhabdomyosarcoma; myeloid hematopoietic tumors, including, for example, acute and chronic myelogenous leukemia, myelodysplastic syndrome and promyelocytic leukemia; central and peripheral nervous system tumors, including, for example, astrocytoma, neuroblastoma, glioma, and schwannoma; and other tumors, including, for example, melanoma, seminoma, teratoma, osteosarcoma, xeroderma pigmentosum, keratoacanthoma, thyroid follicular carcinoma and Kaposi's sarcoma.

In a fourth aspect, provided is a method for preparing the compound of formula I, the method comprising: 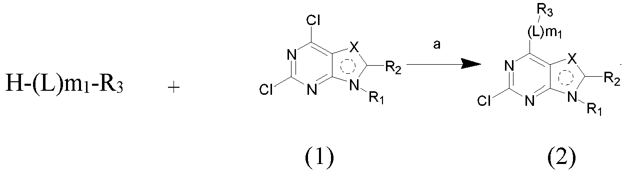 (a) reacting a compound of formula (1) with H-(L)m₁-R₃ to prepare a compound of formula (2), wherein H-(L)m₁-R₃ is an amine compound or a boric acid compound or a borate compound substituted with R₃; and 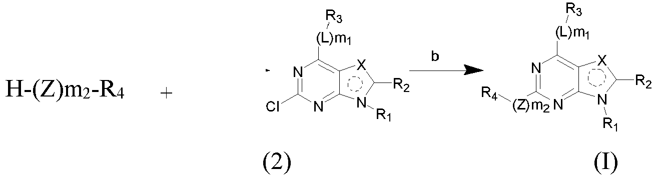 (b) reacting the compound of formula (2) with H-(Z)m₂-R₄ to prepare the compound of formula (I), wherein H-(Z)m₂-R₄ is an amine compound or a boric acid compound or a borate compound or an organotin compound substituted with R₄;
wherein, R₁, R₂, R₃, R₄, L, Z, m₁, m₂ are as defined in the first aspect of the present invention.

In a fifth aspect, provided is a method for preparing the compound represented by formula Ia, wherein the method comprises: 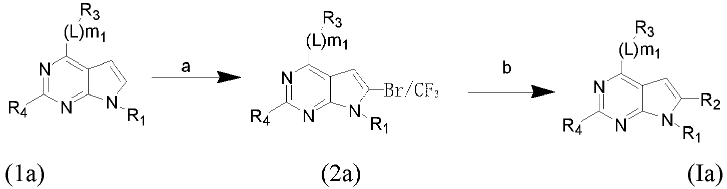 (a1) reacting a compound of formula (1a) with *N*-bromosuccinimide or S-(trifluoromethyl)dibenzothiophenium tetrafluoroborate (Umemoto's reagents) to prepare a compound of formula (2a); and
(b1) reacting the compound of formula (2a) with a boric acid compound R₂-B(OH)₂ to prepare the compound represented by formula (Ia);
wherein, the definitions of R₁, R₂, R₃, R₄, L, and m₁ are as defined in the first aspect of the present invention.

In a sixth aspect, provided is a mutant IDH2 inhibitor, which comprises the compound of the first aspect of the present invention, or a stereoisomer or a tautomer, or a pharmaceutically acceptable salt, a hydrate or a solvate thereof, or the pharmaceutical composition of the second aspect of the present invention.

In a seventh aspect, provided is a in vitro method for inhibiting the proliferation of tumor cells containing mutant IDH2, comprising: contacting the compound of the first aspect of the present invention, or a stereoisomer or a tautomer, or a pharmaceutically acceptable salt, a hydrate or a solvate thereof, or the pharmaceutical composition of the second aspect of the present invention with a mutant IDH2, thereby inhibiting the activity of the mutant IDH2.

In an eighth aspect, provided is a method for preventing and/or treating a disease mediated by mutant IDH2, comprising: administering to a subject in need thereof the compound of the first aspect of the present invention, or a stereoisomer or a tautomer, or a pharmaceutically acceptable salt, a hydrate or a solvate thereof, or the pharmaceutical composition of the second aspect of the present invention.

In another preferred embodiment, the subject comprises a mammal, such as a human.

It should be understood that within the scope of the present invention, the above-mentioned technical features of the present invention and the technical features specifically described in the following (such as in the examples) can be combined with each other to form a new or preferred technical solution, which will not be repeatedly described herein due to space limitation.

### DETAILED DESCRIPTION OF EMBODIMENTS

After an extensive and in-depth research, the inventors have developed a new type of compounds with excellent inhibitory activity against mutant IDH2 for the first time. The compounds of the present invention have excellent selectivity for mutant IDH2, extremely low toxicity to normal cells, and have good druggability and pharmacokinetic activity. On this basis, the present invention has been completed.

### Definitions

As used herein, the term "alkyl" includes linear or branched chain alkyl groups. For example, C₁-C₈ alkyl refers to a linear or branched alkyl group having 1-8 carbon atoms (preferably, 1-6, more preferably, 1-3), such as methyl, ethyl, propyl, isopropyl, butyl, isobutyl, tert-butyl, etc.

As used herein, the term "alkenyl" includes linear or branched alkenyl. For example, C₂-C₈ alkenyl refers to a linear or branched alkenyl having 2-8 carbon atoms (preferably, 2-4), such as ethenyl, allyl, 1-propenyl, isopropenyl, 1-butenyl, 2-butenyl, or similar groups.

As used herein, the term "alkynyl" includes linear or branched alkynyl groups. For example, C₂-C₈ alkynyl refers to a linear or branched alkynyl group having 2-8 carbon atoms (preferably, 2-4), such as ethynyl, propynyl, butynyl, or similar groups .

As used herein, the term "C₃-C₁₀ cycloalkyl" refers to a cycloalkyl having 3-10 carbon atoms (preferably, 3-6). It can be a monocyclic ring, such as cyclopropyl, cyclobutyl, cyclopentyl, cyclohexyl, or the like. It may also be in the form of a bicyclic ring, such as a bridged ring or a spiro ring.

As used herein, the term " C₁-C₈ alkoxy" refers to a linear or branched alkoxy group having 1-8 carbon atoms (preferably, 1-6, more preferably, 1-3); for example, methoxy, ethoxy, propoxy, isopropoxy, butoxy, isobutoxy, tert-butoxy and the like.

As used herein, the term "3-10 membered heterocyclic group having 1-3 heteroatoms selected from the group consisting of N, S, and O" refers to a saturated or partially saturated cyclic group having 3-10 ring atoms of which 1-3 are heteroatoms selected from the group consisting of N, S and O. It can be monocyclic, bicyclic or polycyclic, such as bridged or spirocyclic. Representative examples include, but are not limited to: oxetane, azetidine, tetrahydro-2H-pyranyl, piperidinyl, tetrahydrofuranyl, morpholinyl, pyrrolidinyl, and the like.

As used herein, the term "C₆-C₁₀ aryl group" refers to an aryl group having 6-10 carbon atoms, for example, a phenyl group or a naphthyl group and the like.

As used herein, the term "5-10 membered heteroaryl group having 1-3 heteroatoms selected from the group consisting of N, S, and O" refers to a cyclic aromatic group having 5-10 ring atoms of which 1-3 ring atoms are heteroatoms selected from the group consisting of N, S and O. It may be a monocyclic ring or in a condensed ring form. Specific examples can be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazolyl, furyl, thienyl, isoxazolyl, thiazolyl, oxazolyl, etc.

Unless specifically stated to be "substituted or unsubstituted", the groups described in the present invention can be substituted by one or more substituents (for example 2, 3, 4, 5, etc.) selected from the following group: halogen, C₁-C₆ alkyl, halogenated C₁-C₆ alkyl, C₁-C₆ alkoxy, halogenated C₁-C₆ alkoxy, C₃-C₈ cycloalkyl, halogenated C₃-C₈ cycloalkyl, oxo, -CN, hydroxyl, amino, carboxy, benzyl, C₆-C₁₀ aryl, halogenated C₆-C₁₀ aryl, 5-10 membered heteroaryl with 1-3 heteroatoms selected from N, S and O, halogenated 5-10 membered heteroaryl with 1-3 heteroatoms selected from N, S and O.

As used herein, "halogen" or "halogen atom" refers to F, Cl, Br, and I. More preferably, the halogen or halogen atom is selected from F, Cl and Br. "Halogenated" refers to being substituted with an atom selected from F, Cl, Br, and I.

Unless otherwise specified, the structural formula described in the present invention is intended to include all isomeric forms (such as enantiomers, diastereomers and geometric isomers (or conformational isomers)), for example, R and S configurations containing a asymmetric center, (Z) and (E) isomers of double bonds, etc. Therefore, a single stereochemical isomer of the compound of the present invention or a mixture of its enantiomers, diastereomers or geometric isomers (or conformational isomers) all fall in the scope of the present invention.

As used herein, the term "tautomer" means that structural isomers with different energies can exceed the low energy barrier to convert into each other. For example, proton tautomers (i.e., prototropic change) include interconversion through proton transfer, such as 1H-indazole and 2H-indazole. Valence tautomers include interconversion through the recombination of some bonding electrons.

As used herein, the term "solvate" refers to a complex formed by coordination of a compound of the present invention with solvent molecules at a specific ratio.

As used herein, the term "hydrate" refers to a complex formed by coordination of a compound of the present invention with water.

### Active ingredients

As used herein, "a compound of the present invention" refers to the compound represented by formula I, and also includes an isomer, a racemate, a crystalline or amorphous form, a pharmaceutically acceptable salt, a hydrate or a solvate of the compound of formula I.

As used herein, "a pharmaceutically acceptable salt" refers to a salt formed by a compound of the present invention and an acid or base suitable for use as a medicine. Pharmaceutically acceptable salts include inorganic salts and organic salts. A preferred class of salts are the salts formed by the compounds of the present invention with acids. Acids suitable for salt formation include, but are not limited to: inorganic acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and acidic amino acids such as aspartic acid and glutamic acid. A preferred class of salts are the salts formed by the compounds of the present invention with bases. Suitable bases for salt formation include, but are not limited to: inorganic bases such as sodium hydroxide, potassium hydroxide, sodium carbonate, sodium bicarbonate, and sodium phosphate, and organic bases such as ammonia, triethylamine, and diethylamine.

The compound of the present invention may be in a amorphous form, a crystalline form, or a mixture thereof.

Certain compounds of the present invention can exist in unsolvated as well as solvated forms, including hydrated forms. The solvated form is generally equivalent to the unsolvated form and should be included in the scope of the present invention. Certain compounds of the present invention may exist in a polymorphic or amorphous form. Generally, as far as the application considered in the present invention is concerned, all physical forms are equivalent and should be included in the scope of the present invention.

A compound of the present invention may also contains an unnatural proportion of atomic isotopes at one or more of the isotopic atoms constituting the compound. An unnatural proportion of a certain isotope can be defined as from the naturally found amount of the atom concerned to 100% of that atom. For example, the compound may be incorporated with radioactive isotopes, such as tritium (³H), iodine-125 (¹²⁵I), or carbon-14 (¹⁴C), or non-radioactive isotopes, such as deuterium (²H) or carbon-13 (¹³C). In addition to those uses described in the present application, such isotopic variants may provide additional uses. For example, isotopic variants of the compounds of the invention may have additional uses, including, but not limited to, as diagnostic and/or imaging reagents, or as cytotoxic/radiotoxic therapeutic agents. In addition, isotopic variants of the compounds of the present invention may have altered pharmacokinetic and pharmacodynamic characteristics, thereby helping to increase safety, tolerability or efficacy during treatment. Regardless of whether it is radioactive or not, all isotopic variants of the compounds of the present invention should be included within the scope of the present invention.

In another preferred embodiment, the R₁, R₂, R₃, R₄, L, Z, m₁, and m₂ are each independently a group corresponding to each compound in Table 1.

Preferred compounds of the present invention are shown in Table 1:

**Table 1**

| Compo und No. | Structural formula | Comp ound No. | Structural formula |
|---|---|---|---|
| 1 | 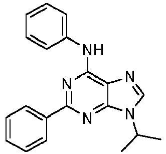 | 2 | 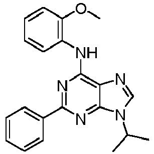 |
| 3 | 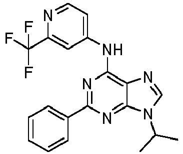 | 4 | 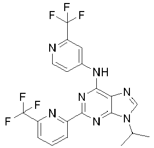 |
| 5 | 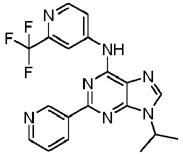 | 6 | 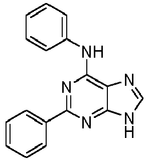 |
| 7 | 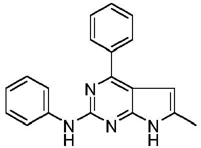 | 8 | 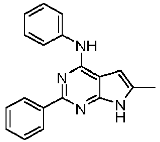 |
| 9 | 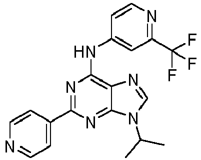 | 10 | 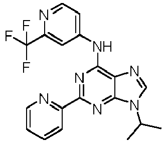 |
| 11 | 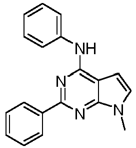 | 12 | 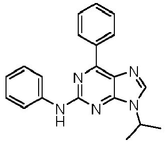 |
| 13 | 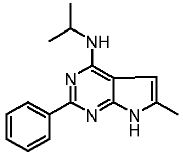 | 14 | 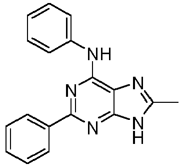 |
| 15 | 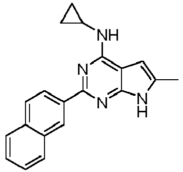 | 16 | 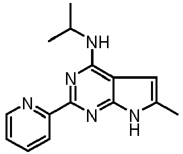 |
| 17 | 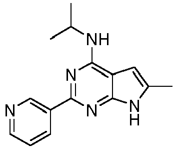 | 18 | 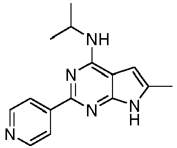 |
| 19 | 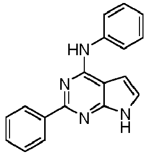 | 20 | 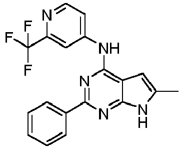 |
| 21 | 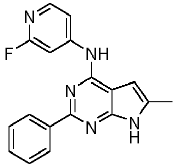 | 22 | 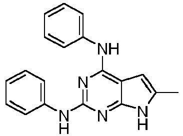 |
| 23 | 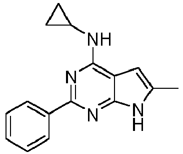 | 24 | 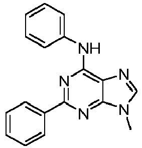 |
| 25 | 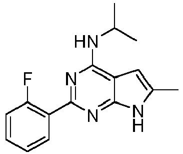 | 26 | 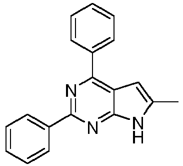 |
| 27 | 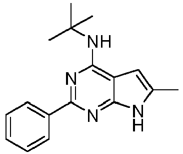 | 28 | 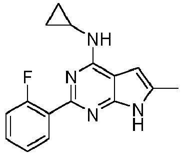 |
| 29 | 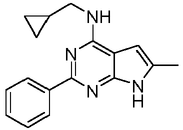 | 30 | 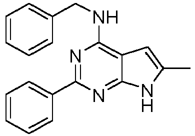 |
| 31 | 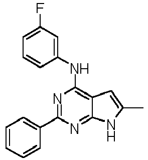 | 32 | 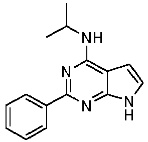 |
| 33 | 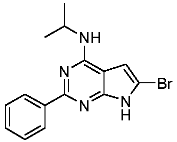 | 34 | 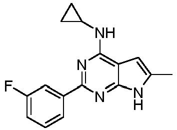 |
| 35 | 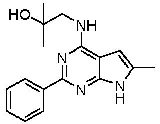 | 36 | 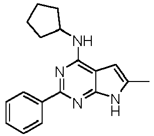 |
| 37 | 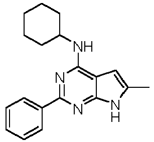 | 38 | 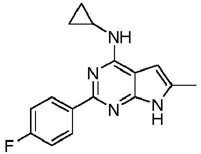 |
| 39 | 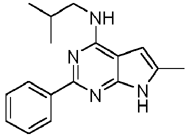 | 40 | 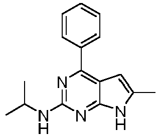 |
| 41 | 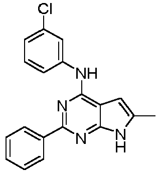 | 42 | 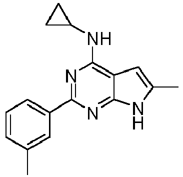 |
| 43 | 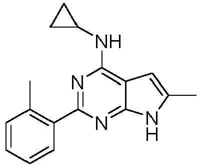 | 44 | 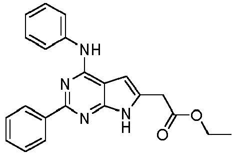 |
| 45 | 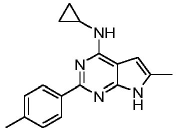 | 46 | 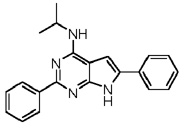 |
| 47 | 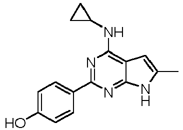 | 48 | 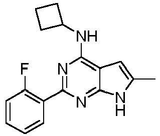 |
| 49 | 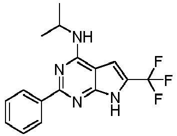 | 50 | 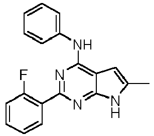 |
| 51 | 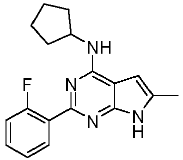 | | |

### Methods of preparation

A method for preparing the compound of formula I is provided, the method comprising: 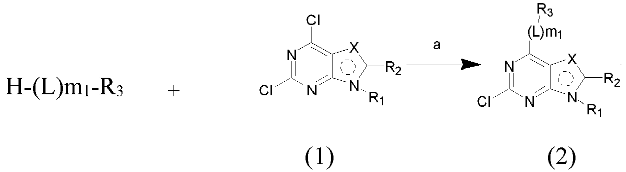 (a) reacting a compound of formula (1) with H-(L)m₁-R₃ to prepare a compound of formula (2), wherein H-(L)m₁-R₃ is an amine compound or a boric acid compound or a borate compound substituted with R₃; and 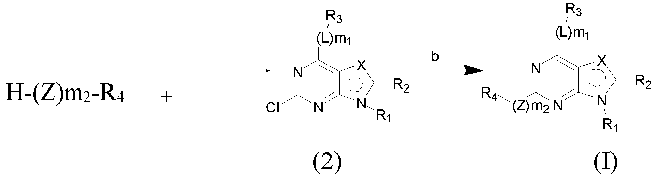 (b) reacting the compound of formula (2) with H-(Z)m₂-R₄ to prepare the compound of formula (I), wherein H-(Z)m₂-R₄ is an amine compound or a boric acid compound or a borate compound or an organotin compound substituted with R₄;
wherein, R₁, R₂, R₃, R₄, L, Z, m₁, m₂ are as defined above.

A method for preparing the compound represented by formula Ia is provided, wherein the method comprises: 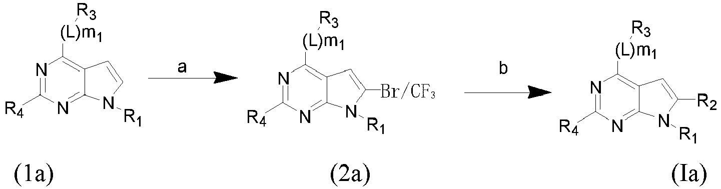 (a1) reacting a compound of formula (1a) with *N*-bromosuccinimide or S-(trifluoromethyl)dibenzothiophenium tetrafluoroborate (Umemoto's reagents) to prepare a compound of formula (2a); and
(b1) reacting the compound of formula (2a) with a boric acid compound R₂-B(OH)₂ to prepare the compound represented by formula (Ia);
wherein, the definitions of R₁, R₂, R₃, R₄, L, and m₁ are as defined above.

### Pharmaceutical compositions and methods of administration

Since the compounds of the present invention have excellent mutant IDH2 inhibitory activity and high selectivity, the compounds of the present invention and the pharmaceutical compositions containing the compounds of the present invention as the main active ingredient can be used to prevent and/or treat (stabilize, alleviate or cure) related diseases mediated by mutant IDH2. Representative diseases include but are not limited to: bladder cancer, breast cancer, kidney cancer, liver cancer, lung cancer (including small cell lung cancer), esophageal cancer, gallbladder cancer, ovarian cancer, pancreatic cancer, gastric cancer, cervical cancer, thyroid cancer, prostate cancer and skin cancer (including squamous cell carcinoma); hematopoietic tumors of the lymphatic system, including, for example, leukemia, acute lymphoid cell leukemia, acute lymphoblastic leukemia, B-cell lymphoma, T-cell lymphoma, Hodgkin's lymphoma, non-Hodgkin's lymphoma, hair cell lymphoma and Burkitt's lymphoma; tumors derived from mesenchymal cells, including, for example, fibrosarcoma and rhabdomyosarcoma; myeloid hematopoietic tumors, including, for example, acute and chronic myelogenous leukemia, myelodysplastic syndrome and promyelocytic leukemia; central and peripheral nervous system tumors, including, for example, astrocytoma, neuroblastoma, glioma, and schwannoma; and other tumors, including, for example, melanoma, seminoma, teratoma, osteosarcoma, xeroderma pigmentosum, keratoacanthoma, thyroid follicular carcinoma and Kaposi's sarcoma.

The pharmaceutical composition of the present invention contains a safe and effective amount of the compound of the present invention and a pharmaceutically acceptable excipient or carrier. The term "a safe and effective amount" refers to: the amount of the compound is sufficient to significantly improve the condition without causing serious side effects. Generally, the pharmaceutical composition contains 1-2000 mg of the compound of the present invention per dosage, and more preferably, contains 10-200 mg of the compound of the present invention per dosage. Preferably, the term "one dosage" is a capsule or tablet.

"A pharmaceutically acceptable carrier" refers to: one or more compatible solid or liquid fillers or gel substances, which are suitable for human use, and must have sufficient purity and sufficiently low toxicity. "Compatibility" here means that the components in the composition can be blended with the compound of the present invention and with each other without significantly reducing the efficacy of the compound. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), wetting agents (such as sodium lauryl sulfate), coloring agents, flavoring agents, stabilizers, antioxidants, preservatives, pyrogen-free water, etc.

The method of administration of the compound or the pharmaceutical composition of the present invention is not particularly limited, and representative administration methods include (but are not limited to): oral, parenteral (intravenous, intramuscular, or subcutaneous).

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following ingredients: (a) fillers or compatibilizers, for example, starch, lactose, sucrose, glucose, mannitol and silicic acid; (b) binders such as hydroxymethyl cellulose, alginate, gelatin, polyvinylpyrrolidone, sucrose and gum arabic; (c) humectants, such as glycerin; (d) disintegrants, such as agar, calcium carbonate, potato starch or tapioca starch, alginic acid, certain complex silicates, and sodium carbonate; (e) sustained-releasing agents, such as paraffin; (f) absorption accelerators, such as quaternary amine compounds; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium lauryl sulfate, or mixtures thereof. In capsules, tablets and pills, the dosage form may also contain buffering agents.

Solid dosage forms such as tablets, sugar pills, capsules, pills and granules can be prepared with coatings and shell materials, such as enteric coatings and other materials known in the art. They may contain opacifying agents, and the active compound or compound in the composition can be released in a certain part of the digestive tract in a delayed manner. Examples of embedding components that can be used are polymeric substances and waxes. If necessary, the active compound can also be formed into microcapsules with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition may also contain adjuvants such as wetting agents, emulsifying agents and suspending agents, sweetening agents, flavoring agents and perfumes.

In addition to the active compound, the suspension may contain suspending agents, for example, ethoxylated isostearyl alcohol, polyoxyethylene sorbitol and sorbitan esters, microcrystalline cellulose, aluminum methoxide and agar, or mixtures of these substances, and the like.

The composition for parenteral injection may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The compounds of the present invention can be administered alone or in combination with other pharmaceutically acceptable compounds (for example, anticancer agents).

When administered in combination, the pharmaceutical composition further includes one or more (2, 3, 4, or more) other pharmaceutically acceptable compounds (for example, anticancer agents). One or more (2, 3, 4, or more) of the other pharmaceutically acceptable compounds (such as anticancer agents) can be used simultaneously, separately or sequentially with the compound of the present invention to prevent and/or treat diseases mediated by mutant IDH2.

When using the pharmaceutical composition, a safe and effective amount of the compound of the present invention is administered to a mammal (such as a human) in need of treatment. The administered dose is the effective dosage considered pharmaceutically. For a person with a body weight of 60 kg, the daily dosage is usually 1 to 2000 mg, preferably 20 to 500 mg. Of course, the specific dosage should also consider factors such as the route of administration, the patient's health status, etc., which are within the range of a skilled physician.

### The main advantages of the present invention include:

(1) The compounds of the present invention have novel structures and excellent inhibitory effects on mutant IDH2, and the compounds of the present invention have almost no activity on wild-type IDH2 (IDH2/WT), and have good selectivity.
(2) The compounds of the present invention have very low toxicity to normal cells, so it can be applied to a subject in a relatively large dose range.
(3) The compounds of the present invention have good druggability and can be very easily prepared into pharmaceutically acceptable salts, thus helping to further form preparations.
(4) The compounds of the present invention and the pharmaceutical compositions containing the compounds of the present invention as the main active ingredient can be used to prevent and/or treat a disease mediated by mutant IDH2.

The present invention will be further explained below in conjunction with specific embodiments. It should be understood that these embodiments are only used to illustrate the present invention and not to limit the scope of the present invention. The experimental methods without specific conditions in the following examples are usually based on conventional conditions, such as the conditions described in Sambrook et al., Molecular Cloning: Laboratory Manual (New York: Cold Spring Harbor Laboratory Press, 1989), or according to the conditions suggested by the manufacturer. Unless otherwise specified, percentages and parts are percentages and parts by weight. The raw materials or instruments used in the examples of the present invention, unless otherwise specified, are commercially available.

The control compound in the examples is Enasidenib (AG-221), CAS: 1446502-11-9, and the structural formula is as follows:

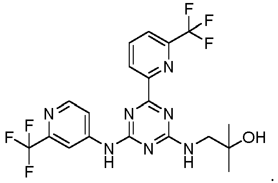

### Example 1

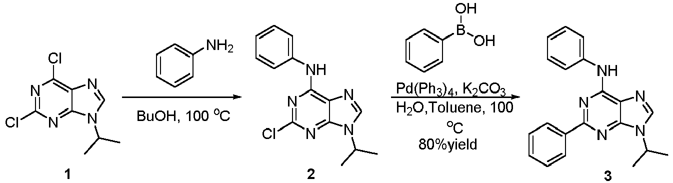

### Step 1: 2-chloro-9-isopropyl-N-phenyl-9H-purin-6-amine (2)

2,6-dichloro-9-isopropyl-9H-purine (138 mg, 0.60 mmol) and 8 mL of n-butanol were added to a 25 mL round bottom flask. Et₃N (96 mg, 0.96 mmol) and aniline (67 mg, 0.72 mmol) were added. The reaction mixture was stirred at 100°C for 16 h (overnight) and concentrated in vacuo. The residue was purified by automatic flash column chromatography (silica gel, DCM:MeOH=20:1) to obtain 2-chloro-9-isopropyl-N-phenyl-9H-purin-6-amine (144 mg, 83 % yield) as a white solid.

ESI m/z: 415.2 (M + H)⁺.

### Step 2: 9-isopropyl-N,2-diphenyl-9H-purin-6-amine (3)

2-chloro-9-isopropyl-N-phenyl-9H-purin-6-amine (100mg, 0.35mmol), phenylboronic acid (50mg, 0.4mmol), Pd(PPh3)₄(40mg,0.03mmol), K₂CO₃(140mg, 1mmol), toluene (5mL), water (1mL) were filled into a 10 ml sealed tube. The reaction mixture was stirred at 100°C in an inert gas for 3 h. After the reaction was completed, the reaction mixture was evaporated and concentrated with silica gel (100-200 mesh) to obtain a powder residue. The product was purified by automatic flash column chromatography (silica gel, EA:PE=1:1) to obtain 92 mg of the product, with a yield of 80%, as a colorless solid.

ESI m/z: 330.2 (M + H)+. 1H NMR (DMSO-d6, 400 MHz) δ 9.91 (s, 1H), 8.41-8.44 (m, 3H), 8.06 (¹³C). d, J=7.6Hz 2H), 7.38-7.54 (m, 3H), 7.36 (m, 2H), 7.06 (t, J=7.2Hz 1H) , 4.88-4.95 (m, J=6.8Hz, 1H), 1.63(d, J=6.4Hz,6H) ppm.

### Example 2

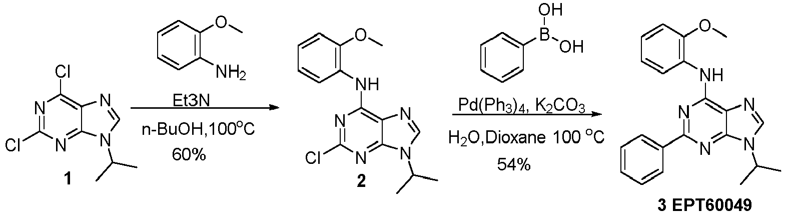

### Step 1: 2-chloro-9-isopropyl-N-(2-methoxyphenyl)-9H-purin-6-amine (2)

2,6-dichloro-9-isopropyl-9H-purine (115 mg, 0.50 mmol) and 5 mL n-butanol were filled into a 10 mL sealed tube. Et₃N (80 mg, 0.80mmol) and 2-methoxyaniline (74 mg, 0.60 mmol) were added. The reaction mixture was stirred at 100°C for 4 h. After the reaction was completed, the reaction mixture was evaporated and concentrated with silica gel (100-200 mesh) to obtain a powder residue. The product was purified by automatic flash column chromatography (silica gel, EA: PE=3:2), and the product was obtained as a white solid (95 mg, yield 60%).

Molecular formula: C₁₅H₁₆CalN₅O, molecular weight: 317.78, ESI m/z: 318.1 (M + H)⁺.

### Step 2: 9-isopropyl-N-(2-methoxyphenyl)-2-phenyl-9H-purin-6-amine (3 EPT60049)

2-chloro-9-isopropyl N-(2-methoxyphenyl)-9H-purin-6-amine (95mg, 0.3 mmol), phenylboronic acid (50mg, 0.4 mmol), Pd(PPh3)₄ (34mg,0.03mmol), K₂CO₃(139mg, 1mmol), dioxane (5mL), water (1mL) were filled into a 10 ml sealed tube. The reaction mixture was heated at 100°C for 3 h in an inert gas. After the reaction was completed, the reaction mixture was evaporated and concentrated with silica gel (100-200 mesh) to obtain a powder residue. The product was purified by automatic flash column chromatography (silica gel, EA: PE=1:1), and the product (58 mg, 54% yield) was obtained as a white sol.

Molecular formula: C₂₁H₂₁N₅O, molecular weight: 359.43, (ESI) m/z=360.2 (M+H)⁺.¹H NMR (400 MHz, DMSO-d₆) 8.63-8.66 (m, 1H), 8.41 (m, 4H), 7.48-7.55(m, 3H), 7.10-7.17(m, 3H), 4.91 (m, J=6.8Hz, 1H) 3.94 (s, 3H) 1.63 (d, J=6.8Hz, 6H) ppm.

### Example 3

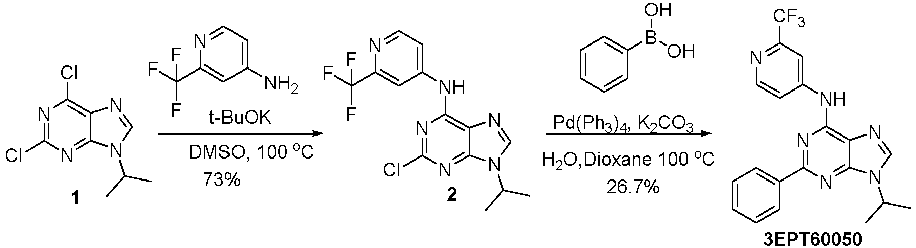

### Step 1: 2-chloro-9-isopropyl-N-(2-(trifluoromethyl)pyridin-4-yl)-9H-purin-6-amine (2)

2,6-dichloro-9-isopropyl-9H-purine (200 mg, 0.86 mmol) and 10 mL DMSO were filled into a 10 mL sealed tube. t-BuOK (150 mg, 1.33 mmol) and 2-(trifluoromethyl)pyridin-4-amine (180 mg, 1.11 mmol) were added. The reaction mixture was heated by microwave at 100°C for 1 h. After the reaction, water (50 mL) was added to the mixture. The mixture was extracted with EA (20 mL 3), the organic layer was compounded, dried over anhydrous sodium carbonate, and filtered. Then the organic layer was concentrated and evaporated with silica gel (100-200 mesh) to obtain a powdery residue. The residue was purified by automatic flash column chromatography (silica gel, EA: PE=7:3) to obtain the title compound (225 mg, yield 73%) as a yellow solid.

Molecular formula: C₁₄H₁₂ClF₃N₆, molecular weight: (ESI) m/z=357.1 (M+H)⁺.

### Step 2: 9-isopropyl-2-phenyl-N-(2-(trifluoromethyl)pyridin-4-yl)-9H-purin-6-amine (3 EPT60050)

2-chloro-9-isopropyl N-(2-(trifluoromethyl)pyridin-4-yl)-9H-purin-6-amine (50mg, 0.14mmol), phenylboronic acid (18mg, 0.15mmol) ), Pd(PPh3)₄ (15mg, 0.014mmol), K₂CO₃(56mg, 0.4mmol), dioxane (5mL), and water (1mL) were respectively filled into a 10-mL sealed tube. The reaction mixture was heated to 100°C in an inert gas for 1.5 h. After the reaction was completed, the reaction mixture was evaporated and concentrated with silica gel (100-200 mesh) to obtain a powder residue. The product was purified by automatic flash column chromatography (silica gel, EA:PE=4:1), and the product was obtained as a white solid (15 mg, yield 26.7%).

Molecular formula: C₂₀H₁₇F₃N₆, molecular weight: 398.39, (ESI) m/z=399.2 (M+H)⁺. ¹H NMR (400 MHz, DMSO-d₆) 10.84 (s,1H), 8.93 (d, J=2.0Hz, 1H), 8.61 ( d, J=5.6Hz, 1H), 8.55 (s,1H), 8.39-8.41 (m,2H), 8.19-8.21 (m, 1H), 7.48-7.54 (m, 3H), 4.92 (m, J=6.8 Hz, 1H), 1.61 (d, *J*=6.8 Hz, 6H) ppm.

### Example 4

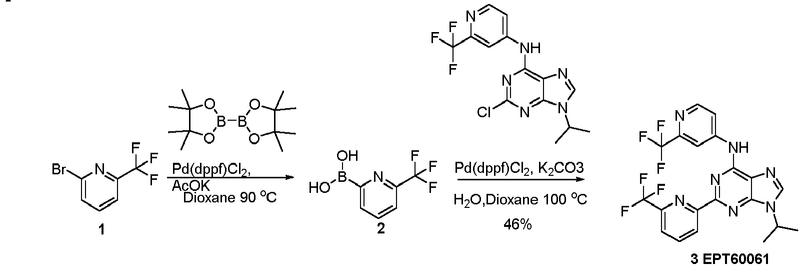

### Step 1 (6-(trifluoromethyl)pyridin-2-yl)boronic acid (2)

2-bromo-6-(trifluoromethyl)pyridine (226mg, 1.0mmol), 4, 4, 4', 4', 5, 5, 5', 5', 5', 5', 5', 5',5'-octamethyl-2,2'-bi(1,3,2-dioxborane) (280mg, 1.1 mmol), Pd(dppf)Cl₂ (140mg,0.2mmol), AcOK (400mg , 4.0mmol) were respectively filled into a 10 mL sealed tube. The reaction mixture was heated at 90c in an inert gas for 4h. The reaction was monitored by LCMS, (ESI) m/z=192.09, no SM-1 remained, and the crude product was used in the next step without further purification.

### Step 2: 9-isopropyl-2-(6-(trifluoromethyl)pyridin-2-yl)-N-(2-(trifluoromethyl)pyridin-4-yl)-9H-pu rine-6 -amine (3 EPT60061)

2-chloro-9-isopropyl N-(2-(trifluoromethyl)pyridin-4-yl)-9H-purin-6-amine (50mg, 0.14mmol), (6-(trifluoromethyl) )pyridin-2-yl)boronic acid (2.5mL dioxane stock solution), Pd(dppf)Cl₂(10mg,0.014mmol), K₂CO₃ (56mg, 0.4mmol), added with water (0.5mL), were added to a 20 ml sealed tube. The reaction mixture was heated to 90°C in an inert gas for 3 h. After the reaction was completed, the reaction mixture was evaporated and concentrated with silica gel (100-200 mesh) to obtain a powder residue. Residue oil was concentrated and evaporated into black oil residue. The product was purified by automatic flash column chromatography (silica gel, MeOH: DCM=1:24), and the product was obtained as a white solid (30 mg, yield 46%).

Molecular formula: C₂₀H₁₅F₆N₇, molecular weight: 467.38, (ESI) m/z=468.2 (M+H)⁺. ¹HNMR (400 MHz, DMSO-d6) 10.93 (s,1H),9.01 (d, J=2.0 Hz,1H), 8.70(d, *J*=8.0 Hz,1H), 8.66 (s,1H), 8.53-8.54 (d, J=5.6 Hz,1H ), 8.42-8.44(m, 1H), 8.26(t, J=8.0 Hz 1H), 8.00 (d, *J*=7.6Hz,1H ) 4.95 (m, *J*=6.8Hz,1H), 1.60-1.62 (d, J=6.8Hz,6H) ppm.

### Example 5

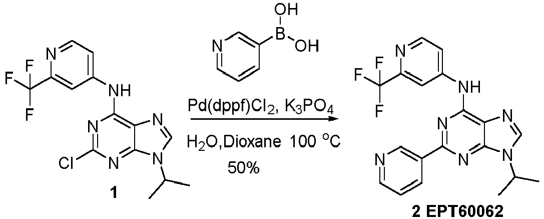

### Step 1: 9-isopropyl-2-(pyridin-3-yl)-N-(2-(trifluoromethyl)pyridin-4-yl)-9H-purin-6-amine (2 EPT60062)

2-chloro-9-isopropyl-N-(2-(trifluoromethyl)pyridine-4-acyl)-9H-purin-6-amine (36mg, 0.10mmol), pyridin-3-ylboronic acid (37mg, 0.30mmol), Pd(dppf)Cl₂(7mg, 0.01mmol), K₃PO₄ (84mg, 0.4mmol), dioxane (2.5mL), and water (0.5mL) were respectively filled into a 10 ml sealed tube. The reaction mixture was heated at 100°C for 4 h in an inert gas. After the reaction was completed, the reaction mixture was evaporated and concentrated with silica gel (100-200 mesh) to obtain a powder residue. The product was purified by automatic flash column chromatography (silica gel, MeOH:DCM=1:10), and the product was obtained as a white solid (20mg, yield 50%).

Molecular formula: C₁₉H₁₆F₃N₇, molecular weight: 399.38, (ESI) m/z=400.2 (M+H)⁺. ¹HNMR (400 MHz, DMSO-d₆) 10.89(s,1H),9.52(d, *J*=1.6Hz,1H), 8.82 (d, *J*=2.0Hz,1H ), 8.58-8.68 (m,4H),8.21-8.23 ( m,1H ), 7.53-7.56(m,1H), 4.92 (m, *J*=6.8Hz,1H),1.60-1.62(d, J=7.2Hz,6H) ppm.

### Example 6

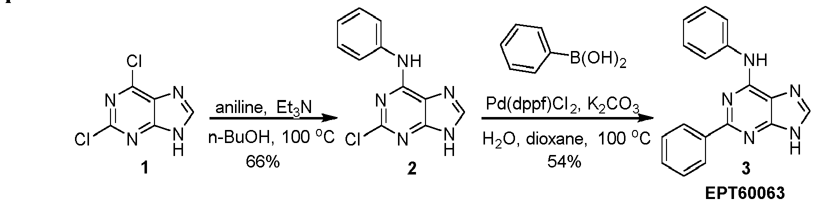

### Step 1: 2-chloro-N-phenyl-9H-purin-6-amine (2)

2,6-dichloro-9H-purine (2,589 mg, 3.11 mmol) and 10 mL of n-butanol were filled into a 25 mL round bottom flask. Et₃N (944 mg, 9.35 mmol) and aniline (348 mg, 3.74 mmol) were added. The reaction mixture was stirred at 100°C for 16 h (overnight), and LCMS analysis confirmed that the reaction was completed. Then the precipitate was filtered and washed with MeOH, and the product was obtained with the yield of 66% (505 mg), as an off-white solid. ESI m/z: 246.1 (M + H)⁺. ¹H NMR (DMSO-*d₆*, 400 MHz) *δ* 7.08 (d, *J* = 7.2 Hz, 1H), 7.36 (t, J = 7.6 Hz, 2H), 7.84 (d, *J* = 7.6 Hz, 2H), 8.30 (s, 1H), 10.18 (s, 1H), 13.29 (s, 1H) ppm.

### Step 2: N,2-diphenyl-9H-purin-6-amine (3, EPT60063)

A mixture of compound 2 (65 mg, 0.26 mmol) and dioxane (2.5 mL) and water (0.5 mL) was added to a 10 mL microwave tube. K₂CO₃ (108 mg, 0.78 mmol), phenylboronic acid (39 mg, 0.32 mmol) and Pd(dppf)Cl₂ (19 mg, 0.026 mmol) were added. The reaction mixture was stirred at 100°C under argon microwave for 4 hours. The completion of the reaction was confirmed by LCMS analysis. The mixture was cooled to room temperature (20°C), a precipitate was formed, which was filtrated and collected. The crude product was purified by silica gel column chromatography (DCM/MeOH=10/1) to obtain the desired white solid (38 mg, yield 54%). ESI m/z: 288.16 (M + H)⁺. ¹H NMR (DMSO-*d₆*, 400 MHz) *δ* 7.07 (d, *J* = 7.2 Hz, 1H), 7.40 (t, J = 7.6 Hz, 2H), 7.53-7.43 (m, 3H), 8.05 (d, *J* = 8.0 Hz, 2H), 8.30 (s, 1H), 8.39-8.37 (m, 2H), 9.84 (s, 1H), 13.20 (s, 1H) ppm.

### Example 7

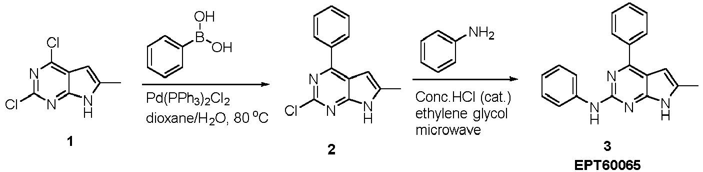

### Step 1: 2-chloro-6-methyl-4-phenyl-7H-pyrrolo[2,3-d]pyrimidine (2)

2,4-dichloro-6-methyl-7H-pyridine (1) (150 mg, 0.743 mmol), phenylboronic acid (145 mg, 1.19 mmol), potassium carbonate (513 mg, 3.71 mmol), bis(triphenylphosphine) palladium(II) chloride (84 mg, 0.12 mmol) in dioxane/water (10/1, 13 mL) was stirred under a nitrogen atmosphere at 80°C overnight. After cooling to room temperature, it was diluted with ethyl acetate (30ml) and water (30ml). The organic layer was separated, and the aqueous layer was extracted with ethyl acetate (30 ml). The synthesized organic layer was concentrated and purified using a C₁₈ column (acetonitrile/water concentration of 55%-60%) to obtain the title compound 2 (110 mg, yield 61%) as an off-white solid.

LC-MS [mobile phase: from 95% water and 5% CH₃CN to 5% water and 95% CH₃CN within 2.5 min], Rt = 1.64 min; MS calculated value: 243.1; MS measured value: 244.0 [M+H]⁺.

¹H NMR (400MHz, DMSO-d₆) δ 12.32 (s, 1H), 8.13-8.11 (m, 2H), 7.61-7.56 (m, 3H), 6.67 (s, 1H), 2.45 (s, 3H).

### Step 2: 6-methyl-N,4-diphenyl-7H-pyrrolo[2,3-d]pyrimidin-2-amine (3)

2-chloro-6-methyl-4-phenyl-7H-pyrrole[2,3-d]pyrimidine (2) (90 mg, 0.37 mmol) and aniline (103 mg, 1.11 mmol) were dissolved in ethylene glycol (3 mL), and a drop of concentrated hydrochloric acid aqueous solution was added. The mixture was stirred for 10 hours in a sealed tube under microwave and nitrogen atmosphere at 150°C. After cooling to room temperature, the mixture was poured into water (30ml), extracted with ethyl acetate (15ml×2), washed with brine (30ml) and concentrated. The residue was purified using a C₁₈ column (acetonitrile/water from 60% to 70%) to obtain the title compound (90 mg, yield 82%) as an off-white solid.

LC-MS purity: 95.84% (214 nm), 98.12% (254 nm); MS calculated value: 300.1; MS measured value: 301.0 [M+H]⁺.

¹H NMR (400 MHz, DMSO-*d*₆) δ 11.55 (s, 1H), 9.24 (s, 1H), 8.13 (d, *J* = 7.2 Hz, 2H), 7.90 (d, *J* = 7.6 Hz, 2H), 7.59-7.50 (m, 3H), 7.27 (t, *J* = 7.6 Hz, 2H), 6.87 (t, *J* = 7.6 Hz, 1H), 6.40 (s, 1H), 2.37 (s, 3H).

### Example 8

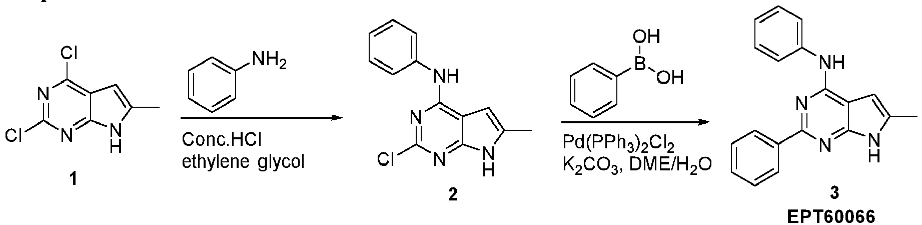

### Step1: 2-chloro-6-methyl-N-phenyl-7H-pyrrolo[2,3-d]pyrimidin-4-amine

To a solution of 2,4-dichloro-6-methyl-7H-pyrrole[2,3-d]pyrimidine(1) (100 mg, 0.495 mmol) and aniline (125 mg, 1.34 mmol) in ethylene glycol (4ml), four drops of concentrated aqueous hydrochloric acid were added. The mixture was stirred overnight at 100°C under a nitrogen atmosphere. After cooling to room temperature, the mixture was poured into water (30ml), extracted with ethyl acetate (15ml×2), washed with brine (30ml) and concentrated. The residue was purified with a C₁₈ column (acetonitrile/water from 50% to 60%) to obtain the title compound 2 (80 mg, yield 63%) as an off-white solid.

LC-MS [mobile phase: from 70% water and 30% CH₃CN to 5% water and 95% CH₃CN within 2.5min], Rt = 1.30 min; MS calculated value: 258.1; MS measured value: 259.0 [M+H]⁺.

¹H NMR (400 MHz, DMSO-*d*₆) δ 11.75 (s, 1H), 9.48 (s, 1H), 7.76-7.74 (dd, *J* = 8.4, 1.2 Hz, 2H), 7.36 (dd, *J=* 8.4, 3.2 Hz, 2H), 7.06 (t, *J=* 7.6 Hz, 1H), 6.40 (s, 1H), 2.34 (s, 3H).

### Step 2: 6-methyl-N,2-diphenyl-7H-pyrrolo[2,3-d]pyrimidin-4-amine

2-chloro-6-methyl-N-phenyl-7H-pyrrole[2,3-d]pyrimidin-4-amine (2) (130 mg, 0.502 mmol), phenylboronic acid (123 mg, 1.01 mmol) ), potassium carbonate (347 mg, 2.51 mmol) and tetravalent (triphenylphosphine) palladium (58 mg, 0.05 mmol) in 1,2-dimethoxyethane/water (5/1, 3.6 mL) were stired under microwave and nitrogen at 100°C for 3.5 hours. After cooling to room temperature, the mixture was poured into water (25ml), extracted with ethyl acetate (25ml×2), washed with brine (30ml) and concentrated. The residue was purified with a C₁₈ column (acetonitrile/water from 50% to 60%) to obtain the title compound (70 mg, yield 47%) as an off-white solid.

LC-MS (ESI): R_{T} = 2.253 min, MS calculated value C₁₉H₁₆N₄ 300.1, m/z measured value 301.0 [M+H]⁺. ¹H NMR (400 MHz, DMSO-d₆) δ 11.69 (s, 1H), 9.22 (s, 1H), 8.37 (d, *J* = 7.2 Hz, 2H), 7.98 (d, *J=* 7.6 Hz, 2H), 7.50-7.37 (m, 5H), 7.03 (t, *J=* 7.2 Hz, 1H), 6.49 (s, 1H), 2.39 (s, 3H).

### Example 9

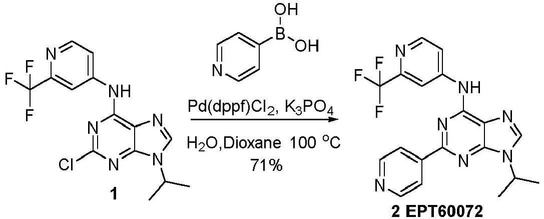

### Step 1: 9-isopropyl-2-(pyridin-4-yl)-N-(2-(trifluoromethyl)pyridin-4-yl)-9H-purin-6-amine (2 EPT60072)

2-chloro-9-isopropyl-N-(2-(trifluoromethyl)pyridin-4-yl)-9H-purin-6-amine (50mg, 0.14mmol), pyridine-4-yl-boronic acid (50mg, 0.40mmol), Pd(dppf)Cl₂(10mg, 0.014mmol), K₃PO₄(84mg, 0.4mmol) were added to a 10mL sealed tube, then dioxane (2.5mL) and water (0.5mL) were added. The mixture was heated at 100°C for 4 hours under an inert atmosphere. After the completion of the reaction, the reaction mixture was concentrated with silica gel (100-200 mesh) and evaporated to obtain a powder residue. The residue was purified by automated flash column chromatography (silica gel, MeOH:DCM=1:10) to obtain the title compound (40 mg, 71% yield) as a white solid.

Molecular formula: C₁₉H₁₆F₃N₇, molecular weight: 399.38, (ESI) m/z=400.2 (M+H)⁺. ¹HNMR (400 MHz, DMSO-d₆) 10.97(s,1H), 8.85(d, *J*=1.6Hz,1H), 8.77-8.79(dd,*J*₁=1.6Hz, *J*₂=14.8Hz,2H ), 8.66-8.68 (m,2H), 8.27-8.30 ( m,3H ), 4.9 (m, *J*=6.8Hz,1H),1.65(d, J=6.8Hz,6H) ppm.

### Example 10

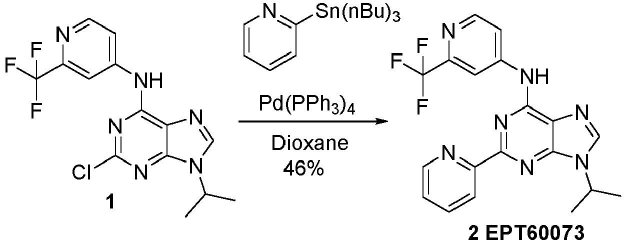

### Step 1: 9-isopropyl-2-(pyridin-2-yl)-N-(2-(trifluoromethyl)pyridin-4-yl)-9H-purin-6-amine (2 EPT60073)

2-chloro-9-isopropyl N-(2-(trifluoromethyl)pyridine-4-acyl)-9H-purin-6-amine (107mg, 0.30mmol), 2-(tributyltinyl)pyridine (310mg, 0.84mmol), Pd(PPh3)₄ (35mg,0.03 mmol), added with dioxane (5.0mL), were added to a 10-mL sealed tube. The reaction mixture was heated at 100°C in an inert gas for 8 h. After the completion of the reaction, the reaction mixture was quenched with saturated KF aqueous solution (2 mL), and the organic phase was concentrated and evaporated with silica gel (100-200 mesh) to obtain a powder residue. The product was purified by automatic flash column chromatography (silica gel, MeOH:DCM=1:19) to obtain the title compound (55mg, yield 46%) as a yellow solid.

Molecular formula: C₁₉H₁₆F₃N₇, molecular weight: 399.38, (ESI) m/z=400.2 (M+H)⁺. ¹H NMR (500 MHz, DMSO-d₆) 10.93(s,1H),9.22(d, *J*=2.5Hz,1H), 8.78-8.80 (m,1H),8.66 (s,1H),8.62(d, *J*=5.5Hz,1H),8.44(d, *J*=8.0Hz,1H), 8.35-8.36(m,1H), 7.98-8.02(m,1H), 7.51-7.55(m,1H), 4.98 (m, 1H), 1.63-1.65(d, J=7.0Hz,6H) ppm.

### Example 11

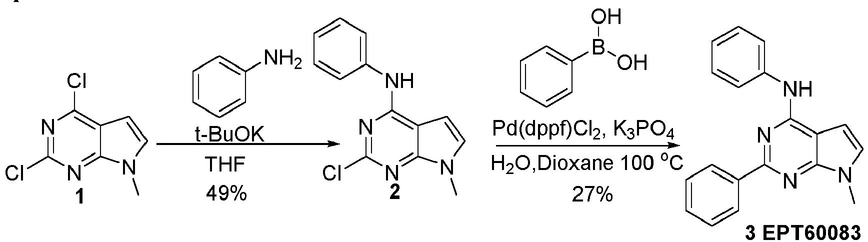

### Step 1: 2-chloro-7-methyl-N-phenyl-7H-pyrrolo[2,3-d]pyrimidin-4-amine (2)

2,4-dichloro-7-methyl-7H-pyridine[2,3-d]pyrimidine (101 mg, 0.50mmol), t-BuOK (85 mg, 0.75mmol), aniline (70mg, 0.75mmol) were resepectively filled into a 20 mL sealed tube, and THF (5 mL) was added. The reaction mixture was stirred at rt condition for 1.5h. After the reaction was completed, the reaction mixture was evaporated and concentrated with silica gel (100-200 mesh) to obtain a powder residue. The product was purified by automatic flash column chromatography (silica gel, PE: EA=7:3), and the product was obtained as a white solid (63 mg, yield 49%).

Molecular formula: C₁₃H₁₁ClN₄, molecular weight: 258.71, (ESI) m/z=259.1 (M+H)⁺.

### Step 2: 7-methyl-N,2-diphenyl-7H-pyrrolo[2,3-d]pyrimidin-4-amine (3 EPT60083)

2-chloro-7-methyl-N-phenyl-7H-pyrrolo[2,3-d)pyrimidin-4-amine (63mg, 0.25mmol), phenylboronic acid (45mg, 0.37mmol), Pd(dppf)Cl₂(18mg,0.025mmol), K₃PO₄ (110mg, 0.5mmol), dioxane (5mL), and water (1mL) were filled into a sealed tube. The reaction mixture was heated at 100°C for 2h in an inert gas. After the reaction was completed, the reaction mixture was evaporated and concentrated with silica gel (100-200 mesh) to obtain a powder residue. The product was purified by automatic flash column chromatography (silica gel, PE: EA=7:3), and the product was obtained as a white solid (20 mg, yield 27%).

Molecular formula: C₁₉H₁₆N₄, molecular weight: 300.37, (ESI) m/z=301.2 (M+H)⁺. ¹H NMR (500 MHz, DMSO-d₆) 9.43 (s,1H), 8.43-8.45 (m,2H), 7.99( d, *J*=7.5Hz,2H ), 7.39-7.51(m,5H), 7.32( d, *J*=3.5Hz,1H ),7.06(t, *J*=7.5Hz,1H), 6.83( d, *J*=3.5Hz,1H ),3.84(s,3H) ppm.

### Example 12

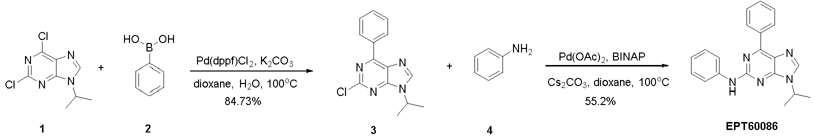

### Step 1 2-chloro-9-isopropyl-6-phenyl-9H-purine (3)

2,6-dichloro-9-isopropyl-9H-purine (1,100 mg, 0.43 mmol), phenylboronic acid (2,63 mg, 0.52 mmol), Pd(dppf)Cl₂(3 mg, 0.0043 mmol) and K₂CO₃(120 mg, 0.86 mmol) were respectively put into a 25 ml round bottom flask. The mixture was suspended in dioxane (5ml) and H₂O (1ml). The reaction was stirred at 100°C under a nitrogen atmosphere for 2 h. The solvent was removed under reduced pressure and purification was performed by automatic flash column chromatography (silica gel, PE:EA=5:1) to obtain 2-chloro-9-isopropyl-6-phenyl-9H-purine (3,100 mg, 84.73% yield) as a white solid.

LCMS: (ESI) m/z=273.08 (M+H)⁺; RT=1.75min.

### Step 2: 9-isopropyl-N,6-diphenyl-9H-purin-2-amine (EPT60086)

A 25 ml round bottom flask was added with 2-chloro-9-isopropyl-6-phenyl-9H-purine (330 mg 0.108 mmol), aniline (415.6 mg 0.162 mmol), Pd (OAc) 2 (0.24 mg , 1.08µmol) BINAP (1.2mg, 2.16µmol) and Cs₂CO₃ (108mg, 0.33mmol). The mixture was suspended in dioxane (5ml). The reaction was stirred at 100°C under a nitrogen atmosphere for 2 h. The solvent was removed under reduced pressure and purification was performed by automatic flash column chromatography (silica gel, PE:EA=5:1) to obtain 9-isopropyl n,6-diphenyl-9H-purin-2-amine (EPT60086, 20 mg , 55.2% yield) as a yellow solid.

LCMS: (ESI) m/z=330.21 (M+H)⁺; RT=1.84 min.

¹H NMR (500 MHz, DMSO) δ 9.59 (s, 1H), 8.81 (dd, *J* = 8.1, 1.5 Hz, 2H), 8.43 (s, 1H), 7.91 (d, *J* = 7.7 Hz, 2H), 7.62 - 7.54 (m, 3H), 7.35 - 7.30 (m, 2H), 6.95 (t, *J* = 7.3 Hz, 1H), 4.81 (s, 1H), 1.61 (d, *J* = 6.8 Hz, 6H).

### Example 13

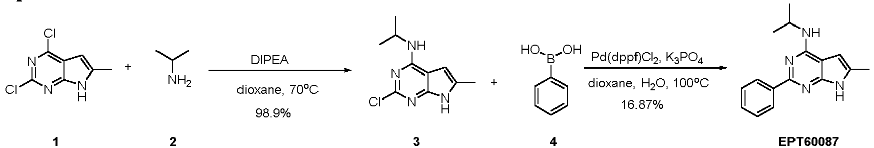

### Step 1: 2-chloro-N-isopropyl-6-methyl-7H-pyrrolo[2,3-d]pyrimidin-4-amine (3)

2,4-dichloro-6-methyl-7H-pyrrole[2,3-d]pyrimidine (1,202 mg, 1 mmol), propaN-2-amine (2,89 mg, 1.5 mmol) and DIPEA (258 mg, 2 mmol) were added to a 25-ml round bottom flask. The reaction mixture was dissolved in dioxane at 70 °C (5 ml) and stirred for 2 h. After extraction with EA (10ml x 3), it was washed with brine (10ml x 3), dried on anhydrous sodium sulfate and concentrated in a vacuum. 2-chloro-N-isopropyl-6-methyl-7H-pyrrolo[2,3-d]pyrimidine-N-4-amine (220 mg, 98.9% yield) was obtained as a brown solid.

LCMS: (ESI) m/z=225.18 (M+H)⁺; RT=1.44min.

### Step 2: N-isopropyl-6-methyl-2-phenyl-7H-pyrrolo[2,3-d]pyrimidin-4-amine (EPT60087)

2-chloro-N-isopropyl-6-methyl-7H-pyridinol[2,3-d] 4-aminopyridine (3,50 mg, 0.22 mmol), phenylboronic acid (4,32 mg, 0.27 mmol), Pd(dppf)Cl₂(2 mg, 0.0022 mmol) and K₃PO₄(93 mg, 0.44 mmol) were respectively put into a 25 ml round bottom flask. The mixture was suspended in dioxane (5ml) and H₂O (1ml). The reaction was stirred for 2 hours under a nitrogen atmosphere at 100°C. The solvent was removed under reduced pressure and purification was performed by automatic flash column chromatography (silica gel, PE:EA=1:1) to obtain N-isopropyl-6-methyl-2-phenyl-7H-pyrrolo[2,3-d] pyrimidine-4-amine (EPT60087, 10 mg, 16.87% yield) as a yellow solid.

LCMS: (ESI) m/z=267.2 (M+H)⁺; RT=1.30min.

¹H NMR (500 MHz, DMSO) δ 11.35 (s, 1H), 8.36 - 8.33 (m, 2H), 7.45 - 7.35 (m, 3H), 6.95 (d, *J* = 7.6 Hz, 1H), 6.25 (dd, *J* = 1.9, 1.0 Hz, 1H), 4.51 (d, *J* = 6.9 Hz, 1H), 2.32 (d, J = 0.7 Hz, 3H), 1.28 (d, *J=* 6.5 Hz, 6H).

### Example 14

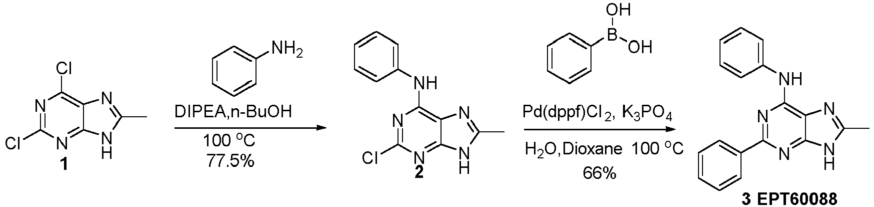

### Step 1: 2-chloro-8-methyl-N-phenyl-9H-purin-6-amine (2)

A 20 ml sealed tube was filled with 2,6-dichloro-8-methyl-9H-purine (101 mg, 0.50 mmol) and 5 ml n-butanol. DIPEA (129 mg, 1.0 mmol) and aniline (70 mg, 0.75 mmol) were added. The reaction mixture was stirred at 100°C for 4 h. After the reaction was completed, the reaction mixture was evaporated and concentrated with silica gel (100-200 mesh) to obtain a powder residue. The product was purified by automatic flash column chromatography (silica gel, MeOH: DCM=1:10), and the product was obtained as a white solid (100 mg, 77.5% yield).

Molecular formula: C₁₂H₁₀ClN₅, molecular weight: 259.70, (ESI) m/z=260.1 (M+H)⁺.

### Step 2: 8-methyl-N,2-diphenyl-9H-purin-6-amine (3 EPT60088)

2-chloro-8-methyl-N-phenyl-9H-purin-6-amine (50mg, 0.2mmol), phenylboronic acid (125mg, 1.0mmol), Pd(dppf)Cl₂(30mg,0.04mmol), K₃PO₄ (212mg, 1.0mmol) were respectively filled into a 10-mL sealed tube, and then 2,2,5 ml of dioxane and 1mL of water were added. The reaction mixture was heated at 100°C in an inert gas for 22 h. After the reaction was completed, the reaction mixture was evaporated and concentrated with silica gel (100-200 mesh) to obtain a powder residue. The product was purified by automatic flash column chromatography (silica gel, DCM: MeOH=19:1) to obtain the title compound (40 mg, yield 66%) as a yellow solid.

Molecular formula: C₁₈H₁₅N₅, molecular weight: 301.35, (ESI) m/z=3020.2 (M+H)⁺. ¹H NMR (400 MHz, DMSO-d₆) 12.91 (s,1H),9.67 (s,1H), 8.33 (d, J=7.2Hz,2H), 8.02(d, J=8.0Hz,2H), 7.32-7.48 (m,5H), 7.01 (m,1H), 2.50 (s, 3H) ppm.

### Example 15

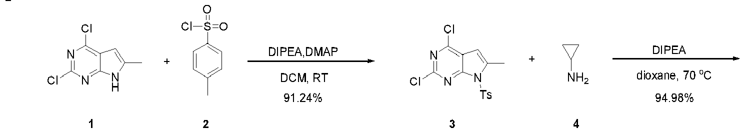

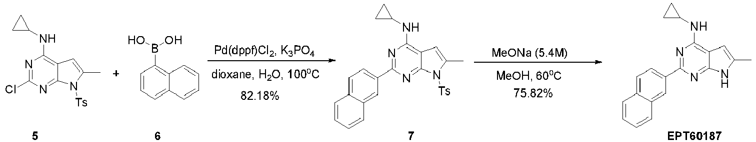

### Step 1: 2,4-dichloro-6-methyl-7-tosyl-7H-pyrrolo[2,3-d]pyrimidine (3)

2-chloro-N-isopropyl-6-methyl-7H-pyrrolo[2,3-d]pyrimidine-N-4-amine (500 mg, 2.47 mmol), tosyl chloride (705 mg, 3.71 mmol) DIPEA (957 mg, 7.42 mmol) and DMAP (30 mg, 0.25 mmol) were added to a 25 ml round-bottom flask. The reaction mixture was dissolved in DCM (5 mL) and stirred for 2 h RT. The residue was concentrated in vacuum and purified by automatic flash column chromatography (silica gel, PE:EA=1:1) to provide 2,4-dichloro-6-methyl-7-toluenesulfonyl-7H-pyridine [2,3-d] pyrimidine (3,800 mg, 91.24% yield) as a yellow solid. LCMS: (ESI) m/z=356.06 (M+H)⁺; RT=1.88min.

### Step 2: 2-chloro-N-cyclopropyl-6-methyl-7-tosyl-7H-pyrrolo[2,3-d]pyrimidin-4-amine (5)

2,4-dichloro-6-methyl-7-tosyl-7H-pyridine [2,3-d] (3,100 mg, 0.28 mmol), cyclopropylamine (4,31 mg, 0.56 mmol) and DIPEA (108 mg, 0.84 mmol) were added to a 25 ml round bottom flask. The reaction mixture was dissolved in dioxane (5ml), and stirred at 70°C for 4h. The residue was concentrated in vacuum, and purification was performed by automatic flash column chromatography (silica gel, PE:EA=85:15) to obtain 2-chloro-N-ring propyl-6-methyl-7-tosyl-7H-pyrrolo[2,3-d]pyrimidin-4-amine (5,100 mg, 94.98% yield) as a white solid. LCMS: (ESI) m/z=377.89(M+H)⁺; RT=1.76min.

### Step 3: N-cyclopropyl-6-methyl-2-(naphthalene-2-yl)-7-tosyl-7H-pyrrolo[2,3-d]pyrimidin-4-amine (7)

2-chloro-N-cyclopropyl-6-methyl-7-tosyl-7-h-pyrrolo[2,3-d]pyrimidin-4-amine (5,100 mg , 0.26 mmol), naphth-1-ylboronic acid (6,134 mg, 0.78 mmol), Pd(dppf)Cl₂(19 mg, 0.026 mmol) and K₃PO₄(165 mg, 0.78 mmol) were added to a 25 ml round bottom flask. The mixture was suspended in dioxane (5ml) and H₂O (1ml). The reaction was carried out overnight under a nitrogen atmosphere at 100°C. The product was a yellow solid N-cyclopropyl-6-methyl-2-(naphthalene-2-yl)-7-tosyl-7H-pyrrolo[2,3-d]pyrimidin-4-amine (7,100 mg, 82.18% yield).

LCMS: (ESI) m/z=469.29 (M+H)⁺; RT=2.18 min

### Step 4: N-cyclopropyl-6-methyl-2-(naphthalene-2-yl)-7H-pyrrolo[2,3-d]pyrimidin-4-amine (EPT60187)

In a 25 ml round bottom flask, N-cyclopropyl-6-methyl-2-(naphthalene-N-2-yl)₇-methylsulfonyl-7H-pyrrolo[2,3-d]pyrimidine-N-4-amine (100 mg, 0.21 mmol) dissolved in MeONa (2 mL, 5.4 M in methanol) and methanol (10 mL) was added and stirred at 60°C for 4 h. The residue was concentrated in vacuum and purified by automatic flash column chromatography (silica gel, PE: EA = 5:1) to obtain N-cyclopropyl-6-methyl-2-(naphthalen-2-yl)-7H-pyrrolo[ 2,3-d]pyrimidin-4-amine (EPT60187 50 mg, yield 75.82%) as a white solid.

LCMS: (ESI) m/z=315.21 (M+H)⁺; RT=1.51min.

¹H NMR (500 MHz, DMSO) δ 11.49 (s, 1H), 8.89 (s, 1H), 8.55 (d, *J=* 8.4 Hz, 1H), 8.04 - 7.89 (m, 3H), 7.57 - 7.48 (m, 2H), 7.43 (d, *J=* 3.1 Hz, 1H), 6.32 (s, 1H), 3.08 (s, 1H), 2.35 (s, 3H), 0.88 - 0.83 (m, 2H), 0.66 - 0.60 (m, 2H).

### Example 16

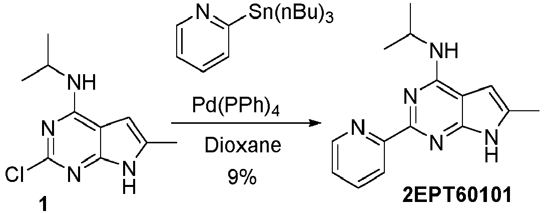

### Step 1: N-isopropyl-6-methyl-2-(pyridin-2-yl)-7H-pyrrolo[2,3-d]pyrimidin-4-amine (2 EPT60101)

2-chloro-N-isopropyl-6-methyl-7H-pyrrole[2,3-d]pyrimidin-4-amine (100mg, 0.44mmol), 2-(tributylstannyl)pyridine (300mg, 0.81 mmol), Pd(PPh3)₄ (50mg,0.04mmol), added with dioxane (5.0mL), were filled into a 10-mL sealed tube. The reaction mixture was heated at 100°C in an inert gas for 16 h. After the completion of the reaction, the reaction mixture was quenched with saturated KF aqueous solution (2 mL), and the organic phase was concentrated and evaporated with silica gel (100-200 mesh) to obtain a powder residue. The residue was purified by automatic flash column chromatography (silica gel, MeOH: DCM=1:19) to obtain the title compound (12 mg, yield 9%) as a yellow solid.

Molecular formula: C₁₅H₁₇N₅, molecular weight: 267.34, (ESI) m/z=268.2 (M+H)⁺. ¹H NMR (400 MHz, DMSO-d₆) 11.37(s,1H)₈.60 (m,1H), 8.28 (d, *J*=8.0Hz,1H), 7.80-7.84 (m,1H), 7.31-7.34 (m,1H), 6.95 (d, *J*=8.0Hz,1H),6.26(s,1H),4.46-4.52 (m,1H),2.30(s,3H),1.29(m,6H) ppm.

### Example 17

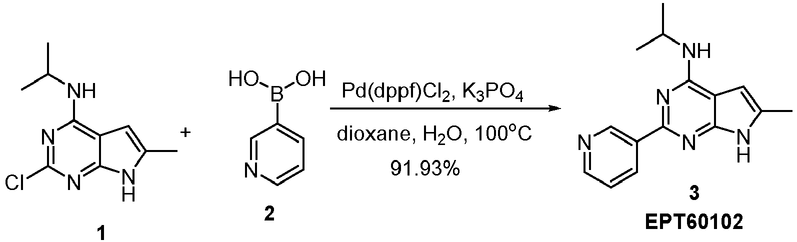

### Step 1: N-isopropyl-6-methyl-2-(pyridin-3-yl)-7H-pyrrolo[2,3-d]pyrimidin-4-amine (EPT60102)

2-chloro-N-isopropyl-6-methyl-7H-pyridin-4-amine (1,50 mg, 0.22 mmol), pyridin-3-ylboronic acid (2,81 mg, 0.66 mmol), Pd(dppf)Cl₂(16 mg, 0.022 mmol) and K₃PO₄(93 mg, 0.44 mmol) were respectively filled into a 25 ml round bottom flask. The mixture was suspended in dioxane (5ml) and H₂O (1ml). The reaction was stirred overnight at 100°C under a nitrogen atmosphere. The solvent was removed under reduced pressure and purification was performed by automatic flash column chromatography (silica gel, DCM:MeOH=95:5) to obtain N-isopropyl-6-methyl-2-(pyridine-3-acyl)-7H-pyridine-4-amine (EPT60102, 54 mg, 91.93% yield) as a yellow solid.

LCMS: (ESI) m/z=268.28 (M+H)⁺; RT=1.21min.

¹H NMR (500 MHz, DMSO) δ 11.46 (s, 1H), 9.47 (d, *J* = 1.4 Hz, 1H), 8.60 - 8.54 (m, 2H), 7.49 - 7.44 (m, 1H), 7.08 (d, *J* = 7.6 Hz, 1H), 6.28 (d, *J* = 0.8 Hz, 1H), 4.51 (dq, *J* = 13.1, 6.6 Hz, 1H), 2.33 (s, 3H), 1.28 (d, *J* = *6.5* Hz, 6H).

### Example 18

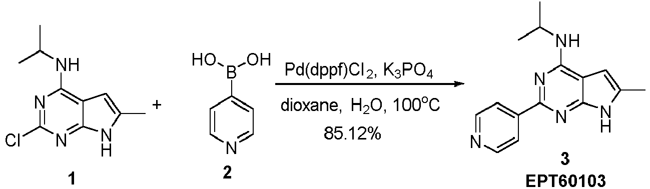

### Step 1: N-isopropyl-6-methyl-2-(pyridin-4-yl)-7H-pyrrolo[2,3-d]pyrimidin-4-amine (EPT60103)

2-chloro-N-isopropyl-6-methyl-7H-pyridin-4-amine (1,50 mg, 0.22 mmol), pyridine-4-ethylboronic acid (2,81 mg, 0.66 mmol), Pd(dppf)Cl₂(16 mg, 0.022 mmol) and K₃PO₄(93 mg, 0.44 mmol) were put into a 25 ml round bottom flask. The mixture was suspended in dioxane (5ml) and H₂O (1ml). The reaction was stirred overnight at 100°C under a nitrogen atmosphere. The solvent was removed under reduced pressure and purification was performed by automated flash column chromatography (silica gel, DCM:MeOH=95:5) to obtain N-isopropyl-6-methyl-2-(pyridine-4-acyl)-7H-pyridine-4-amine (EPT60103, 50 mg, 85.12% yield) as a yellow solid.

LCMS: (ESI) m/z=268.28 (M+H)⁺; RT=1.21min.

¹H NMR (500 MHz, DMSO) δ 11.54 (s, 1H), 8.64 (dd, *J* = 4.5, 1.5 Hz, 2H), 8.20 (dd, *J* = 4.5, 1.6 Hz, 2H), 7.13 (d, *J* = 7.6 Hz, 1H), 6.31 (d, *J* = 0.8 Hz, 1H), 4.52 (dd, *J* = 13.5, 6.7 Hz, 1H), 2.34 (s, 3H), 1.28 (d, *J=* 6.5 Hz, 6H).

### Example 19

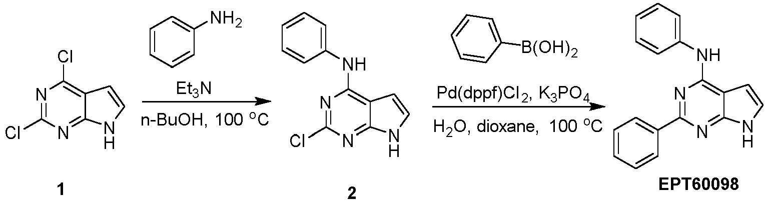

### Step 1: 2-chloro-N-phenyl-7H-pyrrolo[2,3-d]pyrimidin-4-amine (2)

A solution of Compound 1 (100 mg, 0.54 mmol) in n-butanol (5 mL) was added with Et₃N (86.9 mg, 0.86 mmol) and aniline (60.5 mg, 0.65 mmol). The reaction mixture was stirred at 100°C for 16 hours (overnight). LCMS (EPN18040-002-1) showed that the reaction was completed and 10% of SM remained. The solvent was removed in vacuum. The unpurified crude compound was used directly in the next step. (ESI) m/z=245.31 (M+H)⁺

### Step 2: N,2-diphenyl-7H-pyrrolo[2,3-d]pyrimidin-4-amine (EPT60098)

A solution of Compound 2 (60 mg, 0.25 mmol) in dioxane (5 mL) and H₂O (1 mL) was added with Pd(dppf)Cl₂ (36.6 mg, 0.05 mmol) and K₃PO₄ (182.6 mg, 0.86 mmol) and phenylboronic acid (149.5 mg, 1.23 mmol) respectively. The reaction mixture was stirred at 100°C for 16 h (overnight). LCMS (EPN18040-005-1) under Ar showed that the reaction was completed and 10% of SM remained. The solvent was concentrated in vacuo. The residue was purified by automatic flash column chromatography (silica gel, PE/EA=8:1) to obtain the crude product. The residue was diluted with saturated K₂CO₃ solution (100 mL), the mixture was extracted with CH₂Cl₂ (50 mL×3), and then washed with saturated NaCl (100 mL). The obtained organic layer was dried with anhydrous Na₂SO₄, and the solvent was removed in vacuum to obtain the desired product (40 mg, 57.1% yield) as a white solid. (ESI) m/z=287.12 (M+H)⁺. 1H NMR (500 MHz, DMSO-*d*₆) 11.82 (s, 1 H), 9.40 (s, 1 H), 8.38-8.40 (m, 2 H), 7.99-8.01 (m, 2 H), 7.48-7.51 (m, 2 H), 7.40-7.45 (m, 3 H), 7.27-7.28 (m, 1 H), 7.04-7.07 (m, 1 H), 6.82-6.83 (m, 1 H) ppm.

### Example 20

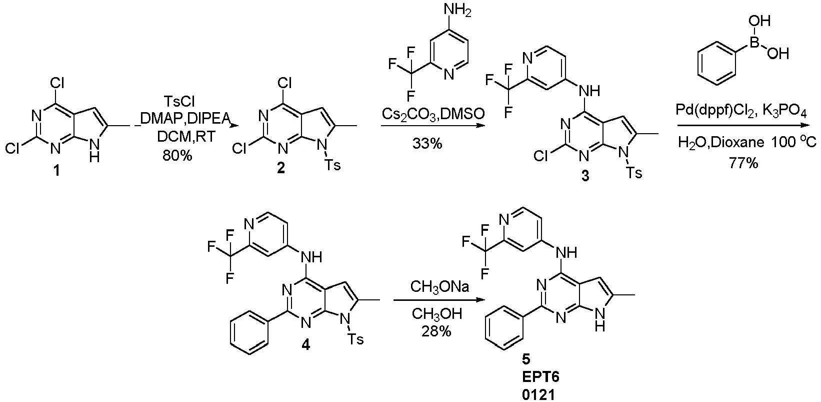

### Step 1: 2,4-dichloro-6-methyl-7-tosyl-7H-pyrrolo[2,3-d]pyrimidine (2)

A 50ml round bottom flask was added with 2,4-dichloro-6-methyl-7H-pyridine[2,3-d] (400mg, 2.0mmol), 4-methylbenzenesulfonyl chloride (570mg, 3.0mmol), DMAP (24mg, 0.2mmoL), DIPEA (1mL), and then DCM (20mL). The reaction mixture was stirred at room temperature for 1 h. After the reaction was completed, the reaction mixture was evaporated and concentrated with silica gel (100-200 mesh) to obtain a powder residue. The product was purified by automatic flash column chromatography (silica gel, EA: PE = 1:10) to obtain the title compound (565 mg, yield 80%) as a white solid.

Molecular formula: C₁₄H₁₁Cl₂N₃O₂S, molecular weight: 356.22, (ESI) m/z=3 5 6.1 (M+H)⁺.

### Step 2: 2-chloro-6-methyl-7-tosyl-N-(2-(trifluoromethyl)pyridin-4-yl)-7H-pyrrolo[2,3-d]pyrimidin e-4 -Amine (3)

2,4-dichloro-6-methyl-7-tosyl-7H-pyrroline[2,3-d]pyrimidine (140mg, 0.4mmol), Cs₂CO₃ (156 mg, 0.48mmol), 2-(trifluoromethyl)pyridine-4-amine (100mg, 0.60mmol), added with DMSO (4ml), were filled into a 20 mL sealed tube. The reaction mixture was stirred at rt for 18h and at 70°C for 2h. The reaction was monitored by LCMS. After the reaction was completed, water (20 mL) was added, and the reaction mixture was extracted with EA (20 mL 2). The organic phase was evaporated and concentrated using silica gel (100-200 mesh) to obtain a powder residue. The residue was purified by automatic flash column chromatography (silica gel, EA:PE=3:2) to obtain the title compound (64mg, yield 33%) as a white solid. Molecular formula: C₂₀H₁₅ClF₃N₅O₂S, molecular weight: 481.88, (ESI) m/z=482.2 (M+H)⁺.

### Step 3: 6-methyl-2-phenyl-7-tosyl-N-(2-(trifluoromethyl)pyridin-4-yl)-7H-pyrrolo[2,3-d]pyrimidi ne- 4-amine (4)

2-chloro-6-7-toluenesulfonyl-N-(2-(trifluoromethyl)pyridine-4-acyl)-7H-pyrroline [2,3-d]phenylboronic acid (12mg , 0.1mmol), Pd(dppf)Cl₂ (5mg,0.008mmol), K₃PO₄ (30mg, 0.15mmol), added with dioxane (2mL) and water (0.4mL), were filled into a 10-mL sealed tube. The reaction mixture was heated to 100c in an inert gas and heated for 1.5h. After the reaction was completed, the reaction mixture was evaporated and concentrated with silica gel (100-200 mesh) to obtain a powder residue. The product was purified by automatic flash column chromatography (silica gel, EA: PE =1:1) to obtain the title compound (20 mg, yield 77%) as a white solid.

Molecular formula: C₂₆H₂₀F₃N₅O₂S, molecular weight: 523.53,(ESI) m/z=524.3(M+H)⁺.

### Step 4: 6-methyl-2-phenyl-N-(2-(trifluoromethyl)pyridin-4-yl)-7H-pyrrolo[2,3-d]pyrimidin-4-ami ne (5 EPT60121 )

6-methyl-2-phenyl-7-toluenesulfonyl-N-(2-(trifluoromethyl)pyridine-4-yl)-7H-pyrrole[2, 3-d]pyrimidin-4-amine (20 mg, 0.04mmol) was dissolved in CH₃OH (3mL), and CH₃ONa (5.4mol/L,0.5mL) was added. The reaction mixture was stirred at 55°C for 2h. After the completion of the reaction, the reaction was quenched with saturated NH₄Cl aqueous solution (1mL), and the reaction was concentrated and evaporated to obtain oily residue, which was purified by reversed-phase column chromatography (C₁₈, H₂O/MeCN =2/3) to obtain the title compound EPN18033-070-A (4mg, 28% yield) as a white solid. Molecular formula: C₁₉H₁₄F₃N₅, molecular weight: 369.35, (ESI) m/z=370.2(M+H)⁺. ¹H NMR (400 MHz, DMSO-d₆) 11.92 (s,1H), 10.05 (s, 1H), 8.84 (d, J=1.6 Hz,1H), 8.58 (d,J=5.6 Hz,1H), 8.34 (d, J=6.8 Hz, 2H), 8.06 (m, 1H), 7.41-7.49 (m, 3H) , 6.52(s, 1H), 2.29 (d, J=3.6 Hz, 3H) ppm.

### Example 21

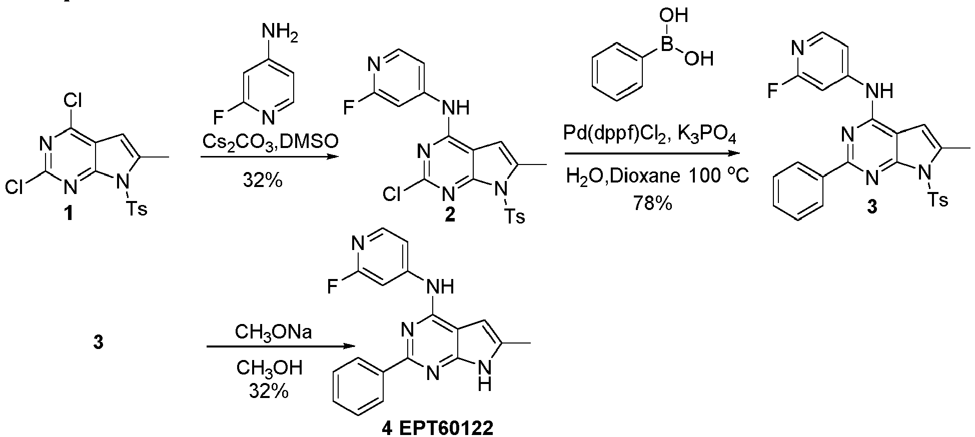

### Step 1: 2-chloro-N-(2-fluoropyridin-4-yl)-6-methyl-7-tosyl-7H-pyrrolo[2,3-d]pyrimidin-4-amine(2 )

2,4-dichloro-6-methyl-7-tosyl-7H-pyridine[2,3-d]pyrimidine (66 mg, 0.2mmol), Cs₂CO₃ (65 mg, 0.2mmol), 2-fluoropyridin -4-amine (45mg, 0.40mmol) were respectively filled into a 20ml sealed tube, and DMSO (2 mL) was added. The reaction mixture was stirred at rt for 18h and at 70°C for 2h. After the reaction was completed, the reaction mixture was evaporated and concentrated with silica gel (100-200 mesh) to obtain a powder residue. The product was purified by automatic flash column chromatography (silica gel, EA:PE=3:2) to obtain the title compound (26 mg, yield 32%) as a white solid.

Molecular formula: C₁₉H₁₅ClFN₅O₂S, molecular weight: 431.87 (ESI) m/z=432.2(M+H)⁺.

### Step 2: N-(2-fluoropyridin-4-yl)-6-methyl-2-phenyl-7-tosyl-7H-pyrrolo[2,3-d]pyrimidin-4-amine (3)

2-chloro-N-(2-fluoropyridine-4-acyl)-6-methyl-7-tosyl-7H-pyrrolo[2,3-d]pyridine-4-amin e (26mg, 0.06mmol ), phenylboronic acid (12mg, 0.1mmol), Pd(dppf)Cl₂(5mg,0.008mmol), K₃PO₄ (30mg, 0.15mmol), dioxane (3mL), and water (0.5mL) were filled into a 10-mL sealed tube. The reaction mixture was heated to 100°C in an inert gas for 5 h. After the reaction was completed, the reaction mixture was evaporated and concentrated with silica gel (100-200 mesh) to obtain a powder residue. The product was purified by automatic flash column chromatography (silica gel, EA: PE = 1:4) to obtain the title compound (22 mg, yield 78%) as a white solid.

Molecular formula: C₂₅H₂₀FN₅O₂S, molecular weight: 473.53, (ESI) m/z=474.3(M+H)⁺.

### Step 3: N-(2-fluoropyridin-4-yl)-6-methyl-2-phenyl-7H-pyrrolo[2,3-d]pyrimidin-4-amine (4 EPT60122)

N-(2-fluoropyridine-4-acyl)-6-methyl-2-phenyl-7-tosyl-7H-pyrroline[2,3-d]pyrimidin-4-a mine (22 mg, 0.046mmol) was dissolved in CH₃OH (3mL), and CH₃ONa (5.4mol/L,0.5mL) was added. The reaction mixture was stirred at 60°C for 2h. After the reaction was completed, the reaction was quenched with saturated NH₄Cl aqueous solution (1mL), and the reaction was concentrated and evaporated to obtain oily residue. The product was purified by reversed-phase column chromatography (C₁₈, H₂O/MeCN =2/3) as a white solid (5mg, yield 32%).

Molecular formula: C₁₈H₁₄FN₅, molecular weight: 319.34 (ESI) m/z=320.2(M+H)⁺. ¹H NMR (400 MHz, DMSO-d₆) 11.90 (s,1H), 9.92(s,1H), 8.31 (¹³C). d, J=7.2Hz,2H),8.07(d, *J*=5.6Hz,1H), 7.93(s,1H), 7.74(d, *J*=4.8Hz,1H), 7.42-7.51(m,3H), 6.52(s,1H), 2.38(s,3H) ppm.

### Example 22

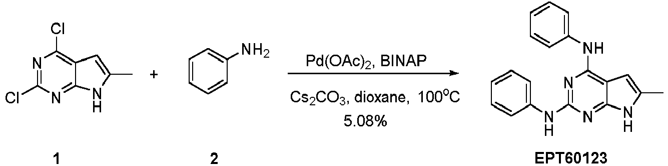

### Step 1: 6-methyl-N₂,N₄-diphenyl-7H-pyrrolo[2,3-d]pyrimidine-2,4-diamine (EPT60123)

2,4-dichloro-6-methyl-7H-pyrrolo[2,3-d]pyrimidine (0.25 1 50 mg mmol), aniline (69 mg, 0.74 mmol), Pd (OAc) 2 (2.8 mg, 12.5 µmol) BINAP (15 mg, 25 µmol) and Cs₂CO₃ (245 mg, 0.75 mmol) were added to a 25 ml round bottom flask. The mixture was suspended in dioxane (2ml). The reaction was stirred at 100°C under a nitrogen atmosphere for 4 h. The solvent was removed under reduced pressure, and purification was performed by automatic flash column chromatography (silica gel, DCM:MeOH=95:5) to provide the crude product, and then reversed-phase column chromatography (NH4HCO3, aq, 0.5%) was used to obtain a white solid 6-methyl-N2,N4-diphenyl-7H-pyrrolo[2,3-d]pyrimidine-2,4-diamine (EPT60123, 4 mg, 5.08% yield).

LCMS: (ESI) m/z=316.25 (M+H)⁺; RT=1.42 min.

¹H NMR (500 MHz, DMSO) δ 11.07 (s, 1H), 8.94 (s, 1H), 8.75 (s, 1H), 7.91 (d, *J* = 7.7 Hz, 2H), 7.81 (d, *J* = 7.7 Hz, 2H), 7.31 (t, *J* = 7.9 Hz, 2H), 7.21 (t, *J* = 7.9 Hz, 2H), 6.99 (t, *J* = 7.3 Hz, 1H), 6.84 (t, *J=* 7.3 Hz, 1H), 6.31 (s, 1H), 2.29 (s, 3H).

### Example 23

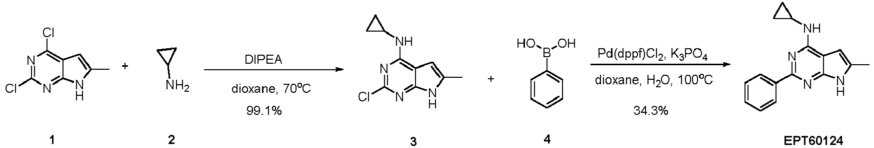

### Step 1: N-cyclopropyl-6-methyl-2-phenyl-7H-pyrrolo[2,3-d]pyrimidin-4-amine (3)

2,4-dichloro-6-methyl-7H-pyridine[2,3-d] (100 mg, 0.5 mmol), cyclopropylamine (2,42 mg, 0.75 mmol) and DIPEA (129 mg, 1 mmol) were added to a 25 ml round bottom flask. The reaction mixture was dissolved in dimethoxy (2ml), stirred at 70°C for 2h, extracted with EA (10ml x 3), washed with brine (10ml x 3), dried over anhydrous sodium sulfate, and concentrated to obtain 2-chloro-N-cyclopropyl-6-methyl-7H-pyridin-4-amine (3,110 mg, yield 99.1%) as a yellow solid.

LCMS: (ESI) m/z=223.22 (M+H)⁺; RT=1.32min.

### Step 2: N-cyclopropyl-6-methyl-2-phenyl-7H-pyrrolo[2,3-d]pyrimidin-4-amine (EPT60124)

2-chloro-N-cyclopropyl-6-methyl-7H-pyridinol[2,3-d] 4-aminopyridine (3,50 mg, 0.22 mmol), phenylboronic acid (4,80 mg, 0.66 mmol), Pd(dppf)Cl₂(16 mg, 0.022 mmol) and K₃PO₄(93 mg, 0.44 mmol) were respectively put into a 25 ml round bottom flask.

The mixture was suspended in dioxane (5ml) and H₂O (1ml). The reaction was carried out overnight under a nitrogen atmosphere at 100°C. The solvent was removed under reduced pressure, and purification was performed by automatic flash column chromatography (silica gel, DCM:MeOH=95:5) to provide the crude product, and then reversed-phase column chromatography (NH₄HCO₃, a.q. 0.5%):MeCN=70:30 was used to obtain a white solid N-cyclopropyl-6-methyl-2-phenyl-7H-pyrrolo[2,3-d]pyrimidin-4-amine (EPT60124, 20 mg, 34.3% yield). LCMS: (ESI) m/z=265.24 (M+H)⁺; RT=1.20min.

¹H NMR (500 MHz, DMSO) δ 11.41 (s, 1H), 8.37 (d, *J* = 7.1 Hz, 2H), 7.46 - 7.30 (m, 4H), 6.28 (s, 1H), 3.02 (dt, *J=* 10.0, 3.3 Hz, 1H), 2.32 (d, *J=* 0.7 Hz, 3H), 0.84 - 0.78 (m, 2H), 0.65 - 0.55 (m, 2H).

### Example 24

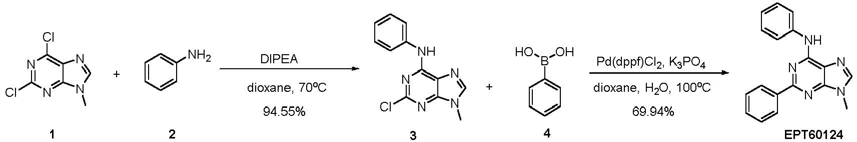

### Step 1: 2-chloro-9-methyl-N-phenyl-9H-purin-6-amine (3)

2,6-dichloro-9-methyl-9H-purine (1,100 mg, 0.49 mmol), aniline (2,69 mg, 0.74 mmol) and DIPEA (191 mg, 1.48 mmol) were added to a 25 ml round bottom flask. The reaction mixture was dissolved in dioxane (5ml), stirred at 70°C for 4h, the residue was concentrated in vacuum, and purified by automatic flash column chromatography (silica gel, DCM:MeOH=95:5) to obtain 2-chloro-9-methyl-N-phenyl-9H-purin-6-amine (3,120 mg, 94.55% yield) as a off white solid.

LCMS: (ESI) m/z=260.19 (M+H)⁺; RT=1.52min.

### Step 2: 9-methyl-N,2-diphenyl-9H-purin-6-amine (EPT60125)

2-chloro-9-methyl-N-phenyl-9H-purin-6-amine (3,50 mg, 0.19 mmol), phenylboronic acid (4,69 mg, 0.57 mmol), Pd(dppf)Cl₂(14 mg, 0.019 mmol) and K₃PO₄(80 mg, 0.38 mmol) were respectively put into a 25 ml round bottom flask. The mixture was suspended in dioxane (5ml) and H₂O (1ml). The reaction was stirred overnight at 100°C under a nitrogen atmosphere. The solvent was removed under reduced pressure and purification was performed by automatic flash column chromatography (silica gel, PE:EA=1:1) to obtain 9-methyl-n,2-diphenyl-9H-purin-6-amine (EPT60125, 40mg, 69.94% yield) as a yellow solid.

LCMS: (ESI) m/z=302.25 (M+H)⁺; RT=1.76min.

¹H NMR (500 MHz, DMSO) δ 9.90 (s, 1H), 8.43 (d, *J* = 7.5 Hz, 2H), 8.30 (s, 1H), 8.05 (d, *J* = 7.9 Hz, 2H), 7.55 - 7.46 (m, 3H), 7.40 (t, *J* = 7.7 Hz, 2H), 7.07 (t, *J* = 7.1 Hz, 1H), 3.87 (s, 3H).

### Example 25

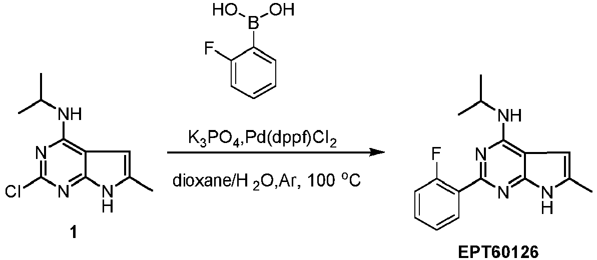

### Step 1: 2-(2-fluorophenyl)-N-isopropyl-6-methyl-7H-pyrrolo[2,3-d]pyrimidin-4-amine (EPT60126)

Compound 1 (50mg, 0.22 mmol) was added to a solution of 2,5 mL of dioxane (5ml) and H₂O (1ml), and K₃PO₄ (163.4 mg, 0.77 mmol), Pd(dppf)Cl₂ (32.7 mg, 0.04 mmol) and 2-fluorophenylboronic acid (154.0 mg, 1.1 mmol) were added. The reaction mixture was stirred at 100°C for 16 h (overnight). LCMS (EPN18040-009-1) showed that the reaction had been completed, and 20% of SM remained. The solvent was concentrated in vacuo. The residue was purified using automatic flash column chromatography (silica gel, PE/EA=8:1), and then purified using flash column (C18 column chromatography, H₂O (NH₄HCO₃, 0.8g/L)/CH₃CN = 70/30), to obtain the title compound (8.5 mg, 13.4% yield) as a white solid. (ESI) m/z=285.24 (M+H)⁺. ¹H NMR (500 MHz, DMSO-*d*₆) 11.42 (s, 1 H), 9.94-9.98 (m, 1 H), 7.38-7.43 (m, 1 H), 7.20-7.26 (m, 2 H), 6.98-6.99 (d, *J* = 7.5 Hz, 1 H), 6.26 (s, 1 H), 4.38-4.42 (m, 1 H), 3.32 (s, 3 H), 1.23-1.25 (d, *J=* 6.5 Hz, 6 H) ppm.

### Example 26

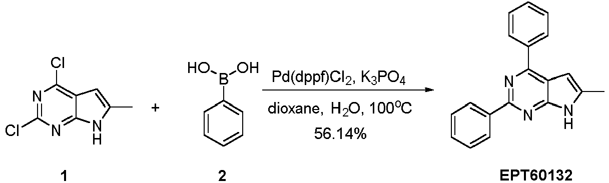

### Step 1: 6-methyl-2,4-diphenyl-7H-pyrrolo[2,3-d]pyrimidine (EPT60132)

2,4-dichloro-6-methyl-7H-pyridine [2,50 mg, 0.25 mmol], phenylboronic acid (2,90 mg, 0.75 mmol), Pd(dppf)Cl₂(18 mg, 0.025 mmol) and K₃PO₄(159 mg, 0.75 mmol) were added to a 25 ml round bottom flask. The mixture was suspended in dioxane (5ml) and H₂O (1ml). The reaction was carried out overnight under a nitrogen atmosphere at 100°C. Using automatic flash column chromatography (silica gel, PE:EA=1:1), the residue was concentrated and purified in vacuo to obtain 6-methyl-2,4-diphenyl-7H-pyrroline [2,3-d] pyrimidine (EPT60132, 40 mg, 56.14% yield) as a white solid. LCMS: (ESI) m/z=286.20 (M+H)⁺; RT=1.83min.

¹H NMR (500 MHz, DMSO) δ 12.17 (s, 1H), 8.56 - 8.50 (m, 2H), 8.31 - 8.26 (m, 2H), 7.65 - 7.44 (m, 6H), 6.65 (s, 1H), 2.48 (s, 3H).

### Example 27

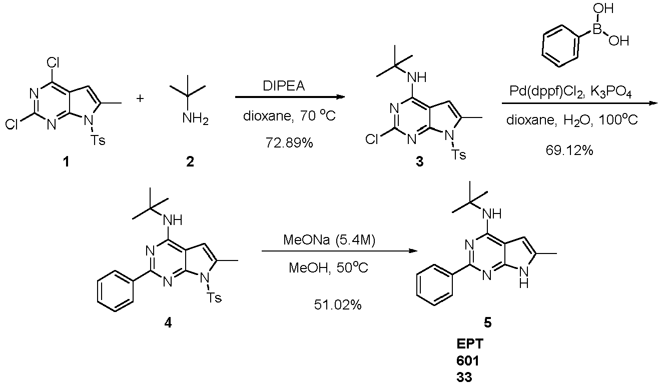

### Step 1: N-(tert-butyl)-2-chloro-6-methyl-7-tosyl-7H-pyrrolo[2,3-d]pyrimidin-4-amine

2,4-dichloro-6-methyl-7-tosyl-7H-pyridine[2,3-d]pyrimidine (100 mg, 0.28 mmol), 2-methylpropyl-2-amine (31 mg, 0.42 mmol) and DIPEA (108 mg, 0.84 mmol) were repectively put into a 25 ml round bottom flask. The reaction mixture was dissolved in dioxane (5ml) and stirred at 70°C for 4h. The residue was concentrated in vacuum and purified by automatic flash column chromatography (silica gel, PE:EA=85:15) to obtain N-(tert-butyl) -2-chloro-6-methyl-7-tosyl-7H-pyrrolo[2,3-d]pyrimidin-4-amine (80 mg, 72.89% yield) as a white solid. LCMS: (ESI) m/z=393.27 (M+H)⁺; RT=1.93min.

### Step 2: N-(tert-butyl)-6-methyl-2-phenyl-7-tosyl-7H-pyrrolo[2,3-d]pyrimidin-4-amine

N-(tert-butyl)-2-chloro-6-methyl-7-tosyl-7H-pyrrolo[2,3-d]pyrimidin-4-amine (80 mg, 0.2 mmol), phenylboronic acid (6,73 mg, 0.6 mmol), Pd(dppf)Cl₂(17 mg, 0.02 mmol) and K₃PO₄(127 mg, 0.6 mmol) were added into a 25 ml round bottom flask. The mixture was suspended in dioxane (5ml) and H₂O (1ml). The reaction was stirred overnight at 100°C under a nitrogen atmosphere. The solvent was removed under reduced pressure and purification was performed by automatic flash column chromatography (silica gel, PE:EA=5:1) to obtain N-(tert-butyl)-6-methyl-2-phenyl-7-tosyl-7H-pyrrole[2,3-d]pyrimidin-4-amine (7,60 mg, 69.12%).

LCMS: (ESI) m/z=435.32 (M+H)⁺; RT=2.02min.

### Step 3: N-(tert-butyl)-6-methyl-2-phenyl-7H-pyrrolo[2,3-d]pyrimidin-4-amine (EPT60133)

N-(tert-butyl)-6-methyl-2-phenyl-7-tosyl-7H-pyrrole[2,3-d]pyrimidin-4-amine (60mg, 0.14 mmol) was dissolved in MeONa (0.5 ml, 5.4M in MeOH) and MeOH (5ml), and the mixture was stirred overnight at 50c. The solvent was removed under reduced pressure and purification was performed by automatic flash column chromatography (silica gel, PE:EA=5:1) to obtain N-(tert-butyl)-6-methyl-2-phenyl-7H-pyrrolo[2,3-d]pyrimidin-4-amine (EPT60133, 20 mg, 51.02% yield) as a white solid.

LCMS: (ESI) m/z=281.20 (M+H)⁺; RT=1.49min.

¹H NMR (500 MHz, DMSO) δ 11.33 (s, 1H), 8.36 - 8.33 (m, 2H), 7.45 (dd, *J* = 10.3, 4.6 Hz, 2H), 7.40 - 7.36 (m, 1H), 6.53 (s, 1H), 6.35 (dd, *J* = 1.9, 1.0 Hz, 1H), 2.31 (d, *J* = 0.6 Hz, 3H), 1.58 (s, 9H).

### Example 28

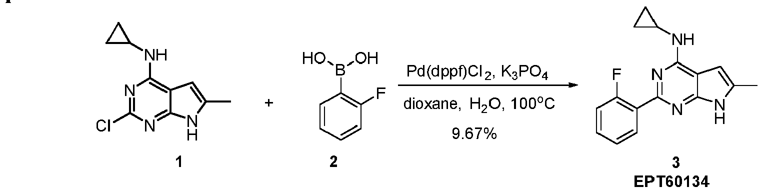

### Step 1: N-cyclopropyl-2-(2-fluorophenyl)-6-methyl-7H-pyrrolo[2,3-d]pyrimidin-4-amine (EPT60134)

2-chloro-N-cyclopropyl-6-methyl-7H-pyridinol[2,3-d]4-aminopyridine (1,50 mg, 0.22 mmol), (2-fluorophenyl)boronic acid ( 2,92 mg, 0.66 mmol), Pd(dppf)Cl₂ (16 mg, 0.022 mmol) and K₃PO₄ (93 mg, 0.44 mmol) were respectively put into a 25 ml round bottom flask. The mixture was suspended in dioxane (5ml) and H₂O (1ml). The reaction was carried out overnight under a nitrogen atmosphere at 100°C. The solvent was removed under reduced pressure. Purification was firstly performed by automatic flash column chromatography (silica gel, PE:EA=1:1) to obtain the crude product, and then reversed-phase column chromatography (NH₄HCO₃, a.q. 0.5%) was used to obtain white solid N-cyclopropyl-2-(2-fluorophenyl)-6-methyl-7H-pyrrolo[2,3-d]pyrimidin-4-amine (EPT60134, 6 mg, 9.67% yield): MeCN=55:45.

LCMS: (ESI) m/z=283.16 (M+H)⁺; RT=1.13min.

¹H NMR (500 MHz, DMSO) δ 11.44 (d, *J* = 35.0 Hz, 1H), 8.02 - 7.89 (m, 1H), 7.44 - 7.34 (m, 2H), 7.28 - 7.19 (m, 2H), 6.27 (d, *J* = 34.6 Hz, 1H), 2.98 - 2.94 (m, 1H), 2.33 (s, 3H), 0.80 - 0.73 (m, 2H), 0.62 - 0.55 (m, 2H).

### Example 29

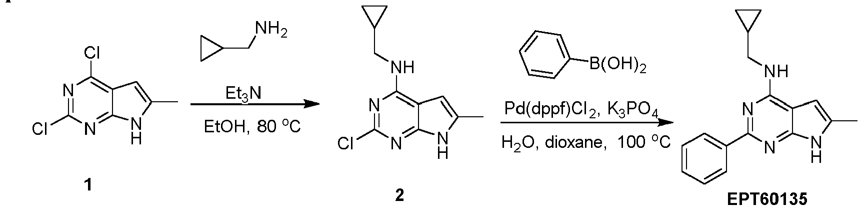

### Step 1: 2-chloro-N-(cyclopropylmethyl)-6-methyl-7H-pyrrolo[2,3-d]pyrimidin-4-amine (2)

Et₃N (202.2 mg, 2.0 mmol) and aminomethylcyclopropane (142.1 mg, 2.0 mmol) were added to a solution of Compound 1 (100 mg, 0.50 mmol) in EtOH (3 ml). The reaction mixture was stirred at 80°C for 2 hours. LCMS (EPN18040-010-1) showed that the reaction was completed and no SM remained. The solvent was concentrated in vacuo. The unpurified crude compound was used directly in the next step. (ESI) m / z = 237.21 (m + H) ⁺.

### Step 2: N-(cyclopropylmethyl)-6-methyl-2-phenyl-7H-pyrrole[2,3-d]pyrimidin-4-amine (EPT60135)

K₃PO₄ (216.5 mg, 1.02 mmol), Pd(dppf)Cl₂ (47.4 mg, 0.058 mmol) and phenylboronic acid (176.9 mg, 1.45 mmol) were respectively added to a solution of Compound 2 (68.5 mg, 0.29 mmol) in dioxane (5 mL) and H₂O (1 mL). The reaction mixture was stirred at 100°C for 16 h (overnight). Under Ar conditions, LCMS (EPN18040-012-1) showed that the reaction was completed and 30% of SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 70/30) to obtain the title compound (7.5 mg, 9.3% yield) as a pale yellow solid. (ESI) m/z=279.26 (M+H)⁺. ¹H NMR (500 MHz, DMSO-d₆) 11.37 (s, 1 H), 8.34-8.36 (m, 2 H), 7.42-7.45 (m, 2 H), 7.31-7.39 (m, 2 H), 6.24 (s, 1 H), 3.44-3.50 (m, 2 H), 2.33 (s, 3 H), 1.18-1.21 (m, 1 H), 0.46-0.48 (m, 2 H), 0.33-0.36 (m, 2 H) ppm.

### Example 30

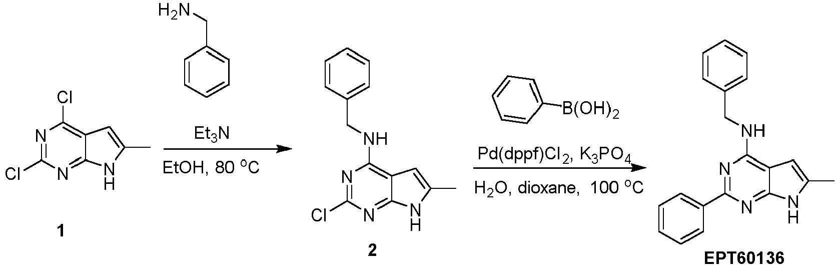

### Step 1: N-benzyl-2-chloro-6-methyl-7H-pyrrolo[2,3-d]pyrimidin-4-amine (2)

Et₃N (101.16 mg, 1.0 mmol) and benzylamine (107.1 mg, 1.0 mmol) were added to a solution of Compound 1 (100 mg, 0.50 mmol) in EtOH (5 mL). The reaction mixture was stirred at 80°C for 2 hours. LCMS (EPN18040-014-1) showed that the reaction was completed and no SM remained. The solvent was concentrated in vacuo. The unpurified crude compound was used directly in the next step. (ESI) m/z=273.28 (M+H)⁺.

### Step 2: N-benzyl-6-methyl-2-phenyl-7H-pyrrolo[2,3-d]pyrimidin-4-amine (EPT60136)

K₃PO₄ (150.2 mg, 0.71 mmol), Pd(dppf)Cl₂ (32.7 mg, 0.04 mmol) and phenylboronic acid (122.1 mg, 1.0 mmol) were respectively added to a solution of Compound 2 (55 mg, 0.2 mmol) in dioxane (5 mL) and H₂O (1 mL). The reaction mixture was stirred at 100°C for 16 h (overnight). LCMS (EPN18040-017-1) under Ar showed that the reaction was completed and 20% of SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 70/30) to obtain the title compound (4.4 mg, 7% yield). (ESI) m/z=315.15 (M+H)⁺. ¹H NMR (500 MHz, DMSO-*d*₆) 11.43 (s, 1 H), 8.31-8.33 (m, 2 H), 7.82-7.84 (m, 1 H), 7.39-7.43 (m, 4 H), 7.35-7.37 (m, 1 H), 7.29-7.32 (m, 2 H), 7.20-7.23 (m, 1 H), 6.25 (s, 1 H), 4.81-4.82 (d, *J=* 6.0 Hz, 2 H), 2.33 (s, 3 H) ppm.

### Example 31

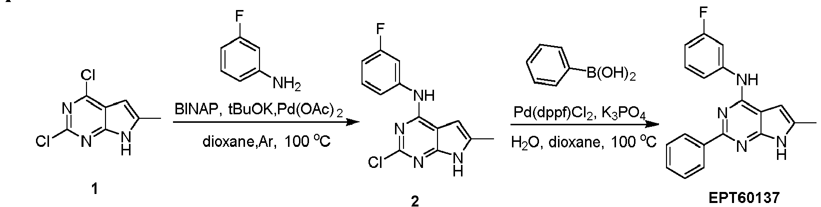

### Step 1: 2-chloro-N-(3-fluorophenyl)-6-methyl-7H-pyrrolo[2,3-d]pyrimidin-4-amine (2)

tBuOK (101.0 mg, 1.0 mmol), pd(OAc)₂ (11.3 mg, 0.05 mmol), BINAP (62.3 mg, 0.1 mmol) ) and 3-fluoroaniline (134.2 mg, 1.1 mmol) were added to a solution of Compound 1 (100 mg, 0.50 mmol) in dioxane (3ml). The reaction mixture was stirred at 100°C for 16 h (overnight). LCMS (EPN18040-015-1) under Ar showed that the reaction was completed, and 20% of SM remained. The solvent was concentrated in vacuo. The residue was purified by automatic flash column chromatography (silica gel, PE/EA=8:1) to obtain the title compound (60 mg, 44.2% yield) as a pale yellow solid. (ESI) m/z=277.21 (M+H)⁺.

### Step 2 N-(3-fluorophenyl)-6-methyl-2-phenyl-7H-pyrrolo[2,3-d]pyrimidin-4-amine (EPT60137)

Pd(dppf)Cl₂ (32.7 mg, 0.04 mmol), K₃PO₄ (163.4 mg, 0.77 mmol) and phenylboronic acid (134.2 mg, 1.1 mmol) were added to a solution of Compound 2 (66.0 mg, 0.22 mmol) in dioxane (5 mL) and H₂O (1 mL). The reaction mixture was stirred at 100°C for 16 h (overnight). LCMS (EPN18040-023-1) under Ar showed that the reaction was completed and 5% of SM remained. The reaction mixture was concentrated in a vacuum. The residue was purified using automatic flash column chromatography (silica gel, PE/EA=8:1) to obtain the crude product. The solid was further purified using flash column (C-18 column chromatography, H₂O (NH₄HCO₃, 0.8g/L)/CH₃CN = 60/40) to obtain the title compound (2.6 mg, 4.1% yield) as a white solid.

(ESI) m/z=319.21 (M+H)⁺. ¹H NMR (500 MHz, DMSO-*d₆*) 11.75 (s, 1 H), 9.44 (s, 1 H), 8.35-8.37 (m, 2 H), 8.09-8.13 (m, 1 H), 7.70 (m, 1 H), 7.49-7.52 (m, 2 H), 7.38-7.45 (m, 2 H), 6.81-6.85 (m, 1 H), 6.52 (s, 1 H), 2.4 (s, 3 H) ppm.

### Example 32

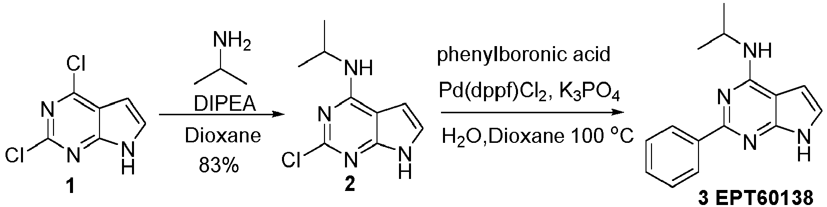

### Step 1: 2-chloro-N-isopropyl-7H-pyrrolo[2,3-d]pyrimidin-4-amine (2)

2,4-dichloro-7H-pyrrolo[2,3-d]pyrimidine (1.0g, 5.31mmol), propylenediamine (3.0mL), and DIPEA (1.0mL) were respectively filled into a 20 mL sealed tube, and then dioxane (10ml) was added. The reaction mixture was stirred at 70°C for 16 h. After the reaction was completed, the mixture was concentrated under atmospheric vacuum distillation. The residue was dissolved in (EtOH)₅mL, added with water (20 mL), and stirred at RT for 10 min. The mixture was filtered, the solid was collected, and dried in vacuum to obtain the target compound (940 mg, yield 83%) as a white solid.

Molecular formula: C₉H₁₁ClN₄, molecular weight: 210.67, (ESI) m/z=211.2(M+H)⁺.

### Step 2: N-isopropyl-2-phenyl-7H-pyrrolo[2,3-d]pyrimidin-4-amine (3 EPT60138)

2-chloro-N-isopropyl-7H-pyrrole[2,3-d]pyridin-4-amine (440mg, 4.0mmol), phenylboronic acid (1.22g, 10.0mmol), Pd(dppf)Cl₂(140mg,0.2mmol) and K₃PO₄ (2.12g, 10.0mmol) were filled into a 10-mL sealed tube, and then dioxane (15mL) and water (3.0mL) were added. The reaction mixture was heated at 100°C for 22 h in an inert gas. After the reaction was completed, the reaction mixture was evaporated and concentrated with silica gel (100-200 mesh) to obtain a powder residue. The product was purified by automatic flash column chromatography (silica gel, EA: PE = 1:3) to obtain the title compound 224 mg, yield 44%) as a white solid.

Molecular formula: C₁₅H₁₆N₄, molecular weight: 252.32, (ESI) m/z=253.2(M+H)⁺. ¹HNMR (500 MHz, DMSO-d₆) 11.50 (s,1H), 8.37(m, 2H),7.37-7.46 (m, 3H),7.19 (d, *J*=7.5Hz,1H),7.08 (m,1H), 6.60 (m,1H) 4.56 (m,1H), 1.30(d, *J*=6.5Hz,6H) ppm.

### Example 33

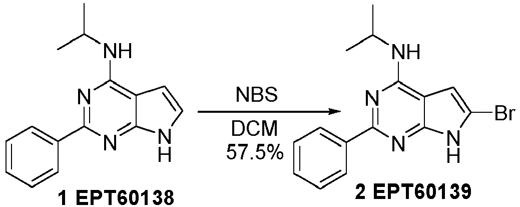

### Step 1: 6-bromo-N-isopropyl-2-phenyl-7H-pyrrolo[2,3-d]pyrimidin-4-amine (2 EPT60139)

N-isopropyl-2-phenyl-7H-pyridinol[2,3-d]pyrimidin-4-amine (200mg, 0.8mmol) was dissolved in DCM (30mL), and then NBS (144mg dissolved in DCM (10mL) solution) was slowly added at 0-10°C for 30min. The reaction mixture was stirred at rt for 20 min. The reaction was monitored by LCMS. After the reaction was completed, the reaction mixture was evaporated and concentrated with silica gel (100-200 mesh) to obtain a powder residue. The product was purified by automatic flash column chromatography (silica gel, EA: PE = 1:8) to obtain the title compound (152 mg, yield 57.5%) as a white solid.

Molecular formula: C₁₅H₁₅BrN₄, molecular weight: 331.22, (ESI) m/z=331.1(M+H)⁺. ¹HNMR (500 MHz, DMSO-d₆) 12.03(s, 1H), 8.35-8.37(m, 2H),7.42-7.49(m, 3H),7.36(d, *J*=2.5Hz, 1H),5.99 (d, *J*=7.5Hz, 1H), 4.55(m,1H), 1.34(d, J=6.5Hz,6H) ppm.

### Example 34

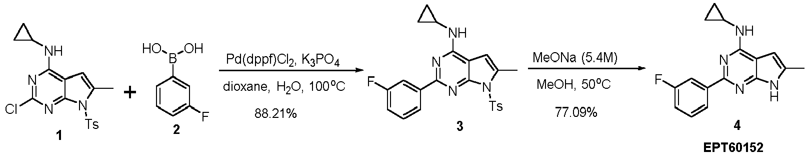

### Step 1: N-cyclopropyl-2-(3-fluorophenyl)-6-methyl-7-tosyl-7H-pyrrolo[2,3-d]pyrimidin-4-amine

**(7)** 2-chloro-N-cyclopropyl-6-methyl-7-tosyl-7H-pyrrolo[2,3-d]pyrimidin-4-amine (100 mg , 0.26 mmol), (3-fluorophenyl)boronic acid (109 mg, 0.78 mmol), Pd(dppf)Cl₂ (19 mg, 0.026 mmol) and K₃PO₄(93 mg, 0.78 mmol) were added in a 25 ml round bottom flask. The mixture was suspended in dioxane (5ml) and H₂O (1ml). The reaction was carried out overnight under a nitrogen atmosphere at 100°C. The solvent was removed under reduced pressure and purification was performed by automatic flash column chromatography (silica gel, PE:EA=1:1) to obtain a white solid N-cyclopropyl-2-(3-fluorophenyl)-6-methyl-7-tosyl-7H-pyrrolo[2,3-d]pyrimidin-4-amine (7,100 mg, 88.21% yield). LCMS: (ESI) m/z=437.32 (M+H)⁺; RT=1.13min.

### Step 2: N-cyclopropyl-2-(3-fluorophenyl)-6-methyl-7H-pyrrolo[2,3-d]pyrimidin-4-amine (EPT60152)

N-cyclopropyl-2-(3-fluorophenyl)-6-methyl-7-tosyl-7H-pyrrolo[2,3-d]pyrimidin-4-amine (100mg, 0.23mmol), dissolved in MeONa (1ml, 5.4M in MeOH solution) and MeOH (5ml), was added to a 25mL round bottom flask, and stirred at 50°C for 2 hours. The residue was concentrated in vacuo and purified by automated flash column chromatography (silica gel, PE:EA = 4:1) to obtain an off-white solid N-cyclopropyl-2-(3-fluorophenyl)-6-methyl-7H-pyrrolo[2,3-d]pyrimidin-4-amine (EPT60152, 50mg, yield 77.09%).

LCMS: (ESI) m/z=283.16 (M+H)⁺; RT=1.31min.

¹H NMR (500 MHz, DMSO) δ 11.48 (s, 1H), 8.21 (d, *J* = 7.8 Hz, 1H), 8.07 (d, *J* = 10.0 Hz, 1H), 7.48 (td, *J* = 8.0, 6.2 Hz, 1H), 7.42 (d, *J* = 3.1 Hz, 1H), 7.21 (d, *J* = 2.7 Hz, 1H), 6.30 (s, 1H), 3.02 (dd, *J* = 6.6, 3.4 Hz, 1H), 2.33 (s, 3H), 0.84 - 0.79 (m, 2H), 0.63 - 0.57 (m, 2H).

### Example 35

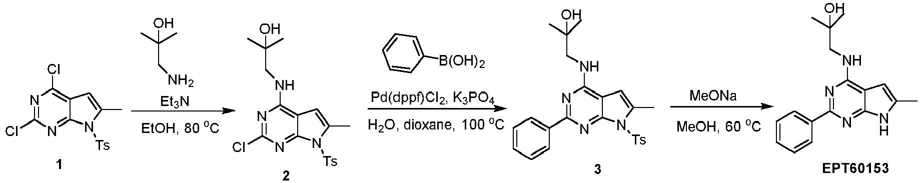

### Step 1: 1-((2-chloro-6-methyl-7-tosyl-7H-pyrrolo[2,3-d]pyrimidin-4-yl)amino)-2-methylpropan-2-ol (2)

Et₃N (56.6 mg, 0.56 mmol) and 1-amino-2-methylpropaN-2-ol (49.8 mg, 0.56 mmol) were added to a solution of Compound 1 (100 mg, 0.28 mmol) in EtOH (5 mL). The reaction mixture was stirred at 80°C for 2 hours. LCMS (EPN18040-019-1) showed that the reaction was completed and no SM remained. The solvent was concentrated in vacuo. The unpurified crude compound was used directly in the next step (ESI) m/z=409.28 (M+H)⁺.

### Step 2: 2-methyl-1-((6-methyl-2-phenyl-7-tosyl-7H-pyrrolo[2,3-d]pyrimidin-4-yl)amino)propan-2 -ol (3)

K₃PO₄ (163.4 mg, 0.77 mmol) and Pd(dppf)Cl₂ (35.9 mg, 0.04 mmol) and phenylboronic acid (134.2 mg, 1.1 mmol) were respectively added to a solution of Compound 2 (90 mg, 0.22 mmol) in dioxane (5 mL) and H₂O (1 mL). The reaction mixture was stirred at 100°C for 16 h (overnight). LCMS (EPN18040-021-1) under Ar showed that the reaction was completed and 35% of SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 70/30) to obtain the title compound (43.5 mg, 43.8% yield) as a pale yellow solid. (ESI) m/z=451.36 (M+H)⁺.

### Step 3: 2-methyl-1-((6-methyl-2-phenyl-7H-pyrrolo[2,3-d]pyrimidin-4-yl)amino)propan-2-ol (EPT60153)

MeONa (0.5 mL, 2.7 mmol) was added to a solution of Compound 3 (43.5 mg, 0.10 mmol) added to MeOH (5 mL). The reaction mixture was stirred at 60°C for 4 hours. LCMS (EPN18040-025-1) showed that the reaction was completed and no SM remained. The solvent was concentrated in vacuo. The residue was diluted with saturated NH₄Cl solution (30ml), the mixture was extracted with CH₂Cl₂ (30ml×3), and washed with saturated NaCl (30ml). The resulting organic layer was dried with anhydrous Na₂SO₄, and the solvent was removed in vacuum. The residue was purified with Flash (C-18 column chromatography, H₂O (NH₄HCO₃, 0.8g/L)/CH₃CN = 40/60) to obtain the title compound (5.8 mg, 20.3% yield) as a white solid.

(ESI) m/z=297.23 (M+H)⁺. ¹H NMR (500 MHz, DMSO-*d₆*) 11.43 (s, 1 H), 8.32-8.34 (m, 2 H), 7.42-7.45 (m, 2 H), 7.36-7.39 (m, 1 H), 7.14 (s, 1 H), 6.31 (s, 1 H), 5.01 (s, 1 H),3.58-3.59 (d, *J* = 5.5 Hz, 2 H), 2.33 (s, 3 H), 1.17 (s, 6 H) ppm.

### Example 36

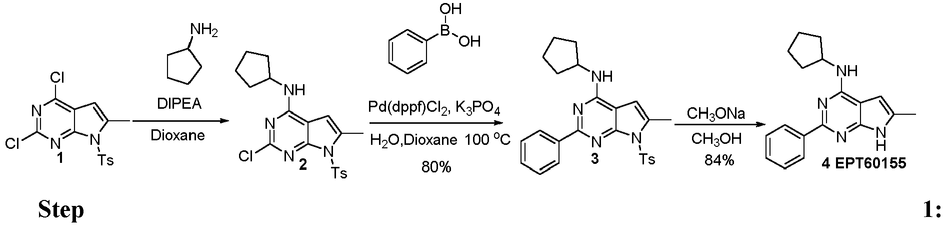

### 2-chloro-N-cyclopentyl-6-methyl-7-tosyl-7H-pyrrolo[2,3-d]pyrimidin-4-amine (2)

2,4-dichloro-6-methyl-7-tosyl-7H-pyridine[2,3-d]pyrimidine (100mg, 0.28mmol), cyclopentylamine (0.5mL), DIPEA (0.5mL) were respectively filled into a 20ml sealed tube, 2,4-dichloro-6-methyl-7-tosyl-7H-pyrrolo[2,3-d]pyrimidine (100mg, 0.28mmol), cyclopentylamine (0.5mL ), dioxane (5ml) were added. The reaction mixture was stirred at 70°C for 16 h. The reaction was monitored by LCMS. Molecular formula: C₁₉H₂₁ClN₄O₂S, molecular weight: 404.91, (ESI) m/z=405.3(M+H)⁺. No SM-1 was remained, the crude product was used in the next step without further purification.

### Step 2: N-cyclopentyl-6-methyl-2-phenyl-7-tosyl-7H-pyrrolo[2,3-d]pyrimidin-4-amine (3)

2-chloro-N-cyclopentyl-6-methyl-7-tosyl-7H-pyrrolo[2,3-d]2,3-d-pyrimidin-4-amine (crude product in 5mL dioxane), phenylboronic acid (110mg, 0.9mmol), Pd(dppf)Cl₂ (20mg, 0.03 mmol), K₃PO₄ (210mg, 1.0mmol) were filled into a 10 ml sealed tube, and then water (1.0mL) was added. The reaction mixture was heated at 100°C in an inert gas for 16 h. After the reaction was completed, the reaction mixture was evaporated and concentrated with silica gel (100-200 mesh) to obtain a powder residue. The product was purified by automatic flash column chromatography (silica gel, EA: PE =1:7), and the product was obtained as a white solid (100 mg, yield 80%).

Molecular formula: C₂₅H₂₆N₄O₂S, molecular weight: 446.57,(ESI) m/z=447.3(M+H)⁺.

### Step 3: N-cyclopentyl-6-methyl-2-phenyl-7H-pyrrolo[2,3-d]pyrimidin-4-amine (4 EPT60155)

N-cyclopentyl-6-methyl-2-phenyl-7-tosyl-7H-pyrrole[2,3-d]pyrimidin-4-amine (100 mg, 0.22 mmol) was dissolved in CH₃OH (5mL), and CH₃ONa (5.4 M,0.5 mL) was added. The reaction mixture was stirred at 55°C for 20 h. After the reaction was completed, the reaction mixture was evaporated and concentrated with silica gel (100-200 mesh) to obtain a powder residue. The product was purified by automatic flash column chromatography (silica gel, EA: PE = 1:3), and the product was obtained as a white solid (55 mg, yield 84%).

Molecular formula: C₁₈H₂₀N₄, molecular weight: 292.39, (ESI) m/z=293.2(M+H)⁺. ¹HNMR (500 MHz, DMSO-d₆)₁1.37(s,1H), 8.34-8.36(m,2H), 7.35-7.44(m,3H), 7.08 ( d, *J*=7.0Hz,1H), 6.27(s,1H), 4.57 (m,1H), 2.31(s,3H), 2.03-2.08(m,2H), 1.73-1.74(m,2H), 1.57-1.63(m,4H) ppm.

### Example 37

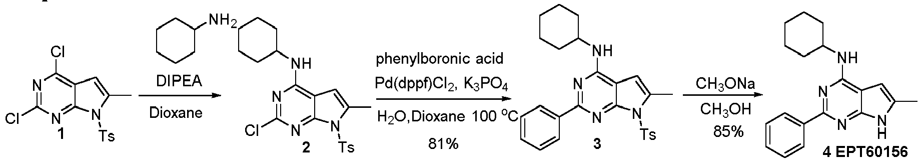

### Step 1: 2-chloro-N-cyclohexyl-6-methyl-7-tosyl-7H-pyrrolo[2,3-d]pyrimidin-4-amine (2)

2,4-dichloro-6-methyl-7-tosyl-7H-pyridine[2,3-d]pyrimidine (100mg, 0.28mmol), cyclohexylamine (0.5mL), DIPEA (0.5mL) were respectively filled into a 20ml sealed tube, and 5mL of dioxane was added. The reaction mixture was stirred at 70°C for 16 h. The reaction was monitored by LCMS. Molecular formula: C₂₀H₂₃ClN₄O₂S, molecular weight: 418.94 (ESI) m/z=419.3(M+H)⁺. No SM-1 was remained. The crude product is used in the next step without further purification.

### Step 2: N-cyclohexyl-6-methyl-2-phenyl-7-tosyl-7H-pyrrolo[2,3-d]pyrimidin-4-amine (3)

2-chloro-N-cyclohexyl-6-methyl-7-tosyl-7H-pyrrolo[2,3-d]2,3-d-pyrimidin-4-amine (crude product in 5mL dioxane), phenylboronic acid (110mg, 0.9mmol), Pd(dppf)Cl₂(20mg, 0.03mmol), K₃PO₄ (210mg, 1.0mmol) were filled into a 10 ml sealed tube, and then water (1.0mL) was added. The reaction mixture was heated at 100°C in an inert gas for 14 h. After the reaction was completed, the reaction mixture was evaporated and concentrated with silica gel (100-200 mesh) to obtain a powder residue. The product was purified by automatic flash column chromatography (silica gel, EA: PE = 1:9) to obtain the title compound (105 mg, yield 81%) as a white solid.

Molecular formula: C₂₆H₂₈N₄O₂S, molecular weight: 460.60 (ESI) m/z=461.3(M+H)⁺.

### Step 3: N-cyclohexyl-6-methyl-2-phenyl-7H-pyrrolo[2,3-d]pyrimidin-4-amine (4 EPT60156)

N-cyclohexyl-6-methyl-2-phenyl-7-tosyl-7H-pyrrole[2,3-d]pyridine-4-amine (105 mg, 0.22mmol) was dissolved in CH₃OH (5mL) , and CH₃ONa (5.4M, 0.5mL) was added. The reaction mixture was stirred at 55°C for 20 h. After the reaction was completed, the reaction mixture was evaporated and concentrated with silica gel (100-200 mesh) to obtain a powder residue. The product was purified by automatic flash column chromatography (silica gel, EA: PE = 3:7), and the product was obtained as a white solid (60 mg, 85% yield).

Molecular formula: C₁₉H₂₂N₄, molecular weight: 306.41, (ESI) m/z=307.2(M+H)⁺. ¹HNMR (500 MHz, DMSO-d₆) 11.34 (s, 1H), 8.33-8.35(m, 2H), 7.41-7.44(m, 2H),7.35-7.38 (m, 1H), 6.95 (d, *J*=7.5 Hz, 1H), 6.21(s, 1H), 4.15(m, 1H),2.31(s, 3H), 2.03(m, 2H) ,1.17-1.80 (m, 8H) ppm.

### Example 38

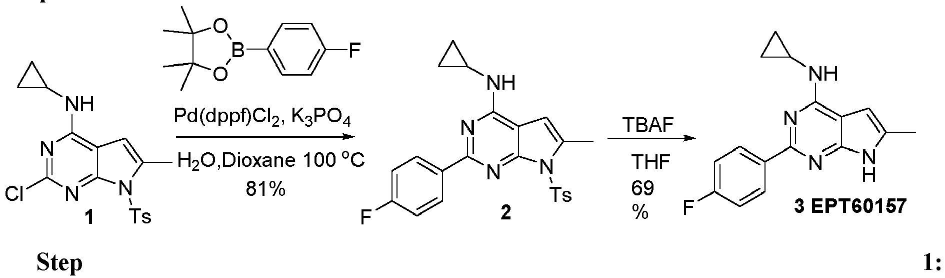

### N-cyclopropyl-2-(4-fluorophenyl)-6-methyl-7-tosyl-7H-pyrrolo[2,3-d]pyrimidin-4-amine (2)

2-chloro-N-cyclopropyl-6-methyl-7-tosyl-7H-pyrrolidine[2,3-d]pyrimidin-4-amine (75mg, 0.2mmol), 2-(4-fluorophenyl)-4,4,5-tetramethyl-1,3,2-dioxborane (100mg, 0.45mmol), Pd(dppf)Cl₂(14mg,0.02mmol), K₃PO₄ (120mg, 0.6mmol), water (1.0 mL), and dioxane (5 mL) were filled into a 10-mL sealed tube. The reaction mixture was heated at 90°C in an inert gas for 16 h. After the reaction was completed, the reaction mixture was evaporated and concentrated with silica gel (100-200 mesh) to obtain a powder residue. The product was purified by automatic flash column chromatography (silica gel, EA: PE = 1:4) to obtain the title compound (71 mg, yield 81%) as a white solid.

Molecular formula: C₂₃H₂₁FN₄O₂S, molecular weight: 436.51 (ESI) m/z=437.3(M+H)⁺.

### Step 3: N-cyclopropyl-2-(4-fluorophenyl)-6-methyl-7H-pyrrolo[2,3-d]pyrimidin-4-amine (EPT60157)

N-cyclopropyl-2-(4-fluorophenyl)-6-methyl-7-tosyl-7H-pyrrole[2,3-d]pyrimidin-4-amine (71 mg, 0.16mmol) was dissolved in THF (5mL), and TBAF (1M, 0.5mL) was added. The reaction mixture was stirred at 55°C for 1 h. After the reaction was completed, the reaction mixture was evaporated and concentrated with silica gel (100-200 mesh) to obtain a powder residue. The product was purified by automatic flash column chromatography (silica gel, EA: PE = 1:4) to obtain the title compound (32 mg, yield 69.5%) as a white solid. Molecular formula: C₁₆H₁₅FN₄, molecular weight: 282.32 (ESI) m/z=283.3(M+H)⁺. ¹HNMR (400 MHz, DMSO-d₆) 11.36(s, 1H), 8.34-8.37(m, 2H),7.32(d, J=3.2Hz, 1H),7.18-7.22(m, 2H), 6.23 (s, 1H), 2.97 (m, 1H), 2.28(s, 3H), 0.74-0.83 (m,2H) ,0.53-0.576 (m,2H) ppm.

### Example 39

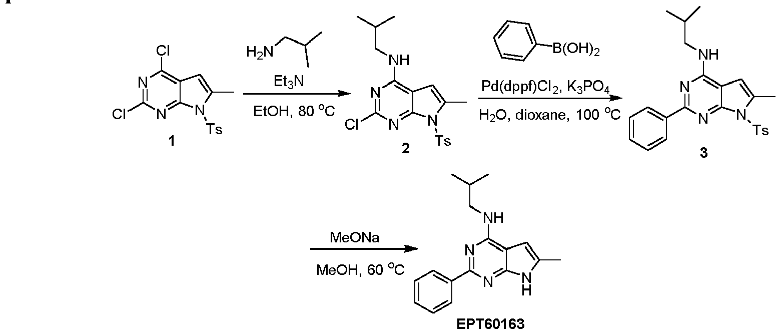

### Step 1: 2-chloro-N-isobutyl-6-methyl-7-tosyl-7H-pyrrolo[2,3-d]pyrimidin-4-amine (2)

Et₃N (56.6 mg, 0.56 mmol) and 2-methylpropyl-1-amine (40.9 mg, 0.56 mmol) were added to a solution of Compound 1 (100 mg, 0.28 mmol) added to EtOH (3ml). The reaction mixture was stirred at 80°C for 4 h. LCMS (EPN18040-040-1) indicated that the reaction had been completed and no SM remained. The solvent was concentrated in vacuo. The unpurified crude compound was used directly in the next step. (ESI) m/z=393.22 (M+H)⁺.

### Step 2: N-isobutyl-6-methyl-2-phenyl-7-tosyl-7H-pyrrolo[2,3-d]pyrimidin-4-amine (3)

K₃PO₄ (163.5 mg, 0.81 mmol), Pd(dppf)Cl₂ (35.9 mg, 0.046 mmol) and phenylboronic acid (134.2 mg, 1.15 mmol) were respectively added to a solution of Compound 2 (90 mg, 0.23 mmol) in dioxane (5 mL) and H₂O (1 mL). The reaction mixture was stirred at 100° C for 16 h (overnight). LCMS (EPN18040-041-1) showed that the reaction was completed and 10% of SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 70/30) to obtain the title compound (15 mg, 15.1% yield) as a pale yellow solid.

(ESI) m/z=435.32 (M+H)⁺.

### Step 3: N-isobutyl-6-methyl-2-phenyl-7H-pyrrolo[2,3-d]pyrimidin-4-amine (EPT60163)

MeONa (0.5 mL, 2.7 mmol) was added to a solution of Compound 1 (15 mg, 0.03 mmol) added to MeOH (3 ml). The reaction mixture was stirred at 60°C for 6 h, LCMS (EPN18040-043-1) showed that the reaction had been completed and no SM remained. The solvent was concentrated in vacuo. The reaction mixture was diluted with saturated NH₄Cl solution (30ml), extracted with CH₂Cl₂ (30ml×3), and washed with saturated NaCl (30ml). The resulting organic layer was dried with anhydrous Na₂SO₄, and the solvent was removed in vacuum. Purification was performed by flash column (C-18 column chromatography, H₂O (NH₄HCO₃, 0.8g/L)/CH₃CN = 70/30) to give the title compound (8.0 mg, 87% yield) as a white solid. (ESI) m/z=281.21 (M+H)⁺. ¹H NMR (500 MHz, DMSO-*d*6) 11.36 (s, 1 H), 8.34-8.35 (d, *J=* 8.5 Hz, 2 H), 7.41-7.44 (m, 2 H), 7.37 (m, 1 H), 7.24-7.26 (m, 1 H), 6.25 (s, 1 H), 3.50 (m, 2 H), 2.30 (s, 3 H), 2.02-2.04 (m, 1 H), 0.78-0.95 (m, 6 H) ppm.

### Example 40

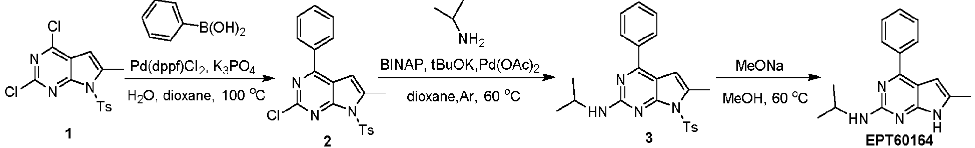

### Step 1: 2-chloro-6-methyl-4-phenyl-7-tosyl-7H-pyrrolo[2,3-d]pyrimidine (2)

K₃PO₄ (320.5 mg, 1.51 mmol), Pd(dppf)Cl₂ (73.4 mg, 0.09 mmol), and phenylboronic acid (52.5 mg, 1.0 mmol) were added respectively to a solution of Compound 1 (150 mg, 0.43 mmol) in dioxane (5 mL) and H₂O (1 mL). The reaction mixture was stirred at 80°C for 16 h (overnight). LCMS (EPN18040-028-1) under Ar conditions showed that the reaction had been completed and no SM remained. The solvent was concentrated in vacuo. The residue was purified by automatic flash column chromatography (silica gel, PE/EA=8:1) to obtain the title compound (90 mg, 54% yield) as a pale yellow solid. (ESI) m/z=398.20 (M+H)⁺.

### Step 2: N-isopropyl-6-methyl-4-phenyl-7-tosyl-7H-pyrrolo[2,3-d]pyrimidin-2-amine (3)

tBuOK (47.1 mg, 0.42 mmol), pd(OAc)₂ (4.5 mg, 0.02 mmol), BINAP (24.9 mg, 0.21 mmol) and propaN-2-amine (25 mg, 0.42 mmol) were added to a solution of compound 2 (85 mg, 0.21 mmol) in dioxane (3 mL). The reaction mixture was stirred at 60°C under Ar for 16 h (overnight). LCMS (EPN18040-034-1) showed that the reaction had been completed and no SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 40/60) to obtain the title compound (30 mg, 34% yield) as a pale yellow solid. (ESI) m/z=421.88 (M+H)⁺.

### Step 3: N-isopropyl-6-methyl-4-phenyl-7H-pyrrolo[2,3-d]pyrimidin-2-amine (EPT60164)

MeONa (0.5 mL, 2.7 mmol) was added to a solution of Compound 3 (30 mg, 0.07 mmol) added to MeOH (3 ml). The reaction mixture was stirred at 60°C for 4 h. LCMS (EPN18040-036-1) indicated that the reaction had been completed and no SM remained. The solvent was concentrated in vacuo. The reaction mixture was diluted with saturated NH₄Cl solution (30ml), extracted with CH₂Cl₂ (30ml×3), and then washed with saturated NaCl (30ml). The resulting organic layer was dried with anhydrous Na₂SO₄, and the solvent was removed in vacuum. Purification was performed by C-18 column chromatography with H₂O (NH₄HCO₃, 0.8g/L)/CH₃CN = 70/30, to obtain the title compound (3.1 mg, 16.4% yield) as a white solid. (ESI) m/z=267.24 (M+H)^{+.1}H NMR (400 MHz, DMSO-*d*6) 11.12 (s, 1 H), 8.00-8.02 (d, *J=* 8.4 Hz, 2 H), 7.43-7.50 (m, 3 H), 6.22-6.26 (m, 2 H), 4.02 (m, 1 H), 2.26 (s, 3 H), 1.45-1.13 (m, 6 H) ppm.

### Example 41

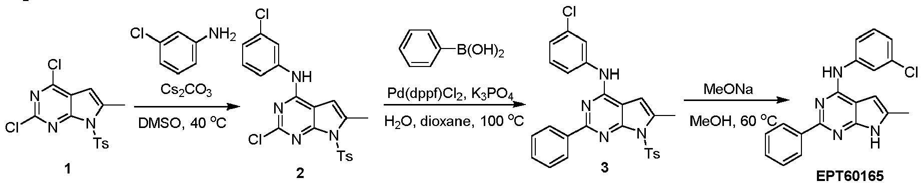

### Step 1: 2-chloro-N-(3-chlorophenyl)-6-methyl-7-tosyl-7H-pyrrolo[2,3-d]pyrimidin-4-amine (2)

Compound 1 (100 mg, 0.50 mmol) was added to DMSO (3 ml), and a solution of Cs₂CO₃ (182.5 mg, 0.56 mmol) and 3-chloroaniline (71.1 mg, 0.56 mmol) was added. The reaction mixture was stirred at 40°C for 16 hours (overnight). LCMS (EPN18040-027-1) showed that the reaction was completed and 5% of SM remained. The reaction mixture was diluted with H₂O (30 mL), extracted with CH₂Cl₂ (50 mL×3), and then washed with saturated NaCl (30 mL). The obtained organic layer was dried with anhydrous Na₂SO₄, and the solvent was removed in vacuo to obtain the desired product (90 mg, 71.6% yield) as a pale yellow solid.

(ESI) m/z=447.20 (M+H)⁺.

### Step 2: N-(3-chlorophenyl)-6-methyl-2-phenyl-7-tosyl-7H-pyrrolo[2,3-d]pyrimidin-4-amine (3)

K₃PO₄ (148.6 mg, 0.70 mmol), Pd(dppf)Cl₂ (29.3 mg, 0.04 mmol), and phenylboronic acid (122.0 mg, 1.0 mmol) were respectively added to a solution of Compound 2 (90 mg, 0.20 mmol) in dioxane (5 mL) and H₂O (1 mL). The reaction mixture was stirred at 100°C for 16 h (overnight). LCMS (EPN18040-031-1) showed that the reaction was completed and 20% of SM remained. The solvent was concentrated in vacuo. The residue was purified using automatic flash column chromatography (silica gel, PE/EA=8:1), and then purification was performed using flash column (C-18 column chromatography, H₂O (NH₄HCO₃, 0.8g/L)/CH₃CN = 70/30) to obtain the title compound (20 mg, 20.3% yield) as a white solid.

(ESI) m/z=489.25 (M+H)⁺.

### Step 3: N-(3-chlorophenyl)-6-methyl-2-phenyl-7H-pyrrolo[2,3-d]pyrimidin-4-amine (EPT60165)

MeONa (0.5 mL, 2.7 mmol) was added to a solution of Compound 3 (20mg, 0.04 mmol) added to MeOH (3ml). The reaction mixture was stirred at 60°C for 5 hours. LCMS (EPN18040-037-1) showed that the reaction was completed and no SM remained. The solvent was concentrated in vacuo. The reaction mixture was diluted with H₂O (30ml), extracted with CH₂Cl₂ (30ml×3), and then washed with saturated NaCl (30ml). The resulting organic layer was dried with anhydrous Na₂SO₄, and the solvent was removed in vacuum. The reaction mixture was diluted with H₂O (30ml), extracted with CH₂Cl₂ (30ml×3), and then washed with saturated NaCl (30ml). The resulting organic layer was dried with anhydrous Na₂SO₄, and the solvent was removed in vacuum. The residue was purified by pre-thin-layer chromatography (PE/EA=2/1) to obtain the desired product (1.9 mg, 8.5% yiled) as a white solid.

(ESI) m/z=335.22 (M+H)^{+ 1}H NMR (400 MHz, DMSO-*d*6) 11.73 (s, 1 H), 9.39 (s, 1 H), 8.32-8.34 (m, 3 H), 7.80-7.82 (m, 1 H), 7.35-7.47 (m, 4 H), 7.01-7.04 (m, 1 H), 6.47 (m, 1 H), 2.36 (m, 3 H) ppm.

### Example 42

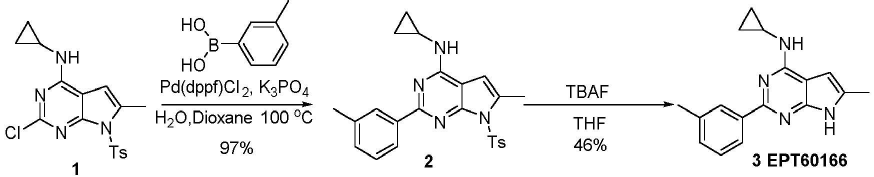

### Step 1: N-cyclopropyl-6-methyl-2-(m-tolyl)-7-tosyl-7H-pyrrolo[2,3-d]pyrimidin-4-amine (2)

N-cyclopropyl-6-methyl-2-(m-tolyl)-7-tosyl-7H-pyrrolo[2,3-d], m-tolueneboronic acid (40mg, 0.3mmol), Pd(dppf)Cl₂(14mg,0.02mmol), K₃PO₄ (63mg, 0.3mmol), added with water (0.5mL) and dioxane (3mL), were filled into a 10-mL sealed tube. The reaction mixture was heated at 90°C in an inert gas for 16 h. After the reaction was completed, the reaction mixture was evaporated and concentrated with silica gel (100-200 mesh) to obtain a powder residue. The product was purified by automatic flash column chromatography (silica gel, EA: PE = 1:3), and the product was obtained as a white solid (84 mg, 97% yield).

Molecular formula: C₂₄H₂₄N₄O₂S, molecular weight: 432.54, (ESI) m/z=433.3(M+H)⁺.

### Step 2: N-cyclopropyl-6-methyl-2-(m-tolyl)-7H-pyrrolo[2,3-d]pyrimidin-4-amine (3 EPT60166)

N-cyclopropyl-6-methyl-2-(m-tolyl)-7-tosyl-7H-pyrroline[2,3-d]pyrimidin-4-amine (84mg, 0.20mmol) was dissolved ino THF (5 mL), TBAF (2M, 2.0 mL) was added. The reaction mixture was stirred at 55°C for 24 h. After the reaction was completed, the reaction mixture was evaporated and concentrated with silica gel (100-200 mesh) to obtain a powder residue. The product was purified by automatic flash column chromatography (silica gel, EA: PE = 1:3) to obtain the title compound (26 mg, yield 46%) as a white solid.

Molecular formula: C₁₇H₁₈N₄, molecular weight: 278.36, (ESI) m/z= 279.2(M+H)⁺. ¹HNMR (500 MHz, DMSO-*d*6) 11.39 (s,1H), 8.16-8.19 (m, 2H),7.29-7.32 (m,2H), 7.18 ( d, *J*=7.5Hz,1H) , 6.28(s,1H), 3.01(s,1H), 2.38 (s, 3H), 2.32 (s,3H), 0.78-0.85 (m,2H) ,0.58-0.61(m,2H) ppm.

### Example 43

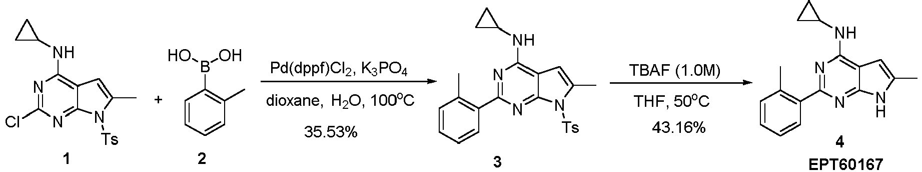

### Step 1: N-cyclopropyl-6-methyl-2-(o-tolyl)-7-tosyl-7H-pyrrolo[2,3-d]pyrimidin-4-amine (3)

2-chloro-N-cyclopropyl-6-methyl-7-tosyl-7H-pyrroline[2,3-d]pyrimidin-4-amine (50 mg , 0.13 mmol), o-toluene boronic acid (53 mg, 0.39 mmol), Pd(dppf)Cl₂ (9 mg, 0.013 mmol) and K₃PO₄(83 mg, 0.39mmol) were added to a 25 ml round bottom flask. The mixture was suspended in dioxane (5ml) and H₂O (1ml). The reaction was carried out overnight under a nitrogen atmosphere at 100°C. The solvent was removed under reduced pressure and purification was firstly performed by automatic flash column chromatography (silica gel, PE:EA=90:10) to obtain N-cyclopropyl-6-methyl-2-(o-tolyl)-7-toluenesulfonyl -7H-pyrrolo[2,3-d]pyrimidin-4-amine (7,20 mg, 35.53% yield) as a yellow solid.

LCMS: (ESI) m/z=433.12 (M+H)⁺; RT=1.91min.

### Step 2: N-cyclopropyl-6-methyl-2-(o-tolyl)-7H-pyrrolo[2,3-d]pyrimidin-4-amine (EPT60167)

In a 25 ml round bottom flask, N-cyclopropyl-6-methyl-2-(o-tolyl)-7-toluenesulfonyl -7H-pyrrolo[2,3-d]pyrimidin-4-amine (0.05 7, 20 mg, mmol) was dissolved in TBAF (1 ml, 1.0 M in tetrahydrofuran) and tetrahydrofuran (2 ml) and the mixture was stirred at 50°C for 4 h. The residue was concentrated by automatic flash in vacuum and purified by column chromatography (silica gel, DCM: methanol = 97:3) to give a yellow solid N-cyclopropyl-6-methyl-2-(o-tolyl)-7H-pyrrolo[2,3-d]pyrimidin-4-amine (EPT60167 6 mg, 43.16% yield).

LCMS: (ESI) m/z=279.24 (M+H)⁺; RT=1.23min.

¹H NMR (500 MHz, DMSO) δ 11.35 (s, 1H), 7.73 (d, *J* = 6.4 Hz, 1H), 7.31 (d, *J* = 2.5 Hz, 1H), 7.24 (d, *J* = 5.8 Hz, 3H), 6.27 (s, 1H), 2.91 (s, 1H), 2.55 (s, 3H), 2.33 (s, 3H), 0.75 (d, *J* = 5.2 Hz, 2H), 0.57 (s, 2H).

### Example 44

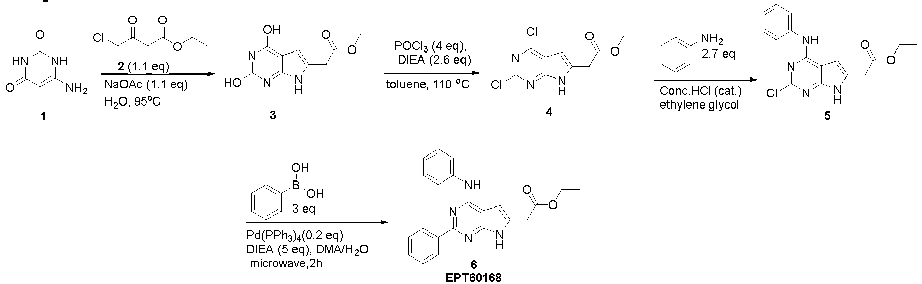

### Step 1: 2-(2,4-dihydroxy-7H-pyrrolo[2,3-d]pyrimidin-6-yl)-ethyl acetate (3)

To a suspension of 6-aminopyrimidine-2,4(1H,3H)-dione (1) (15.0 g, 118 mmol) in water (300 mL) was added sodium acetate (10.7 g, 130 mmol). The mixture was heated to 95°C. 4-chloro-3-oxalic acid ethyl ester (2) (21.4 g, 130 mmol) was added dropwise. Then the mixture was let stand for 30 min, stirred at 95°C overnight, cooled and filtered. The cake was washed with water (30mlx3) and acetone (30mlx2), and dried under vacuum to obtain the title compound (5.0 g, yield 18%) as a yellow solid.

¹H NMR (400 MHz, DMSO-*d*₆): δ 11.49 (s, 1H), 11.15 (s, 1H), 10.46 (s, 1H), 6.05 (s, 1H), 4.08 (q, *J* = 7.2 Hz, 2H), 3.60 (s, 2H), 1.20 (t, *J* = 7.2 Hz, 3H).

### Step 2: 2-(2,4-dichloro-7H-pyrrolo[2,3-d]pyrimidin-6-yl) ethyl acetate (4)

2-(2-phenyl-4-(phenylamino)-7H-pyrrole[2,3-d]pyrimidin-6-yl) ethyl acetate (3) (5.00 g, 2.11 mmol), POCl₃ (12.9 g, 8.44 mmol) were added to toluene (12ml). The reaction was heated to 70°C, N, N-diisopropylethylamine (7.08 g, 5.49 mmol) was added dropwise for 10 min, and the mixture was stirred at 110°C overnight. The mixture was cooled to room temperature and poured into water (50 mL). DIEA (10 mL) was added, and then the mixture was stired for 30 min, added with ethyl acetate (50 mL), and filtered. The filtrate was extracted with ethyl acetate (50 mL×2), and the mixed organic layer was washed with brine (20 mL), dried over Na₂SO₄, and filtered. After the filtrate was concentrated, the residue was purified by silica gel column chromatography (petroleum ether: ethyl acetate, 5:1 to 3:1) to obtain 1.1 g of a crude product. The crude oil was reacted with (petroleum ether: ethyl acetate = 10:1, 5 mL) melamine (880 mg, yield 15%) to obtain a yellow solid.

¹H NMR (400 MHz, DMSO-*d*₆): δ 12.74 (s, 1H), 6.54 (s, 1H), 4.14 (q, *J* = 6.8 Hz, 2H), 3.95 (s, 2H), 1.21 (t, *J=* 6.8 Hz, 3H).

### Step 3: 2-(2-chloro-4-(phenylamino)-7H-pyrrolo[2,3-d]pyrimidin-6-yl) ethyl acetate (5)

Two drops of concentrated aqueous hydrochloric acid were added to a solution of ethyl acetate (4) (880 mg, 3.21 mmol) and aniline (806 mg, 8.67 mmol) in ethylene glycol (15ml). The mixture was heated to 95°C and stirred for 5 hours. After cooling to room temperature, the mixture was poured in 80 ml of water, stired for 10 minutes, and filtered. The filter cake was washed with water (10 mL×5) and petroleum ether (10 mL×5), and dried in vacuo to obtain the title compound (950 mg, yield 90%) as a pink solid.

¹H NMR (400 MHz, DMSO-*d*₆): δ 11.86 (s, 1H), 9.61 (s, 1H), 7.76 (d, *J* = 7.6 Hz, 2H), 7.36 (t, *J* = 8.0 Hz, 2H), 7.07 (t, *J* = 7.6 Hz, 1H), 6.61 (s, 1H), 4.13 (q, *J* = 7. 2 Hz, 2H), 3.81 (s, 2H), 1.22 (t, *J* = 7.2 Hz,3H).

### Step 4: 2-(2-phenyl-4-(phenylamino)-7H-pyrrolo[2,3-d]pyrimidin-6-yl) ethyl acetate (6, EPT60168)

2-ethyl 2-(2-chloro-4-(phenylamino)-7H-pyridinol [2,3-d]] ethyl acetate (5) (500 mg, 1.51 mmol), phenylboronic acid (553 mg, 4.53 mmol), DIEA (974 mg, 7.55 mmol) and Pd(PPh3)₄ (349 mg, 0.30 mmol) in DMA/water (5 mL/1 mL) were added in a sealed tube, and the mixture was stirred at 140°C in microwave for 3 hours. After cooling to room temperature, the mixture was poured into water (30 mL) and extracted with ethyl acetate (20 mL 2). The combined organic layer was concentrated and the residue was purified by a C₁₈ column (acetonitrile: water from 60% to 80% during 15 minutes) to give a crude product (160 mg). 60 mg of the crude product was purified by pre-thin-layer chromatography (petroleum ether: ethyl acetate = 3:1) to obtain the title compound (30 mg, yield 14%) as a yellow solid.

¹H NMR (400MHz, DMSO-*d*₆): δ 11.79 (s, 1H), 9.35 (s, 1H), 8.37 (d, *J* = 9.2 Hz, 2H), 7.98 (d, *J=* 10.4 Hz, 2H), 7.51-7.40 (m, 5H), 7.04 (t, *J=* 10.0 Hz, 1H), 6.69 (s, 1H), 4.14 (q, *J*= 9.6 Hz, 2H), 3.85 (s, 2H), 1.23 (t, *J=* 9.6 Hz, 3H).

LC-MS [mobile phase: from 80% water (0.02% NH₄OAc) and 20% CH₃CN to 5% water (0.02% NH₄OAc) and 95% CH₃CN within 6.5 min], Rt = 3.981 min; Purity: 94.35% (214 nm), 96.03% (254 nm); MS calculated: 372.2; MS measured: 373.0 [M+H]⁺.

### Example 45

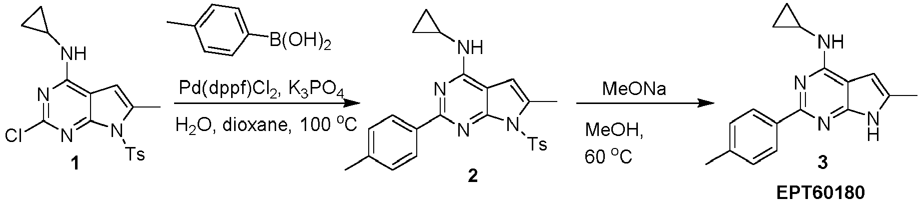

### Step 2: N-cyclopropyl-6-methyl-2-(p-tolyl)-7-tosyl-7H-pyrrolo[2,3-d]pyrimidin-4-amine (2)

K₃PO₄ (201.7 mg, 0.95 mmol), Pd(dppf)Cl₂ (36.6 mg, 0.05 mmol) and 4-methylphenylboronic acid (183.6 mg, 1.35 mmol) were respectively added to a solution of Compound 1 (100 mg, 0.27 mmol) in dioxane (5 mL) and H₂O (1 mL). The reaction mixture was stirred at 100°C for 16 h (overnight). Under Ar conditions, LCMS (EPN18040-044-1) showed that the reaction was completed and 30% of SM remained. The solvent was concentrated in vacuo. The residue was purified by automatic flash column chromatography (silica gel, PE/EA=20/1) to obtain the title compound (60 mg, 43.8% yield) as a pale yellow solid. (ESI) m/z=433.36 (M+H)⁺.

### Step 2: N-cyclopropyl-6-methyl-2-(p-tolyl)-7H-pyrrolo[2,3-d]pyrimidin-4-amine (EPT60180)

MeONa (0.5 mL, 2.7 mmol) was added to a solution of Compound 2 (60 mg, 0.14 mmol) added to MeOH (3 ml). The reaction mixture was stirred at 60°C for 4 h. LCMS (EPN18040-046-1) indicated that the reaction had been completed and no SM remained. The solvent was concentrated in vacuo. The reaction mixture was diluted with saturated NH₄Cl solution (30ml), extracted with CH₂Cl₂ (30ml×3), and then washed with saturated NaCl (30ml). The resulting organic layer was dried with anhydrous Na₂SO₄, and the solvent was removed in vacuum. Purification was performed by C-18 column chromatography with H₂O (NH₄HCO₃, 0.8g/L)/CH₃CN = 70/30, to obtained the title compound (3.1 mg, 16.4% yield) as a white solid. (ESI) m/z=279.24 (M+H)⁺. ¹H NMR (500 MHz, DMSO-*d*6) 11.26 (s, 1 H), 8.25-8.26 (d, *J* = 8.0 Hz, 2 H), 7.31-7.32 (m, 1 H), 7.22-7.24 (m, 2 H), 6.23 (s, 1 H), 2.99 (s, 1 H), 2.35 (s, 3 H), 2.31 (s, 3 H), 0.79-0.81 (s, 2 H), 0.58-0.59 (s, 2 H) ppm.

### Example 46

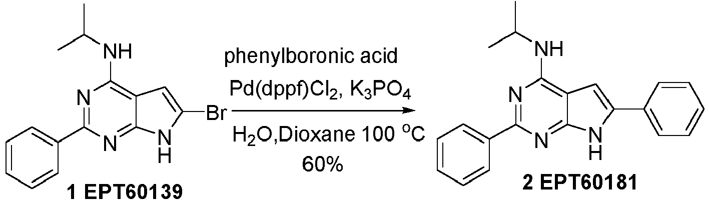

### Step 1: N-isopropyl 2,6-diphenyl-7H-pyrrolo[2,3-d]pyrimidin-4-amine (5 EPT60181)

6-bromo-N-isopropyl-2-phenyl-7H-pyrrole[2,3-d]pyrimidin-4-amine (50mg, 0.15mmol), phenylboronic acid (61mg, 0.5mmol), Pd(dppf)Cl₂(10mg,0.015mmol), K₃PO₄ (110mg, 0.5mmol), water (1.0 mL), and dioxane (5 mL) were filled into a 10 ml sealed tube. The reaction mixture was heated at 90°C in an inert gas for 16 h. The reaction was monitored by LCMS. After the reaction was completed, the reaction mixture was evaporated and concentrated with silica gel (100-200 mesh) to obtain a powder residue. The product was purified by automatic flash column chromatography (silica gel, EA: PE=35:65), and the product was obtained as a white solid (30 mg, yield 60%).

Molecular formula: C₂₁H₂₀N₄, molecular weight: 328.42, (ESI) m/z=329.3(M+H)⁺.¹H NMR (400 MHz, DMSO-d6) 11.86(s,1H), 8.36-8.38(m, 2H), 7.33-7.50 (m, 8H), 7.24 (d, J=2.4Hz, 1H), 5.06 (d, J=7.2Hz, 1H), 4.41-4.46 (m,1H),1.17 ( d, J=6.4Hz, 6H) ppm.

### Example 47

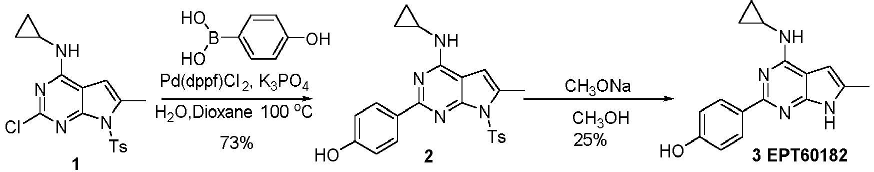

### Step 1: 4-(4-(cyclopropylamino)-6-methyl-7-tosyl-7H-pyrrolo[2,3-d]pyrimidin-2-yl)phenol (2)

2-chloro-N-cyclopropyl-6-methyl-7-tosyl-7-h-pyrrolo[2,3-d]pyrimidine-4-amino (110mg, 0.3mmol), (4-hydroxyphenyl)boronic acid (84mg, 0.6mmol), Pd(dppf)Cl₂ (20mg, 0.03mmol), K₃PO₄ (124mg, 0.6mmol), water (1.0mL), dioxane (5mL) were added into a 10 ml sealed tube. The reaction mixture was heated at 90°C in an inert gas for 16 h. After the reaction was completed, the reaction mixture was evaporated and concentrated with silica gel (100-200 mesh) to obtain a powder residue. The product was purified by automatic flash column chromatography (silica gel, EA: PE = 3:7) to obtain the title compound (88.8 mg, yield 73.6%) as a white solid.

Molecular formula: C₂₃H₂₂N₄O₃S, molecular weight: 434.51, (ESI) m/z=435.3(M+H)⁺.

### Step 2: 4-(4-(cyclopropylamino)-6-methyl-7H-pyrrolo[2,3-d]pyrimidin-2-yl)phenol (3 EPT60182)

4-(4-(cyclopropylamino)-6-methyl-7-tosyl-7H-pyrrole[2,3-d]pyrimidin-2-yl)phenol (50mg, 0.115mmol) was dissolved in CH₃OH (2mL), and CH₃ONa (1.0mL) was added. The reaction mixture was stirred at 55°C for 16 h. After the completion of the reaction, the reaction was quenched with saturated NH₄Claqueous solution (1 mL), and then the reaction mixture was evaporated and concentrated with silica gel (100-200 mesh) to obtain a powdery residue. The product was purified by automatic flash column chromatography (silica gel, EA: PE = 2:3) to obtain the title compound (8 mg, yield 25%) as a white solid.

Molecular formula: C₁₆H₁₆N₄O, molecular weight: 280.33 (ESI) m/z= 281.2(M+H)⁺. ¹H NMR (400 MHz, DMSO-*d*6) 11.23(s, 1H), 9.54(s, 1H), 8.15 (d, *J*=8.4 Hz, 2H), 7.18 (d, *J*=2.8 Hz, 1H), 6.75 (d, *J*=8.8 Hz, 2H), 6.20(s, 1H), 2.94-2.98(m, 1H), 2.26(s, 3H), 0.73-0.77 (m,2H), 0.52-0.56 (m,2H) ppm.

### Example 48

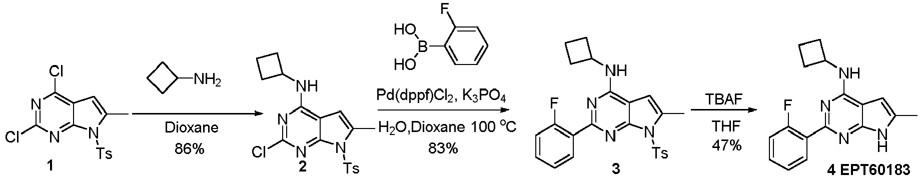

### Step 1: 2-chloro-N-cyclobutyl-6-methyl-7-tosyl-7H-pyrrolo[2,3-d]pyrimidin-4-amine (2)

2,4-dichloro-6-methyl-7-tosyl-7-h-pyrrolo[2,3-d]pyrimidine (107mg, 0.3mmol) and cyclobutylamine (185mg, 2.6 mmol) were respectively filled into a 20ml sealed tube, and then dioxane (5mL) was added. The reaction mixture was stirred at 70°C for 3h. The reaction was monitored by LCMS.

Molecular formula: C₁₈H₁₉ClN₄O₂S, molecular weight: 390.89, (ESI) m/z=391.2(M+H)⁺, which was used directly in the next step.

### Step 2: N-cyclobutyl-2-(2-fluorophenyl)-6-methyl-7-tosyl-7H-pyrrolo[2,3-d]pyrimidin-4-amine (3)

2-chloro-N-cyclobutyl-6-methyl-7-toluenesulfonyl-7-H-pyrrolo[2,3-d]pyridine-4-amine (crude product in 5mL dioxane), (2-fluorophenyl)boronic acid (140mg, 1.0mmol), Pd(dppf)Cl₂ (20mg, 0.03mmol), K₃PO₄ (210mg, 1.0mmol) were filled into a 10 ml sealed tube, and then water (1.0mL) was added. The reaction mixture was heated at 90°C in an inert gas for 16 h. After the reaction was completed, the reaction mixture was evaporated and concentrated with silica gel (100-200 mesh) to obtain a powder residue. The product was purified by automatic flash column chromatography (silica gel, EA: PE = 15:85) to obtain the title compound (112 mg yield 83%) as a white solid.

Molecular formula: C₂₄H₂₃FN₄O₂S, molecular weight: 450.53,(ESI) m/z=451.3(M+H)⁺.

### Step 4: N-cyclobutyl-2-(2-fluorophenyl)-6-methyl-7H-pyrrolo[2,3-d]pyrimidin-4-amine (4 EPT60183)

N-cyclobutyl-2-(2-fluorophenyl)-6-methyl-7-tosyl-7H-pyrrole[2,3-d]pyrimidin-4-amine (112mg, 0.25mmol) was dissolved in THF (5 mL), TBAF (2M, 2.0 mL) was added. The reaction mixture was stirred at 55°C for 1 h. After the reaction was completed, the reaction mixture was evaporated and concentrated with silica gel (100-200 mesh) to obtain a powder residue. The product was purified by automatic flash column chromatography (silica gel, EA: PE=35:65), and the product was obtained as a white solid (35 mg, yield 47.3%).

Molecular formula: C₁₇H₁₇FN₄, molecular weight: 296.35, (ESI) m/z=297.2 (M+H)⁺. ¹H NMR (400 MHz, DMSO-*d*6) 11.41(s, 1H), 7.91-7.95(m, 1H),7.34-7.39 (m,2H), 7.16-7.23 (m, 2H), 6.21(s, 1H), 4.61-4.67(m, 1H), 2.24-2.32(m, 5H), 1.96-2.06 (m, 2H) ,1.64-1.70(m, 2H) ppm.

### Example 49

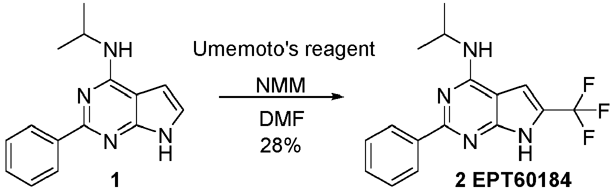

### Step 1: N-isopropyl-2-phenyl-6-(trifluoromethyl)-7H-pyrrolo[2,3-d]pyrimidin-4-amine (2 EPT60184)

N-isopropyl-2-phenyl-7H-pyrrolo[2,3-d]pyrimidin-4-amine (50mg, 0.2mmol), Umemoto's reagents (136mg, 0.4mmol), 4-methylmorpholine (41mg, 0.4mmol) were filled into a 10 ml sealed tube, and DMF (1mL) was added. The reaction mixture was stirred at room temperature for 3h. The reaction was monitored by LCMS. After the reaction was completed, the reaction mixture was evaporated and concentrated with silica gel (100-200 mesh) to obtain a powder residue. The product was purified by automatic flash column chromatography (silica gel, DCM) to obtain the product as a yellow solid (18 mg, yield 28%). Molecular formula: C₁₆H₁₅F₃N₄, molecular weight: 320.32, (ESI) m/z=321.2(M+H)⁺. ¹HNMR (500 MHz, DMSO-d₆)₁2.78 (s,1H), 8.37-8.40(m, 2H),7.67(d, J=7.5Hz, 1H) ,7.43-7.50 (m,3H), 7.22(s, 1H), 4.54-4.58(m, 1H),1.31(m, 6H) ppm.

### Example 50

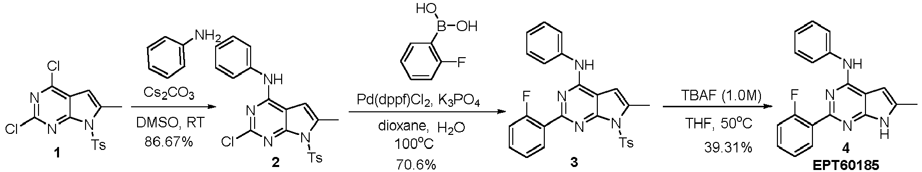

### Step 1: 2-chloro-6-methyl-N-phenyl-7-tosyl-7H-pyrrolo[2,3-d]pyrimidin-4-amine (2)

2,4-dichloro-6-methyl-7-tosyl-7H-pyridine[2,3-d]pyrimidine (50 mg, 0.14 mmol), aniline (26 mg, 0.28 mmol) and Cs₂CO₃ (138 mg, 0.42 mmol) were added in a 25ml round bottom flask. The mixture was suspended in DMSO (3ml) and stirred at 50°C for 1 h. The residue was concentrated in vacuo to obtain 2-chloro-6-methyl-N-phenyl-7-tosyl-7H-pyrrolo[2,3-d] pyrimidine-4-amine (50 mg, 86.67% yield) as a yellow solid. The crude product was used in the next step without further purification.

LCMS: (ESI) m/z=413.24 (M+H)⁺; RT=1.88min.

### Step 2: 2-(2-fluorophenyl)-6-methyl-N-phenyl-7-tosyl-7H-pyrrolo[2,3-d]pyrimidin-4-amine (3)

2-chloro-6-methyl-N-phenyl-7-tosyl-7H-pyridinol[2,3-d] 4-aminopyrimidine (50 mg, 0.12 mmol), (2-fluorophenyl)boronic acid (50 mg, 0.36 mmol), Pd(dppf)Cl₂ (9 mg, 0.012 mmol) and K₃PO₄(51 mg, 0.24 mmol were respectively filled into a 25 ml round bottom flask. The mixture was suspended in dioxane (5ml) and H₂O (1ml). The reaction was carried out overnight under a nitrogen atmosphere at 100°C. The solvent was removed under reduced pressure and purification was performed by automatic flash column chromatography (silica gel, PE:EA=1:1) to obtain the product as a yellow solid 2-(2-fluorophenyl)-6-methyl-N-phenyl-7 -tosyl-7H-pyrrolo[2,3-d]pyrimidin-4-amine (7,40 mg, 70.62% yield). LCMS: (ESI) m/z=473.33 (M+H)⁺; RT=1.96min.

### Step 3: 2-(2-fluorophenyl)-6-methyl-N-phenyl-7H-pyrrolo[2,3-d]pyrimidin-4-amine (EPT60185)

In a 25 ml round bottom flask, 2-(2-fluorophenyl)₆-methyl-N-phenyl-7-methylsulfonyl-7H-pyrrolo[2,3-d]pyrimidine-N-4-ami ne (40 mg, 0.08 mmol) was dissolved in TBAF (1 ml, 1.0 M in tetrahydrofuran) and tetrahydrofuran (2 ml) and the mixture was stirred at 50°C for 8 hours. The residue was concentrated by automatic flash in vacuum and purification was performed by column chromatography (silica gel, DCM: methanol = 97:3) to give a white solid 2-(2-fluorophenyl)-6-methyl-N-phenyl-7H -pyrrolo[2,3-d]pyrimidin-4-amine (10 mg, EPT60185 39.31% yield).

LCMS: (ESI) m/z=319.21 (M+H)⁺; RT=1.52min.

¹H NMR (400 MHz, dmso) δ 11.76 (s, 1H), 9.23 (s, 1H), 8.05 - 7.95 (m, 3H), 7.46 (dd, *J* = 12.5, 5.9 Hz, 1H), 7.30 (dt, *J* = 8.2, 6.7 Hz, 4H), 6.98 (t, *J* = 7.3 Hz, 1H), 6.51 (s, 1H), 2.39 (s, 3H).

### Example 51

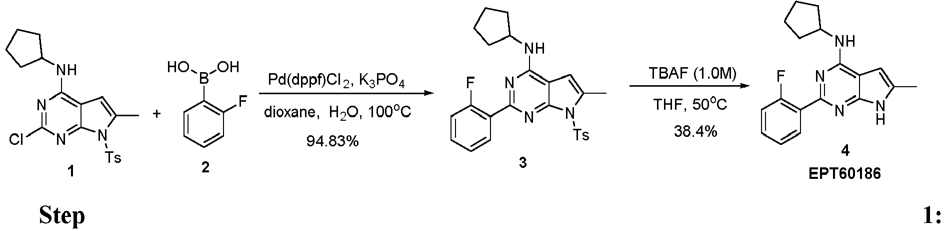

### N-cyclopentyl-2-(2-fluorophenyl)-6-methyl-7-tosyl-7H-pyrrolo[2,3-d]pyrimidin-4-amine (3)

2-chloro-N-cyclopentyl-6-methyl-7-tosyl-7H-pyrrolo[2,3-d]pyrimidin-4-amine (100 mg , 0.25 mmol), (2-fluorophenyl)boronic acid (105 mg, 0.75 mmol), Pd(dppf)Cl₂ (18 mg, 0.025 mmol) and K₃PO₄(106 mg, 0.5 mmol) were added in a 25 ml round bottom flask. The mixture was suspended in dioxane (5ml) and H₂O (1ml). The reaction was carried out overnight under a nitrogen atmosphere at 100°C. The solvent was removed under reduced pressure and purification was performed by automatic flash column chromatography (silica gel, PE:EA=60:40) to obtain a pale yellow solid N-cyclopentyl-2-(2-fluorophenyl)-6-methyl-7 -Tosyl-7H-pyrrolo[2,3-d]pyrimidin-4-amine (7,110 mg, 94.83%). LCMS: (ESI) m/z=465.37 (M+H)⁺; RT=1.99min.

### Step 2: N-cyclopentyl-2-(2-fluorophenyl)-6-methyl-7H-pyrrolo[2,3-d]pyrimidin-4-amine (EPT60186)

In a 25 ml round bottom flask, N-cyclopentyl-2-(2-fluorophenyl)-6-methyl-7-methylsulfonyl-7H-pyrrolo[2,3-d]pyrimidine-N-4-amine (40 mg, 0.09 mmol) was dissolved in TBAF (1 ml, 1.0 M in tetrahydrofuran) and tetrahydrofuran (2 ml) and the mixture was stirred at 50°C for 8 hours. The residue was concentrated by automatic flash in vacuum and purification was perfomed by column chromatography (silica gel, DCM: methanol = 97:3) to give a white solid N-cyclopentyl-2-(2-fluorophenyl)-6-methyl- 7H-pyrrolo[2,3-d]pyrimidin-4-amine (EPT60186, 25 mg, 38.4% yield).

LCMS: (ESI) m/z=311.21 (M+H)⁺; RT=1.34min.

¹H NMR (500 MHz, DMSO) δ 11.42 (s, 1H), 7.97 (td, *J* = 7.8, 1.7 Hz, 1H), 7.44 - 7.37 (m, 1H), 7.28 - 7.19 (m, 2H), 7.09 (d, *J* = 6.9 Hz, 1H), 6.28 (d, *J* = 0.9 Hz, 1H), 4.48 (dd, *J* = 13.8, 6.9 Hz, 1H), 2.32 (s, 3H), 2.01 (dd, *J* = 9.3, 5.4 Hz, 2H), 1.72 (s, 2H), 1.56 (d, *J* = 4.6 Hz, 4H).

### Biological Examples

### Example 52 Determination of the inhibitory activity of the compounds on IDH2/R140Q

In this example, the inhibitory activity of a compound on IDH2/R140Q was determined by measuring the reduction of the cofactor NADPH. The compound was incubated with IDH2/R140Q and NADPH, and then the reaction was initiated by adding α-KG. After a certain time of reaction under linear conditions, lipoamide dehydrogenase and the corresponding substrate resazurin were added for detection. Lipoamide dehydrogenase terminated the IDH2/R140Q reaction by subtracting the available cofactor NADPH. It oxidized NADPH to NADP, and reduced resazurin to highly fluorescent resorufin. The production of resorufin was detected to quantify the amount of cofactor NADPH remaining after a specific reaction time.

Specically, in a 96-well plate, in a total reaction volume of 50 µL, a mixture of 1.2 nM IDH2/R140Q, serially diluted inhibitors, and 5 µM NADPH in a reaction buffer containing Tris-HCl 50 mM (pH 7.5), 150 mM NaCl, 10 mM MgCl₂, BSA 0.05%, 10% Glycerol, and 2.5 mM β-mercaptoethanol, was pre-incubated at 25°C for 16 hours. α-KG was added to 1mM, and the reaction was carried out at 25°C for 40 minutes. Then 25 µL of a stop mixed solution (lipoamide dehydrogenase 36 µg/mL, resazurin 30 µM) prepared with the above reaction buffer was added to convert resazurin to resorufin to measure the remaining NADPH. After 10 minutes of incubation at 25°C, the fluorescence value was measured by Tecan INFINITE F FLEX under Ex544/Em590. The enzyme activity was measured at 10 concentrations for each compound, and multiple background holes without enzyme and full enzyme activity holes without compound were set up in the reaction. The concentration of dimethyl sulfoxide in the system was less than or equal to 2%. The value of IC₅₀ was obtained by the formula: Y=100/(1+10^((LogIC₅₀-X)^{∗}HillSlope)) using XLFit5 software (IDBS Software). The multi-well plate in this example was purchased from Thermo Fisher Scientific, NADPH, α-KG, lipoamide dehydrogenase, and resazurin were purchased from Shenggong Bioengineering Co., Ltd., and IDH2/R140Q was purchased from Abcam (ab198153).

According to the biological method described in this example, the selected compounds of the present invention were analyzed, and the results are shown in Table 2. Among them, "A" in Table 2 refers to the inhibitory activity against IDH2/R140Q with IC₅₀≤100nM; "B" refers to the inhibitory activity against IDH2/R140Q with 100nM <IC₅₀≤1µM; "C" refers to the inhibitory activity against IDH2/R140Q with 1µM <IC₅₀≤10µM; "D" refers to the inhibitory activity against IDH2/R140Q with IC₅₀ >10µM.

**Table 2 IDH2/R140Q inhibitory activity of preferred compounds**

| Compo und No. | Structural formula | IC₅₀ | Comp ound No. | Structural formula | IC₅₀ |
|---|---|---|---|---|---|
| 1 | 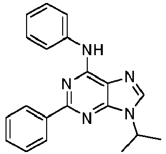 | D (1h) | 2 | 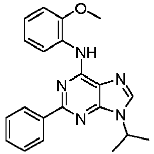 | D (1h) |
| 3 | 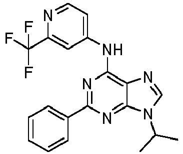 | D (1h) | 4 | 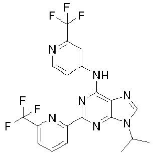 | D (1h) |
| 5 | 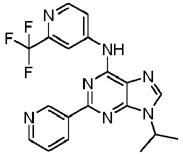 | D (1h) | 6 | 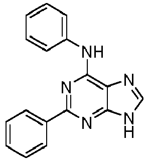 | D (1h) |
| 7 | 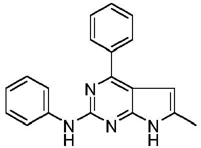 | C | 8 | 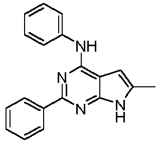 | B |
| 9 | 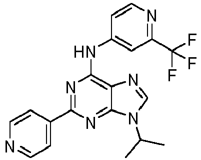 | D (1h) | 10 | 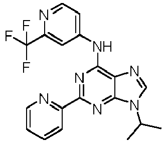 | D (1h) |
| 11 | 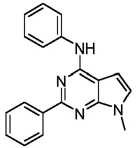 | D (1h) | 12 | 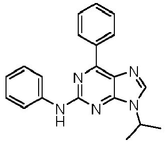 | D (1h) |
| 13 | 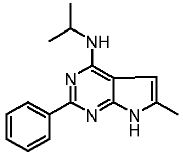 | B | 14 | 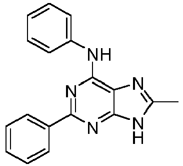 | D (1h) |
| 15 | 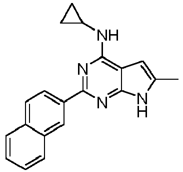 | D | 16 | 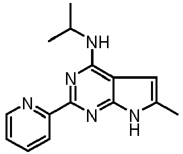 | D |
| 17 | 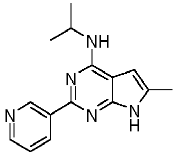 | D | 18 | 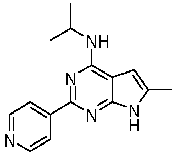 | D |
| 19 | 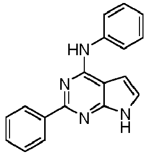 | D | 20 | 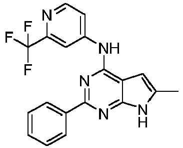 | B |
| 21 | 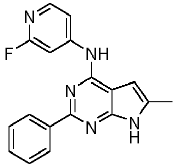 | D | 22 | 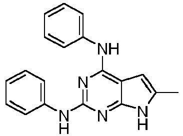 | D |
| 23 | 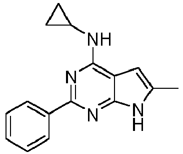 | B | 24 | 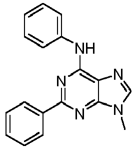 | D |
| 25 | 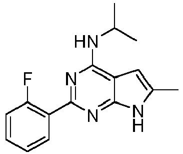 | B | 26 | 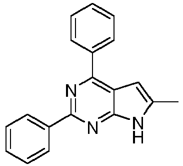 | D |
| 27 | 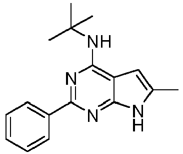 | D | 28 | 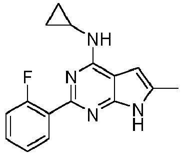 | B |
| 29 | 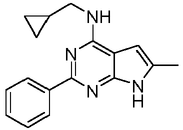 | C | 30 | 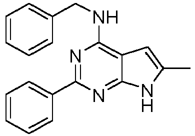 | D |
| 31 | 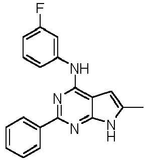 | C | 32 | 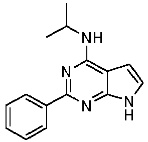 | D |
| 33 | 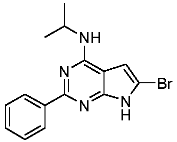 | D | 34 | 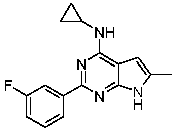 | C |
| 35 | 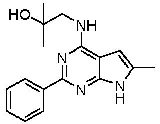 | D | 36 | 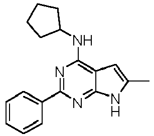 | A |
| 37 | 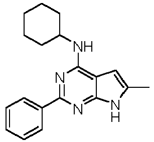 | C | 38 | 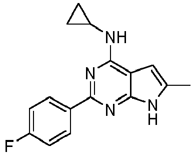 | C |
| 39 | 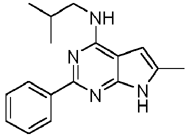 | B | 40 | 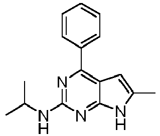 | D |
| 41 | 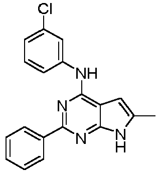 | C | 42 | 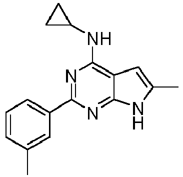 | B |
| 43 | 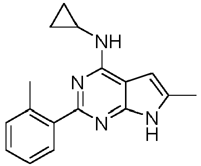 | C | 44 | 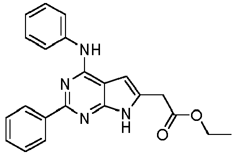 | C |
| 45 | 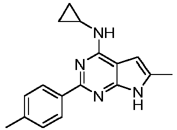 | C | 46 | 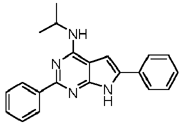 | D |
| 47 | 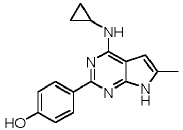 | B | 48 | 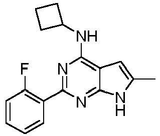 | A |
| 49 | 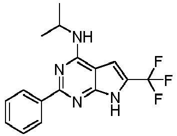 | A | 50 | 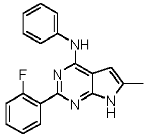 | B |
| 51 | 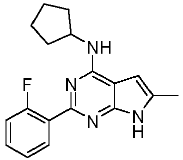 | A | AG-2 21 | 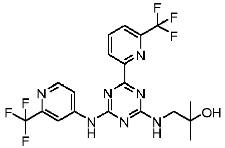 | A |

The results show that the compounds listed in the present appcatlion have very good IDH2/R140Q inhibitory activity. Among them, the inhibitory effects of compounds with activity levels A and B are extremely obvious.

### Example 53 Determination of the selectivity of compounds to wild-type IDH2 (IDH2/WT)

The compound was incubated with IDH2/WT and NADP, and then the reaction was initiated by adding isocitrate. After a certain time of reaction under linear conditions, lipoamide dehydrogenase and resazurin were added to detect the amount of fluorescent substances. In this experiment, NADP was reduced to NADPH. The latter reduced resazurin to highly fluorescent resorufin under the action of lipoamide dehydrogenase. The production of resorufin was detected to quantify the amount of cofactor NADPH generated after a specific reaction time, so as to calculate the compound's inhibitory effect on IDH2/WT.

Specically, in a 96-well plate, in a total reaction volume of 50 µL, a mixture of 0.6 nM IDH2/WT, serially diluted inhibitors, and 50 µM NADP in a reaction buffer containing Tris-HCl 20 mM (pH 7.5), 150 mM NaCl, 10 mM MgCl₂, 10% Glycerol, 0.03% BSA, and 2.5 mM β-mercaptoethanol, was pre-incubated at 25°C for 16 hours. Isocitrate was added to 50µM and the reaction was carried out at 25°C for 30 minutes. Then 25 µL of a mixed solution (lipoamide dehydrogenase 36 µg/mL, resazurin 30 µM) prepared with the above reaction buffer was added to convert resazurin to resorufin to measure the NADPH produced. After 10 minutes of incubation at 25°C, the fluorescence value was measured by Tecan INFINITE F FLEX under Ex544/Em590. The enzyme activity was measured at 10 concentrations for each compound, and multiple background holes without enzyme and full enzyme activity holes without compound were set up in the reaction. The concentration of dimethyl sulfoxide in the system was less than or equal to 2%. The value of IC₅₀ was obtained by the formula: Y=100/(1+10^((LogIC₅₀-X)^{∗}HillSlope)) using XLFit5 software (IDBS Software). In the method, the multi-well plate was purchased from Thermo Fisher Scientific, NADP, α-KG, lipoamide dehydrogenase, and resazurin were purchased from Shenggong Bioengineering Co., Ltd., and IDH2/WT was obtained by purification after E. coli overexpression. The test results of some compounds are shown in Table 3.

**Table 3 Inhibitory activity of some compounds on IDH2/WT**

| Compo und No. | Structural formula | IC₅₀ | Comp ound No. | Structural formula | IC₅₀ |
|---|---|---|---|---|---|
| 23 | 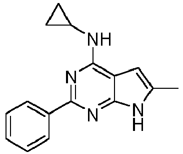 | >5uM | 25 | 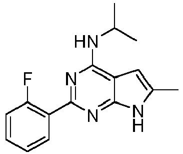 | >5uM |
| 28 | 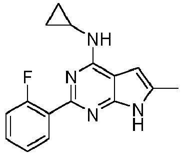 | >5uM | 36 | 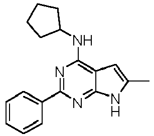 | >5uM |

The results show that the compounds of the present invention have almost no activity against wild-type IDH2 (IDH2/WT) and have good selectivity.

### Example 54 Detection of inhibition of mutant IDH2 activity at the cell level

2-hydroxyglutarate dehydrogenase (2HGDH) can reduce NAD+ to NADH in the presence of 2-HG. The latter can be quantitatively detected by diaphorase and its substrate Resazurin.

The glioma cells U87MG overexpressing IDH2/R140Q mutation were cultured in 1% sodium pyruvate high-sugar MEM, 10% FBS, and placed in a CO₂ incubator (37°C, 5% CO₂, 95% air) for cultivation.

The cells were digested with trypsin and seeded in a 96-well plate at a density of 1×10⁴ with a medium of 200 µL and cultured in a 37°C incubator overnight. The next day, the test compound was added to a final concentration of 0.1% for DMSO. After 24 hours of culturing, 100µL of the medium was aspirated, and a 10KD Nanosep^{®} ultrafiltration tube (purchased from PALL) was used for centrifugation at i4000g for 10 minutes. The protein and other components in the medium that may interfere with the results were filtered, and the follow-up method was used to detect the content of 2-HG. 50uL CellTiter-Glo (purchased from Promega) was added to the 96-well plate with the remaining 100µL of medium to detect cell survival;

Extracellular 2-HG detecting system:
(1) 50µL reaction system: reaction buffer (50mM Tris pH7.5, 100mM NaCl, 20mM MgCl₂, 0.05% BSA), in which the final concentration of NAD+ was 40µM, the final concentration of 2HGDH was 20nM, and the test sample was added to 5µL of medium; the reaction solution was mixed and centrifuged, and the reaction was carried out for 1 hour at 25°C in the dark;
(2) 25µL color developing system: color development buffer (50mM Tris pH7.5, 100mM NaCl, 20mM MgCl₂, 0.05% BSA), in which the final concentration of diaphorase was 36µg/mL, and the final concentration of resazurin sodium was 3µM; 25µL of the above-mentioned color developing solution was added to the 50µL reaction system in (1), followed by mixing and centrifugation, and fluorescence measurement was immediately performed under Ex544/Em590.

Preparation of 2-HG standard curve: a 2-HG stock solution was diluted to 20µM with reaction buffer, and a 2-fold gradient diluting was performed for a total of 6 points. Afterwards, the above-mentioned 2-HG was measured according to the extracellular 2-HG detecting system, and a standard curve was calculated and drawn.

Calculation of extracellular 2-HG content:
The fluorescence value obtained in the extracellular 2-HG detecting system was calculated using the 2-HG standard curve to calculate the content of 2-HG in the medium, and DMSO was used as a negative control to calculate the compound's inhibition against IDH2/R140Q mutation producing 2-HG activity.
The selected compounds of the present invention were analyzed according to the method described in this example, and the results are shown in Table 3. Among them, "A" in Table 4 refers to the inhibitory activity against IDH2/R140Q at the cellular level with IC₅₀≤100nM; "B" refers to the inhibitory activity against IDH2/R140Q at the cellular level with 100nM <IC₅₀≤1µM; "C" refers to the inhibitory activity against IDH2/R140Q at the cellular level with 1µM <IC₅₀≤10µM; "D" refers to the inhibitory activity of IDH2/R140Q at the cellular level with IC₅₀>10µM.

**Table 4 Inhibition against mutant IDH2/R140Q activity by preferred compounds at the cellular level**

| Compound No. | Structural formula | IC₅₀ | Compound No. | Structural formula | IC₅₀ |
|---|---|---|---|---|---|
| 3 | 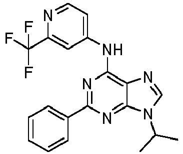 | D | 4 | 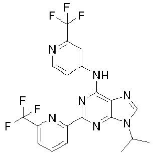 | D |
| 5 | 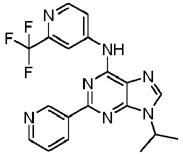 | D | 6 | 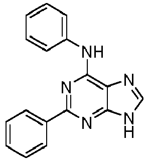 | D |
| 7 | 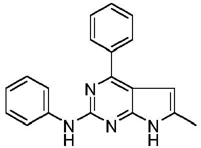 | C | 8 | 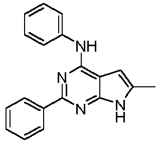 | C |
| 9 | 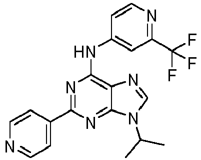 | D | 10 | 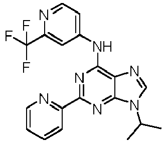 | D |
| 12 | 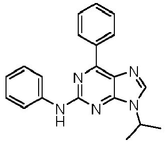 | D | 13 | 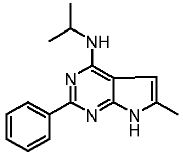 | B |
| 14 | 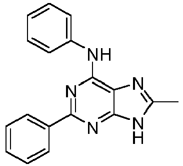 | D | 16 | 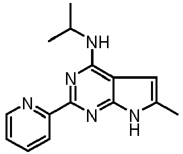 | C |
| 17 | 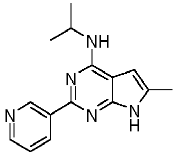 | D | 18 | 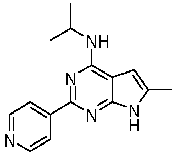 | D |
| 19 | 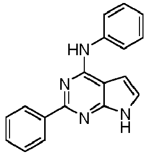 | D | 20 | 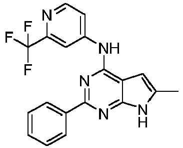 | C |
| 21 | 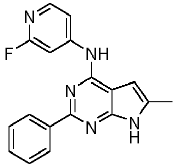 | C | 22 | 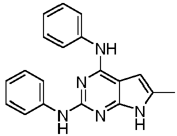 | C |
| 23 | 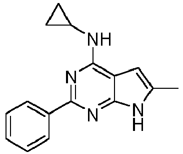 | B | 24 | 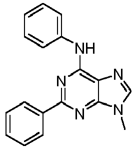 | D |
| 25 | 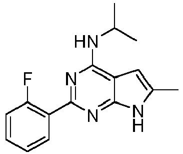 | B | 28 | 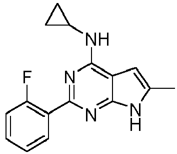 | A |
| 29 | 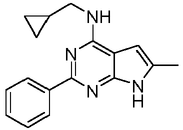 | C | 31 | 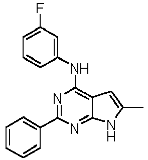 | C |
| 34 | 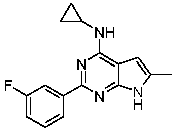 | B | 36 | 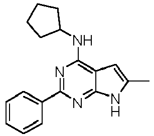 | B |
| 37 | 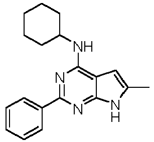 | C | 38 | 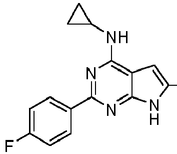 | C |
| 39 | 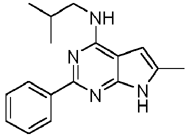 | B | 40 | 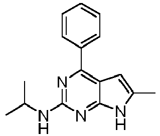 | D |
| 41 | 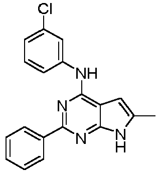 | C | 42 | 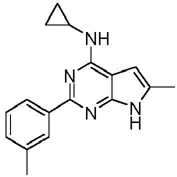 | B |
| 43 | 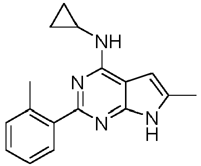 | B | 44 | 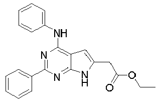 | C |
| 45 | 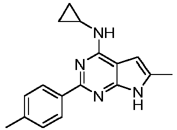 | C | 46 | 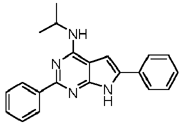 | D |
| 47 | 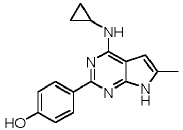 | B | 48 | 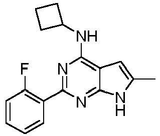 | A |
| 49 | 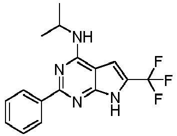 | A | 50 | 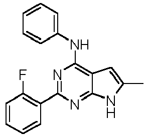 | B |
| 51 | 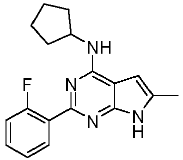 | A | AG-2 21 | 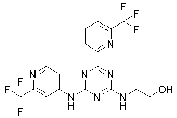 | A |

The results show that the tested compounds can inhibit IDH2/R140Q mutant cells from producing 2-HG at a relatively low concentration, showing the compounds' inhibitory effect on the activity of mutant IDH2 at the cellular level.

All documents mentioned in the present invention are cited as references in the present application, as if each document was individually cited as a reference. In addition, it should be understood that after reading the above teaching content of the present invention, those skilled in the art can make various changes or modifications to the present invention, and these equivalent forms also fall within the scope defined by the appended claims of the present application.

## Claims

1. A pyrimido five-membered heterocyclic compound represented by formula I, 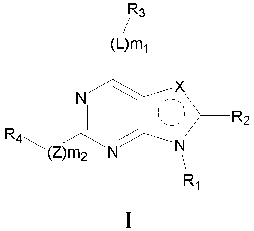 or a stereoisomer or a tautomer, or a pharmaceutically acceptable salt, a hydrate or a solvate thereof,
wherein,
R₁ is absent or selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl;
R₂ is selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₁-C₈ alkoxy, substituted or unsubstituted C₁-C₈ carboxy, substituted or unsubstituted C₂-C₂₀ ester group, substituted or unsubstituted C₆-C₁₀ aryl, or substituted or unsubstituted 5-10 membered heteroaryl with 1-3 heteroatoms selected from N, S and O;
X is selected from N, O, S or CR₅; wherein R₅ is hydrogen, halogen, -CN, substituted or unsubstituted C₁-C₈ alkyl, substituted or unsubstituted C₂-C₈ alkenyl, substituted or unsubstituted C₂-C₈ alkynyl, or substituted or unsubstituted C₃-C₁₀ cycloalkyl;
m₁ is 0, 1, 2, 3, or 4; each L is independently absent or selected from O, S, -CO-, -NH- or -CH₂-;
m₂ is 0, 1 or 2; each Z is independently absent or selected from O, S, -CO-, -NH- or -CH₂-;
R₃ is selected from hydrogen, halogen, -CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₆-C₁₀ aryl, substituted or unsubstituted 5-10 membered heteroaryl with 1-3 heteroatoms selected from N, S and O, substituted or unsubstituted 4-8 membered heterocyclic group having 1-3 heteroatoms selected from N, S and O;
R₄ is selected from hydrogen, halogen, CN, substituted or unsubstituted C₁-C₈ alkyl, substituted or unsubstituted C₂-C₈ alkenyl, substituted or unsubstituted C₂-C₈ alkynyl, substituted or unsubstituted C₃-C₁₀ cycloalkyl, substituted or unsubstituted C₆-C₁₀ aryl, substituted or unsubstituted 5-10 membered heteroaryl with 1-3 heteroatoms selected from N, S and O;
unless otherwise specified, the term "substituted" refers to being substituted by one or more (for example, 2, 3, 4, etc.) substituents selected from the following group: halogen, C₁-C₆ alkyl, halogenated C₁-C₆ alkyl, C₁-C₆ alkoxy, halogenated C₁-C₆ alkoxy, C₃-C₈ cycloalkyl, halogenated C₃-C₈ cycloalkyl, oxo, -CN, hydroxyl, amino, carboxy, benzyl, C₆-C₁₀ aryl, halogenated C₆-C₁₀ aryl, 5-10 membered heteroaryl with 1-3 heteroatoms selected from N, S and O, halogenated 5-10 membered heteroaryl with 1-3 heteroatoms selected from N, S and O.

2. The compound of claim 1, wherein the compound has a structure represented by formula Ia: 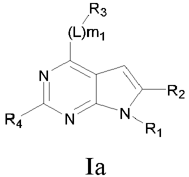 wherein, R₁, R₂, R₃, R₄, L, and m₁ are as defined in claim 1.

3. The compound of claim 1, wherein L is NH, m₁ is 1, and Z is absent, and m₂ is 0.

4. The compound of claim 1, wherein R₂ is methyl or trifluoromethyl.

5. The compound of claim 1, wherein R₄ is a fluorine-substituted phenyl group.

6. The compound of claim 1, wherein X is CR₅, wherein R₅ is selected from the group consisting of H, C₁-C₄ alkyl, or C₃-C₄ cycloalkyl.

7. The compound of claim 1, wherein the compound is compound #1, #2, #3, #4, #5, #6, #7, #8, #9, #10, #11, #12, #13, #14, #15, #16, #17, #18, #19, #20, #21, #22, #23, #24, #25, #26, #27, #28, #29, #30, #31, #32, #33, #34, #35, #36, #37, #38, #39, #40, #41, #42, #43, #44, #45, #46, #47, #48, #49, #50, or #51 in Table 1, or a pharmaceutically acceptable salt thereof.

8. The compound of claim 1, wherein the compound is compound #28, #48, #49, or #51 in Table 1, or a pharmaceutically acceptable salt thereof.

9. A pharmaceutical composition comprising: (1) the compound of claim 1, or a stereoisomer or a tautomer, or a pharmaceutically acceptable salt, a hydrate or a solvate thereof; 2) a pharmaceutically acceptable carrier.

10. A use of the compound of claim 1, or a stereoisomer or a tautomer, or a pharmaceutically acceptable salt, a hydrate or a solvate thereof, or the pharmaceutical composition of claim 9 for the manufacture of a medecament for preventing and/or treating a disease mediated by mutant IDH2.

11. The use of claim 10, wherein the disease mediated by mutant IDH2 is cancer; preferably, the cancer is selected from bladder cancer, breast cancer, kidney cancer, liver cancer, lung cancer (including small cell lung cancer), esophageal cancer, gallbladder cancer, ovarian cancer, pancreatic cancer, gastric cancer, cervical cancer, thyroid cancer, prostate cancer and skin cancer (including squamous cell carcinoma); hematopoietic tumors of the lymphatic system, including, for example, leukemia, acute lymphoid cell leukemia, acute lymphoblastic leukemia, B-cell lymphoma, T-cell lymphoma, Hodgkin's lymphoma, non-Hodgkin's lymphoma, hair cell lymphoma and Burkitt's lymphoma; tumors derived from mesenchymal cells, including, for example, fibrosarcoma and rhabdomyosarcoma; myeloid hematopoietic tumors, including, for example, acute and chronic myelogenous leukemia, myelodysplastic syndrome and promyelocytic leukemia; central and peripheral nervous system tumors, including, for example, astrocytoma, neuroblastoma, glioma, and schwannoma; and other tumors, including, for example, melanoma, seminoma, teratoma, osteosarcoma, xeroderma pigmentosum, keratoacanthoma, thyroid follicular carcinoma and Kaposi's sarcoma.

12. A method for preparing the compound of formula I, the method comprising: 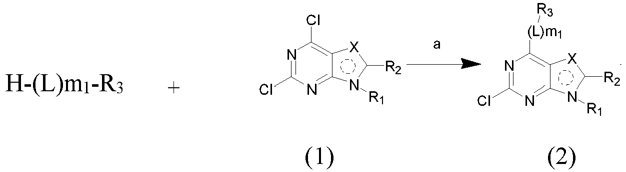 (a) reacting a compound of formula (1) with H-(L)m₁-R₃ to prepare a compound of formula (2), wherein H-(L)m₁-R₃ is an amine compound or a boric acid compound or a borate compound substituted with R₃; and 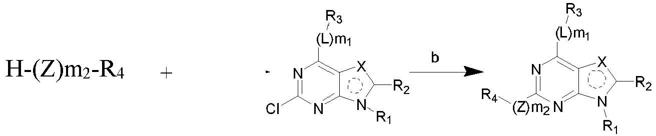 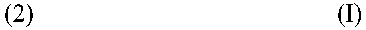 (b) reacting the compound of formula (2) with H-(Z)m₂-R₄ to prepare the compound of formula (I), wherein H-(Z)m₂-R₄ is an amine compound or a boric acid compound or a borate compound or an organotin compound substituted with R₄;
wherein, R₁, R₂, R₃, R₄, L, Z, m₁, m₂ are as defined in claim 1.

13. A method for preparing the compound represented by formula Ia, wherein the method comprises: 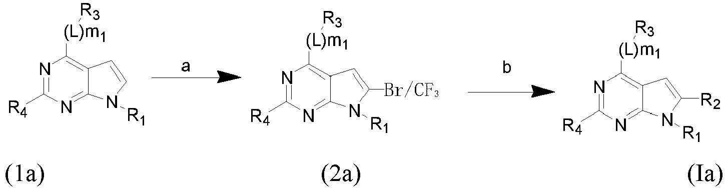 (a1) reacting a compound of formula (1a) with *N*-bromosuccinimide or S-(trifluoromethyl)dibenzothiophenium tetrafluoroborate (Umemoto's reagents) to prepare a compound of formula (2a); and
(b1) reacting the compound of formula (2a) with a boric acid compound R₂-B(OH)₂ to prepare the compound represented by formula (Ia);
wherein, the definitions of R₁, R₂, R₃, R₄, L, and m₁ are as defined in claim 1.

14. An in vitro method for inhibiting the proliferation of tumor cells containing mutant IDH2, wherein the method comprises: contacting the compound of claims 1-8, or a stereoisomer or a tautomer, or a pharmaceutically acceptable salt, a hydrate or a solvate thereof, or the pharmaceutical composition of claim 9 with a mutant IDH2, thereby inhibiting the activity of the mutant IDH2.

15. A method for preventing and/or treating a disease mediated by mutant IDH2, wherein the method comprises: administering to a subject in need thereof the compound of claims 1-8, or a stereoisomer or a tautomer, or a pharmaceutically acceptable salt, a hydrate or a solvate thereof, or the pharmaceutical composition of claim 9.
